# EUROPEAN PATENT SPECIFICATION

(11) **EP 2 142 543 B1**
(45) Date of publication and mention of the grant of the patent: **22.05.2013**
(21) Application number: 08742815.7
(22) Date of filing: 11.04.2008
(51) Int. Cl.: C07D 471/04, A61K 31/519, A61P 35/00

(54) **PYRIDO [2, 3-D]PYRIMIDIN-7-ONE COMPOUNDS AS INHIBITORS OF PI3K-ALPHA FOR THE TREATMENT OF CANCER**
PYRIDO [2,3-D]PYRIMIDIN-7-ONVERBINDUNGEN ALS INHIBITOREN VON PI3K-ALPHA ZUR BEHANDLUNG VON KREBS
COMPOSÉS PYRIDO [2, 3-D]PYRIMIDIN-7-ONE UTILISÉS EN TANT QU'INHIBITEURS DE PI3K-ALPHA POUR LE TRAITEMENT DU CANCER

(30) Priority: 11.04.2007 US 911189 P
(43) Date of publication of application: 13.01.2010
(73) Proprietor: Exelixis, Inc., South San Francisco, CA 94083 (US)
(72) Inventor: BUHR, Chris A., Redwood City, California 94061 (US); BAJJALIEH, William, San Francisco, California 94116 (US); JOSHI, Anagha Abhijit, Fremont, California 94536 (US); LARA, Katherine, San Mateo, California 94403 (US); MA, Sunghoon, Foster City, California 94404 (US); MARLOWE, Charles K., Emerald Hills, California 94062 (US); WANG, Longcheng, Palo Alto, California 94306 (US); YEUNG, Bryan K. S., Chromos 138670 (SG)
(74) Representative: Main, Malcolm Charles
(86) International application number: PCT/US2008/004753
(87) International publication number: WO 2008/127678

(56) References cited:
- WO-A-96/34867
- WO-A-2005/105801
- WO-A-2008/021389
- WO-A1-2007/044698
- US-A1- 2004 009 993

## Description

### Cross-Reference to Related Applications

The Applicants claim priority under 35 U.S.C. 119(e) to US Provisional Application No. 60/911,189 filed on April 11, 2007.

### Field of the Invention

This invention relates to the field of protein kinases and inhibitors thereof. In particular, the invention relates to inhibitors of phosphatidylinositol 3-kinase (PI3K) signaling pathways, and methods of their use.

### Summary of the Related Art

Phosphatidylinositol 3-kinase (PI3Kα), a dual specificity lipid kinase, is composed of an 85 kDa regulator subunit and a 110 kDa catalytic subunit. The protein encoded by this gene represents the catalytic subunit, which uses ATP to phosphorylate PtdIns, PtdIns4P and Ptdlns(4,5)P2. PTEN, a tumor suppressor which inhibits cell growth through multiple mechanisms, can dephosphorylate PIP3, the major product of PIK3CA. PIP3, in turn, is required for translocation of protein kinase B (AKT1, PKB) to the cell membrane, where it is phosphorylated and activated by upstream kinases. The effect of PTEN on cell death is mediated through the PIK3CA/AKT1 pathway.

PI3Kα has been implicated in the control of cytoskeletal reorganization, apoptosis, vesicular trafficking, proliferation and differentiation processes. Increased copy number and expression of PIK3CA or activating mutations in the p110a catalytic subunit of PI3KCA are associated with a number of malignancies such as ovarian cancer (Campbell et al., Cancer Res 2004, 64, 7678-7681; Levine et al., Clin Cancer Res 2005, 11, 2875-2878; Wang et al., Hum Mutat 2005, 25, 322; Lee et al., Gynecol Oncol 2005, 97, 26-34), cervical cancer, breast cancer (Bachman, et al. Cancer Biol Ther 2004, 3, 772-775; Levine, et al., supra; Li et al., Breast Cancer Res Treat 2006, 96, 91-95*;* Saal et al., Cancer Res 2005, 65, 2554-2559; Samuels and Velculescu, Cell Cycle 2004, 3, 1221-1224), colorectal cancer (Samuels, et al. Science 2004, 304, 554; Velho et al. Eur JCancer 2005, 41, 1649-1654), endometrial cancer (Oda et al. Cancer Res. 2005, 65, 10669-10673), gastric carcinomas (Byun et al., Int J Cancer 2003, 104, 318-327; Li et al., supra; Velho et al., supra; Lee et al., Oncogene 2005, 24, 1477-1480), hepatocellular carcinoma (Lee et al., *id*.), small and non-small cell lung cancer (Tang et al., Lung Cancer 2006, 51, 181-191; Massion et al., Am J Respir Crit Care Med 2004, 170, 1088-1094), thyroid carcinoma (Wu et al., J Clin Endocrinol Metab 2005, 90, 4688-4693), acute myelogenous leukemia (AML) (Sujobert et al., Blood 1997, 106, 1063-1066), chronic myelogenous leukemia (CML) (Hickey and Cotter J Biol Chem 2006, 281, 2441-2450), and glioblastomas (Hartmann et al. Acta Neuropathol (Berl) 2005, 109, 639-642; Samuels et al., supra).

WO 26/34862 describes 6-aryl pyrido(2,3-d) pyrimidine 7-imines, 7-ones and 7-thiones as inhibirors of protein tyrosine kinases. WO 2005/105801 describes pyrrolyl substituted pyrido(2,3-1)pyrimidin-7-ones and their use as PI3K inhibitors. US 2004/0009993 describes pyrido(2,3-d)pyrimidin-7(8H)ones and their use as telomerase inhibitors.

In view of the important role of PI3Kα in biological processes and disease states, inhibitors of this protein kinase are desirable.

### SUMMARY OF THE INVENTION

The following only summarizes certain aspects of the invention and is not intended to be limiting in nature. These aspects and other aspects and embodiments are described more fully below. In the event of a discrepancy between the express disclosure of this specification and the references incorporated by reference, the express disclosure of this specification shall control.

The invention provides compounds that inhibit, regulate, and/or modulate PI3K that are useful in the treatment of hyperproliferative diseases, such as cancer, in mammals. This invention also provides methods of making the compound, such compounds for use in the treatment of hyperproliferative diseases in mammals, especially humans, and to pharmaceutical compositions containing such compounds.

A first aspect of the invention provides a compound of Formula (III): 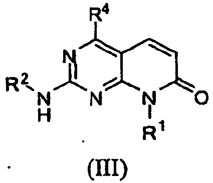 or a pharmaceutically acceptable salt and additionally optionally as a solvate or hydrate thereof, wherein
- R¹: is alkyl, cycloalkyl, or heterocycloalkyl;
- R⁴: is alkyl;
- R²: is aryl which is substituted with R¹⁸ and additionally optionally substituted with one or two R⁸;
- each R⁸,: when present, is independently hydroxy, halo, alkyl, haloalkyl, alkylcarbonyl, alkoxy, alkoxycarbonyl, haloalkoxy, alkoxyalkyl, amino, alkylamino, dialkylamino, aminocarbonyl, alkylaminocarbonyl, dialkylaminocarbonyl, aminoalkyl, alkylaminoalkyl, dialkylaminoalkyl, aminoalkyloxy, alkylaminoalkyloxy, dialkylaminoalkyloxy, or alkoxyalkylaminoalkyl;
- R¹⁸: is -L-R⁹, -C(O)NR²³R^{23a}, or -N(R¹²)C(O)R¹³,
- L: is heterocycloalkyl; and
- R⁹: is -C(O)NHR¹⁰, -C(O)R¹⁵, -S(O)₂R¹⁶, or -S(O)₂NR¹⁷R¹⁹;
- R¹⁰: is alkyl, cycloalkyl, cycloalkylalkyl, aryl, arylalkyl, heterocycloalkyl, heterocycloalkylalkyl, heteroaryl, or heteroarylalkyl;
- R¹¹: is hydrogen or alkyl;
- each R¹²: is independently hydrogen or alkyl;
- each R¹³, R¹⁵, R¹⁶, and R¹⁹: is independently alkyl;
- R¹⁷: is hydrogen or alkyl;
- R²³: is hydrogen or alkyl;
- R^{23a}: is substituted cycloalkyl, and substituted heterocycloalkyl, substituted heteroaryl, or optionally substituted aryl;

In one embodiment of the first aspect of the invention, the compound is as defined in claim 10.

Also described is a Compound of Formula **I:** 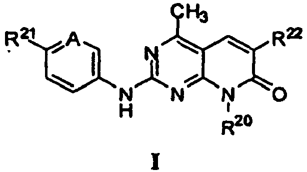 optionally as a pharmaceutically acceptable salt and additionally optionally as a solvate or hydrate thereof, wherein
- A: is =CH- or =N-; and
- R²⁰: is alkyl, cycloalkyl, or heterocycloalkyl; R²¹ is pyrazolyl, thiadiazolyl, -C(O)OH, -C(O)NHR²³ or -C(O)R²⁵; R²² is hydrogen or halo; R²³ is alkyl or phenylmethyl; and R²⁵ is piperidinyl or piperazinyl where the piperazinyl and piperidinyl are optionally substituted with one group selected from alkyl and alkoxycarbonyl; or
- R²⁰: is cycloalkyl or heterocycloalkyl; R²¹ is piperidinyl (where the piperidinyl is optionally substituted with one group selected from alkyl and alkoxycarbonyl); R²² is hydrogen or halo; or
- R²⁰: is alkyl; R²¹ is piperidinyl (where the piperidinyl is optionally substituted with one group selected from alkyl and alkoxycarbonyl); R²² is hydrogen; or
- R²⁰: is alkyl, cycloalkyl, or heterocycloalkyl; R²¹ is imidazolyl; and R²² is bromo; or
- R²⁰: is alkyl or heterocycloalkyl; R²¹ is amino, alkylamino, or dialkylamino; R²² is hydrogen or halo; or
- R²⁰: is alkyl or heterocycloalkyl; R²¹ is imidazolyl; R²² is hydrogen; or
- R²⁰: is cycloalkyl; R²¹ is amino, alkylamino, dialkylamino, or piperazinyl (optionally substituted with one group selected from alkyl and alkoxycarbonyl); and R²² is halo; or
- R²⁰: is ethyl; R²¹ is piperazinyl optionally substituted with one group selected from alkyl and alkoxycarbonyl; and R²² is hydrogen; or
- R²⁰: is heterocycloalkyl; R²¹ is piperazinyl optionally substituted with one group selected from alkyl and alkoxycarbonyl; and R²² is hydrogen.

Also described is a Compound of Formula II 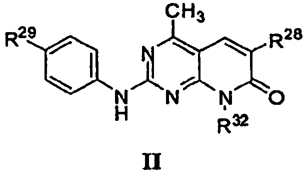 or a single isomer thereof, optionally as a pharmaceutically acceptable salt and additionally optionally as a solvate and additionally optionally as a hydrate thereof, where
- R³²: is cyclopentyl; R²⁸ is R^{28a} and R^{28a} is thiazol-2-yl or thiazol-5-yl; and R²⁹ is R^{29a} and R^{29a} is piperazinyl or piperidinyl, each of which is optionally substituted with one group selected from alkyl and alkoxycarbonyl; or

According to a further aspect of the invention, there is provided a compound of the first aspect of the invention for use in medicine.

According to a further aspect, there is provided a compound of the first aspect of the invention for use in the treatment of cancer.

R³² is ethyl, R²⁸ is R^{28b} and R^{28b} is heteroaryl, and R²⁹ is R^{29b} and R^{29b} is pyrazolyl.

In a fifth aspect, the invention is directed to a pharmaceutical composition which comprises a compound of Formula (III) optionally as a pharmaceutically acceptable salt and additionally optionally as a solvate or hydrate thereof and a pharmaceutically acceptable carrier, excipient, or diluent.

Disclosed is a method of inhibiting the *in vivo* activity of PI3Kα, the method comprising administering to a patient an effective PI3Kα-inhibiting-inhibiting amount of a compound of Formula (III) or I optionally as a pharmaceutically acceptable salt and additionally optionally as a solvate or hydrate thereof or pharmaceutical composition thereof.

Disclosed is a method for treating a disease, disorder, or syndrome which method comprises administering to a patient a therapeutically effective amount of a compound of Formula (III) or I optionally as a pharmaceutically acceptable salt and additionally optionally as a solvate or hydrate thereof, or a pharmaceutical composition comprising a therapeutically effective amount of a compound of Formula (III) or I and a pharmaceutically acceptable carrier, excipient, or diluent.

Disclosed is a method of treating cancer which method comprises administering to a patient a therapeutically effective amount of a compound of Formula (III) or I optionally as a pharmaceutically acceptable salt and additionally optionally as a solvate or hydrate thereof, or a pharmaceutical composition comprising a therapeutically effective amount of a compound of Formula (III) or I and a pharmaceutically acceptable carrier, excipient, or diluent in combination with one or more treatments selected from surgery, one or more chemotherapeutic agents, one or more hormone therapies, one or more antibodies, one or more immunotherapies, radioactive iodine therapy, and radiation,

Disclosed is a compound of Formula (IX): 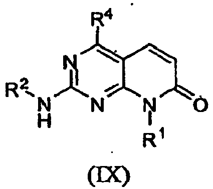 or a pharmaceutically acceptable salt or solvate thereof, wherein
- R¹: is hydrogen, optionally substituted alkyl, optionally substituted cycloalkyl, optionally substituted cycloalkylalkyl, optionally substituted aryl, optionally substituted arylalkyl, optionally substituted heterocycloalkyl, optionally substituted heterocycloalkylalkyl, optionally substituted heteroaryl, or optionally substituted heteroarylalkyl;
- R⁴: is optionally substituted alkyl;
- R²: is aryl or heteroaryl, each of which is substittued with R¹⁸ and optionally additionally substituted with one or two R⁸;
- each R⁸,: when present, is independently hydroxy, halo, alkyl, haloalkyl, alkylcarbonyl, alkoxy, alkoxycarbonyl, haloalkoxy, alkoxyalkyl, amino, alkylamino, dialkylamino, aminocarbonyl, alkylaminocarbonyl, dialkylaminocarbonyl, aminoalkyl, alkylaminoalkyl, dialkylaminoalkyl, aminoalkyloxy, alkylaminoalkyloxy, dialkylaminoalkyloxy, or alkoxyalkylaminoalkyl;
- R¹⁸: is -L-R⁹, -C(O)NR²³R^{23a}, -Y-C(O)R¹³, -Y-C(O)YR²⁴, -C(O)R²⁵, -S(O)R¹⁶, -S(O)₂R¹⁶, -S(O)NR¹⁷R¹⁹, or -S(O)₂NR²⁶R^{26a} wherein
L is optionally substituted cycloalkyl, optionally substituted heterocycloalkyl, optionally substituted aryl, or optionally substituted heteroaryl; and
R⁹ is independently -C(O)NR¹⁰R¹¹, -Y-C(O)R¹³, -Y-C(O)-YR¹⁴, -C(O)R¹⁵, -S(O)R¹⁶, -S(O)₂R¹⁶, -S(O)NR¹⁷R¹⁹, or -S(O)₂NR¹⁷R¹⁹ wherein each Y is independently -0-, -S-, or -N(R¹²)- and each R¹² is independently hydrogen or alkyl;
- R¹⁰: is independently hydrogen, alkyl, optionally substituted cycloalkyl, optionally substituted cycloalkylalkyl, optionally substituted aryl, optionally substituted arylalkyl, optionally substituted heterocycloalkyl, optionally substituted heterocycloalkylalkyl, optionally substituted heteroaryl, or optionally substituted heteroarylalkyl;
- R¹¹: is independently hydrogen or alkyl;
- each R¹³, R¹⁴, R¹⁵, R¹⁶, R¹⁷, and R¹⁹: is independently optionally substituted alkyl, optionally substituted cycloalkyl, optionally substituted cycloalkylalkyl, optionally substituted aryl, optionally substituted arylalkyl, optionally substituted heterocycloalkyl, optionally substituted heterocycloalkylalkyl, optionally substituted heteroaryl, or optionally substituted heteroarylalkyl;
- R²³: is hydrogen or alkyl;
- R^{23a}: is substituted cycloalkyl, substituted heterocycloalkyl, substituted heteroaryl, and substituted aryl;
- R²⁴: is alkyl, optionally substituted cycloalkyl, optionally substituted cycloalkylalkyl, optionally substituted aryl, optionally substituted arylalkyl, optionally substituted heterocycloalkyl, optionally substituted heterocycloalkylalkyl, optionally substituted heteroaryl, or optionally substituted heteroarylalkyl;
- R²⁵ and R^{26a}: are independently substituted cycloalkyl, substituted heterocycloalkyl, substituted heteroaryl, optionally substituted aryl, optionally substituted cycloalkylalkyl, optionally substituted heterocycloalkylalkyl, optionally substituted heteroarylalkyl, or optionally substituted arylalkyl; and
- R²⁶: is hydrogen or alkyl; and
provided that the compound is not selected from 8-cyclopentyl-2-[(4-hydroxyphenyl)amino]-4-methylpyrido[2,3-d]pyrimidin-7(8*H*)-one; 8-cyclopentyl-4-methyl-2-{[4-(methoxy)phenyl]amino}pyrido[2,3-*d*]pyrimidin-7(8*H*)-one; 8-cyclopentyl-4-methyl-2-[(4-piperazin-1-ylphenyl)amino]pyrido[2,3-*d*]pyrimidin-7(8*H*)-one; 8-cyclopentyl-2-[(4-fluorophenyl)amino]-4-methylpyrido[2,3-*d*]pyrimidin-7(8*H*)-one; 2-[(4-aminophenyl)amino]-8-cyclopentyl-4-methylpyrido[2,3-d]pyrimidin-7(8*H*)-one; methyl 3-[8-cyclopentyl-4-methyl-7-oxo-7,8-dihydropyrido[2,3-*d*]pyrimidin-2-yl)amino]benzoate; methyl 4-[8-cyclopentyl-4-methyl-7-oxo-7,8-dihydropyrido[2,3-*d*]pyrimidin-2-yl)amino]benzoate; 8-cyclopentyl-4-methyl-2-(phenylamino)pyrido[2,3-d]pyrimidin-7(8*H*)-one; 2-{[4-(4-ethylpiperazin-1-yl)phenyl]amino}-4-methyl-8-(1-methylethyl)pyrido[2,3-d]pyrimidin-7(8*H*)-one; 8-cyclopentyl-2-{[4-(1*H*-imidazol-1-yl)phenyl]amino}-4-methylpyrido[2,3-d]pyrimidin-7(8*H*)-one; and 8-cyclopentyl-2-{[4-(4-ethylpiperazin-1-yl)phenyl]amino}-4-methylpyrido[2,3-*d*]pyrimidin-7(8*H*)-one.

Also disclosed is a method of preparing a Compound of Formula I, II, or III comprising
(a) reacting an intermediate of formula 7a: 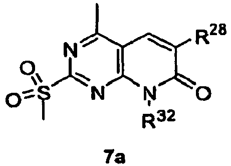 where R²⁸ and R³² are as defined in the Summary of the Invention for a Compound of Formula II, with an intermediate of formula 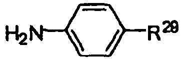 to yield a Compound of Formula II where R²⁹ is as defined in the Summary of the Invention; and optionally further resolving individual isomers, optionally further modifying one of the R²⁸, R³², and R²⁹ groups, and optionally forming a pharmaceutically acceptable salt, solvate, and/or hydrate thereof; or
(b) reacting an intermediate of formula 26a 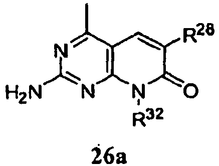 where R²⁸ and R³² are as defined in the Summary of the Invention for a Compound of Formula II, with an intermediate of formula 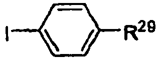 to yield a Compound of Formula II where R²⁹ is as defined in the Summary of the Invention; and optionally further resolving individual isomers, optionally further modifying one of the R²⁸, R³², and R²⁹ groups, and optionally forming a pharmaceutically acceptable salt, solvate, and/or hydrate thereof; or
(c) reacting an intermediate of formula 18a: 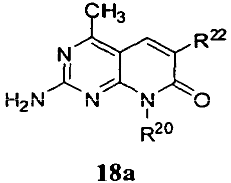 where R²⁰ and R²² are as defined in the Summary of the Invention for a Compound of Formula I, with an intermediate of formula 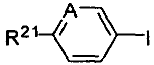 to yield a Compound of Formula I where A and R²¹ is as defined in the Summary of the Invention; and optionally further resolving individual isomers, optionally further modifying one of the R²⁰, R²¹, and R²² groups, and optionally forming a pharmaceutically acceptable salt, solvate, and/or hydrate thereof; or
(d) reacting an intermediate of formula 17(a) 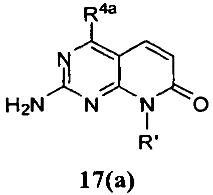 where R' is R¹ and R^{4a} is R⁴ for a Compound of Formula III (as defined in the Summary of the Invention) or R' is R²⁰ and R^{4a} is methyl (for a Compound of Formula I), with an intermediate of formula R²¹ (where R² is as defined in the Summary of the Invention for a Compound of Formula III) or 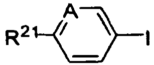 (where A and R²¹ are as defined in the Summary of the Invention for a Compound of Formula I) to yield respectively a Compound of Formula III or I; and optionally further resolving individual isomers, optionally further modifying one of the R¹, R², R⁴, R²⁰, R²¹, and R²² groups, and optionally forming a pharmaceutically acceptable salt, solvate, and/or hydrate thereof.

### DETAILED DESCRIPTION OF THE INVENTION

### Abbreviations and Definitions

The following abbreviations and terms have the indicated meanings throughout:

| **Abbreviation** | **Meaning** |
|---|---|
| br | broad |
| °C | degrees Celsius |
| d | doublet |
| dd | doublet of doublet |
| dt | doublet of triplet |
| DCM | dichloromethane |
| DME | 1,2-dimethoxyethane |
| DMF | *N,N*-dimethylformamide |
| DMSO | dimethyl sulfoxide |
| EI | electron impact ionization |
| g | gram(s) |
| h or hr | hour(s) |
| HPLC | high pressure liquid chromatography |
| L | liter(s) |
| M | molar or molarity |
| m | multiplet |
| mg | milligram(s) |
| MHz | megahertz (frequency) |
| Min or min | minute(s) |
| mL | milliliter(s) |
| µL | microliter(s) |
| µM | micromole(s) or micromolar |
| mM | millimolar |
| mmol | millimole(s) |
| mol | mole(s) |
| MS | mass spectral analysis |
| N | normal or normality |
| nM | nanomolar |
| NMR | nuclear magnetic resonance spectroscopy |
| q | quartet |
| RT or r.t. | Room temperature |
| s | singlet |
| t or tr | triplet |
| TFA | trifluoroacetic acid |
| THF | tetrahydrofuran |
| TLC | thin layer chromatography |

The symbol "-" means a single bond, "=" means a double bond, "≡" means a triple bond, "-̅-̅-̅-̅" means a single or double bond. The symbol " 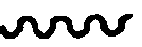 " refers to a group on a double-bond as occupying either position on the terminus of a double bond to which the symbol is attached; that is, the geometry, E- or Z-, of the double bond is ambiguous. When a group is depicted removed from its parent formula, the "∼" symbol will be used at the end of the bond which was theoretically cleaved in order to separate the group from its parent structural formula.

When chemical structures are depicted or described, unless explicitly stated otherwise, all carbons are assumed to have hydrogen substitution to conform to a valence of four. For example, in the structure on the left-hand side of the schematic below there are nine hydrogens implied. The nine hydrogens are depicted in the right-hand structure. Sometimes a particular atom in a structure is described in textual formula as having a hydrogen or hydrogens as substitution (expressly defined hydrogen), for example, -CH₂CH₂-. It is understood by one of ordinary skill in the art that the aforementioned descriptive techniques are common in the chemical arts to provide brevity and simplicity to description of otherwise complex structures.

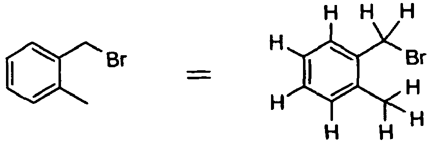

If a group "R" is depicted as "floating" on a ring system, as for example in the formula: 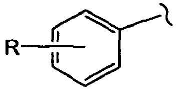 then, unless otherwise defined, a substituent "R" may reside on any atom of the ring system, assuming replacement of a depicted, implied, or expressly defined hydrogen from one of the ring atoms, so long as a stable structure is formed.

If a group "R" is depicted as floating on a fused ring system, as for example in the formulae: 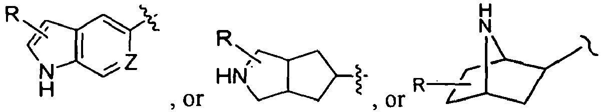 then, unless otherwise defined, a substituent "R" may reside on any atom of the fused ring system, assuming replacement of a depicted hydrogen (for example the -NH- in the formula above), implied hydrogen (for example as in the formula above, where the hydrogens are not shown but understood to be present), or expressly defined hydrogen (for example where in the formula above, "Z" equals =CH-) from one of the ring atoms, so long as a stable structure is formed. In the example depicted, the "R" group may reside on either the 5-membered or the 6-membered ring of the fused ring system. In the formula depicted above, when y is 2 for example, then the two "R's" may reside on any two atoms of the ring system, again assuming each replaces a depicted, implied, or expressly defined hydrogen on the ring.

When a group "R" is depicted as existing on a ring system containing saturated carbons, as for example in the formula: 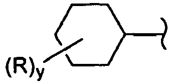 where, in this example, "y" can be more than one, assuming each replaces a currently depicted, implied, or expressly defined hydrogen on the ring; then, unless otherwise defined, where the resulting structure is stable, two "R's" may reside on the same carbon. A simple example is when R is a methyl group; there can exist a geminal dimethyl on a carbon of the depicted ring (an "annular" carbon).

"Acyl" means a -C(O)R radical where R is optionally substituted alkyl, optionally substituted alkenyl, cycloalkyl, cycloalkylalkyl, aryl, aralkyl, heteroaryl, heteroaralkyl, heterocycloalkyl, or heterocycloalkylalkyl, as defined herein, e.g., acetyl, trifluoromethylcarbonyl, or 2-methoxyethylcarbonyl, and the like.

"Acylamino" means a -NRR' radical where R is hydrogen, hydroxy, alkyl, or alkoxy and R' is acyl, as defined herein.

"Acyloxy" means an -OR radical where R is acyl, as defined herein, e.g. cyanomethylcarbonyloxy, and the like.

"Administration" and variants thereof (e.g., "administering" a compound) in reference to a compound of the invention means introducing the compound or a prodrug of the compound into the system of the animal in need of treatment. When a compound of the invention or prodrug thereof is provided in combination with one or more other active agents (e.g., surgery, radiation, and chemotherapy, etc.), "administration" and its variants are each understood to include concurrent and sequential introduction of the compound or prodrug thereof and other agents.

"Alkenyl" means a means a linear monovalent hydrocarbon radical of two to six carbon atoms or a branched monovalent hydrocarbon radical of three to 6 carbon atoms which radical contains at least one double bond, e.g., ethenyl, propenyl, 1-but-3-enyl, and 1-pent-3-enyl, and the like.

"Alkoxy" means an -OR group where R is alkyl group as defined herein. Examples include methoxy, ethoxy, propoxy, isopropoxy, and the like.

"Alkoxyalkyl" means an alkyl group, as defined herein, substituted with at least one, in another example one, two, or three, alkoxy groups as defined herein. Representative examples include methoxymethyl and the like.

"Alkoxyalkylamino" means an -NRR' group where R is hydrogen, alkyl, or alkoxyalkyl and R' is alkoxyalkyl, as defined herein.

"Alkoxyalkylaminoalkyl" means an alkyl group substituted with at least one, specifcially one or two, alkoxyalkylamino group(s), as defined herein.

"Alkoxycarbonyl" means a -C(O)R group where R is alkoxy, as defined herein.

"Alkyl" means a linear saturated monovalent hydrocarbon radical of one to six carbon atoms or a branched saturated monovalent hydrocarbon radical of three to 6 carbon atoms, e.g., methyl, ethyl, propyl, 2-propyl, butyl (including all isomeric forms), or pentyl (including all isomeric forms), and the like.

"Alkylamino" means an -NHR group where R is alkyl, as defined herein.

"Alkylaminoalkyl" means an alkyl group substituted with one or two alkylamino groups, as defined herein.

"Alkylaminoalkyloxy" means an -OR group where R is alkylaminoalkyl, as defined herein.

"Alkylcarbonyl" means a -C(O)R group where R is alkyl, as defined herein.

"Alkynyl" means a linear monovalent hydrocarbon radical of two to six carbon atoms or a branched monovalent hydrocarbon radical of three to 6 carbon atoms which radical contains at least one triple bond, e.g., ethynyl, propynyl, butynyl, pentyn-2-yl and the like.

"Amino" means -NH₂.

"Aminoalkyl" means an alkyl group substiuted with at least one, in another example one, two or three, amino groups.

"Aminoalkyloxy" means an -OR group where R is aminoalkyl, as defined herein.

"Aminocarbonyl" means a -C(O)NH₂ group.

"Alkylaminocarbonyl" means a -C(O)NHR group where R is alkyl as defined herein.

"Aryl" means a monovalent six- to fourteen-membered, mono- or bi-carbocyclic ring, wherein the monocyclic ring is aromatic and at least one of the rings in the bicyclic ring is aromatic. Unless stated otherwise, the valency of the group may be located on any atom of any ring within the radical, valency rules permitting. Representative examples include phenyl, naphthyl, and indanyl, and the like.

"Arylalkyl" means an alkyl radical, as defined herein, substituted with one or two aryl groups, as defined herein, e.g., benzyl and phenethyl, and the like.

"Aryloxy" means an -OR group where R is aryl, as defined herein.

"Carboxyalkyl" means an alkyl group, as defined herein, substituted with at least one, in another example one or two, -C(O)OH group(s).

"Cycloalkyl" means a monocyclic or fused bicyclic, saturated or partially unsaturated (but not aromatic), monovalent hydrocarbon radical of three to ten carbon ring atoms. Fused bicyclic hydrocarbon radical includes bridged ring systems. Unless stated otherwise, the valency of the group may be located on any atom of any ring within the radical, valency rules permitting. One or two ring carbon atoms may be replaced by a -C(O)-, -C(S)-, or -C(=NH)-group. The term cycloalkyl includes, but is not limited to, cyclopropyl, cyclobutyl, cyclopentyl, cyclohexyl, cyclohexyl, or cyclohex-3-enyl, and the like.

"Cycloalkylalkyl" means an alkyl group substituted with at least one, in another example one or two, cycloalkyl group(s) as defined herein.

"Dialkylamino" means a -NRR' radical where R and R' are alkyl as defined herein, or an N-oxide derivative, or a protected derivative thereof, e.g., dimethylamino, diethylamino, *N,N*-methylpropylamino or *N,N*-methylethylamino, and the like.

"Dialkylaminoalkyl" means an alkyl group substituted with one or two dialkylamino groups, as defined herein.

"Dialkylaminoalkyloxy" means an -OR group where R is dialkylaminoalkyl, as defined herein. Representative examples include 2-(*N,N-*diethylamino)-ethyloxy, and the like.

"Dialkylaminocarbonyl" means a -C(O)NRR' group where R and R' are alkyl as defined herein.

"Halogen" or "halo" refers to fluorine, chlorine, bromine and iodine.

"Haloalkoxy" means an -OR' group where R' is haloalkyl as defined herein, e.g., trifluoromethoxy or 2,2,2-trifluoroethoxy, and the like.

"Haloalkyl" mean an alkyl group substituted with one or more halogens, in another example one, two, three, four, or five halo atoms, e.g., trifluoromethyl, 2-chloroethyl, and 2,2-difluoroethyl, and the like.

"Heteroaryl" means a monocyclic, fused bicyclic, or fused tricyclic, monovalent radical of 5 to 14 ring atoms containing one or more, in another example one, two, three, or four ring heteroatoms independently selected from -O-, -S(O)ₙ- (n is 0, 1, or 2), -N-, -N(R^{x})-, and the remaining ring atoms being carbon, wherein the ring comprising a monocyclic radical is aromatic and wherein at least one of the fused rings comprising a bicyclic or tricyclic radical is aromatic. One or two ring carbon atoms of any nonaromatic rings comprising a bicyclic or tricyclic radical may be replaced by a -C(O)-, -C(S)-, or -C(=NH)- group. R^{x} is hydrogen, alkyl, hydroxy, alkoxy, acyl, or alkylsulfonyl. Fused bicyclic radical includes bridged ring systems. Unless stated otherwise, the valency may be located on any atom of any ring of the heteroaryl group, valency rules permitting. When the point of valency is located on the nitrogen, R^{x} is absent. The term heteroaryl includes, but is not limited to, 1,2,4-triazolyl, 1,3,5-triazolyl, phthalimidyl, pyridinyl, pyrrolyl, imidazolyl, thienyl, furanyl, indolyl, 2,3-dihydro-1*H*-indolyl (including, for example, 2,3-dihydro-1*H-*indol-2-yl or 2,3-dihydro-1*H*-indol-5-yl, and the like), isoindolyl, indolinyl, isoindolinyl, benzimidazolyl, benzodioxol-4-yl, benzofuranyl, cinnolinyl, indolizinyl, naphthyridin-3-yl, phthalazin-3-yl, phthalazin-4-yl, pteridinyl, purinyl, quinazolinyl, quinoxalinyl, tetrazoyl, pyrazolyl, pyrazinyl, pyrimidinyl, pyridazinyl, oxazolyl, isooxazolyl, oxadiazolyl, benzoxazolyl, quinolinyl, isoquinolinyl, tetrahydroisoquinolinyl (including, for example, tetrahydroisoquinolin-4-yl or tetrahydroisoquinolin-6-yl, and the like), pyrrolo[3,2-c]pyridinyl (including, for example, pyrrolo[3,2-c]pyridin-2-yl or pyrrolo[3,2-c]pyridin-7-yl, and the like), benzopyranyl, thiazolyl, isothiazolyl, thiadiazolyl, benzothiazolyl, benzothienyl, and the derivatives thereof, or N-oxide or a protected derivative thereof.

"Heteroarylalkyl" means an alkyl group, as defined herein, substituted with at least one, in another example one or two heteroaryl group(s), as defined herein.

"Heteroatom" refers to O, S, N, and P.

"Heterocycloalkyl" means a saturated or partially unsaturated (but not aromatic) monovalent monocyclic group of 3 to 8 ring atoms or a saturated or partially unsaturated (but not aromatic) monovalent fused bicyclic group of 5 to 12 ring atoms in which one or more, in another example one, two, three, or four ring heteroatoms independently selected from O, S(O)ₙ (n is 0, 1, or 2), N, N(R^{y}) (where R^{y} is hydrogen, alkyl, hydroxy, alkoxy, acyl, or alkylsulfonyl), the remaining ring atoms being carbon. One or two ring carbon atoms may be replaced by a -C(O)-, -C(S)-, or -C(=NH)- group. Fused bicyclic radical includes bridged ring systems. Unless otherwise stated, the valency of the group may be located on any atom of any ring within the radical, valency rules permitting. When the point of valency is located on a nitrogen atom, R^{y} is absent. The term heterocycloalkyl includes, but is not limited to, azetidinyl, pyrrolidinyl, 2-oxopyrrolidinyl, 2,5-dihydro-1*H*-pyrrolyl, piperidinyl, 4-piperidonyl, morpholinyl, piperazinyl, 2-oxopiperazinyl, tetrahydropyranyl, 2-oxopiperidinyl, thiomorpholinyl, thiamorpholinyl, perhydroazepinyl, pyrazolidinyl, imidazolinyl, imidazolidinyl, dihydropyridinyl, tetrahydropyridinyl, oxazolinyl, oxazolidinyl, isoxazolidinyl, thiazolinyl, thiazolidinyl, quinuclidinyl, isothiazolidinyl, octahydroindolyl, octahydroisoindolyl, decahydroisoquinolyl, tetrahydrofuryl, and tetrahydropyranyl, and the derivatives thereof and N-oxide or a protected derivative thereof.

"Heterocycloalkylalkyl" means an alkyl radical, as defined herein, substituted with one or two heterocycloalkyl groups, as defined herein, e.g., morpholinylmethyl, N-pyrrolidinylethyl, and 3-(*N*-azetidinyl)propyl, and the like.

"Heterocycloalkylalkyloxy means an -OR group where R is heterocycloalkylalkyl, as defined herein.

"Optional" or "optionally" means that the subsequently described event or circumstance may or may not occur, and that the description includes instances where said event or circumstance occurs and instances in which it does not. One of ordinary skill in the art would understand that with respect to any molecule described as containing one or more optional substituents, only sterically practical and/or synthetically feasible compounds are meant to be included. "Optionally substituted" refers to all subsequent modifiers in a term. So, for example, in the term "optionally substituted arylC₁₋₈ alkyl," optional substitution may occur on both the "C₁₋₈ alkyl" portion and the "aryl" portion of the molecule may or may not be substituted. A list of exemplary optional substitutions is presented below in the definition of "substituted."

"Optionally substituted alkoxy" means an -OR group where R is optionally substituted alkyl, as defined herein.

"Optionally substituted alkyl" means an alkyl radical, as defined herein, optionally substituted with one or more group(s), in another example one, two, three, four, or five groups, independently selected from alkylcarbonyl, alkenylcarbonyl, cycloalkylcarbonyl, alkylcarbonyloxy, alkenylcarbonyloxy, amino, alkylamino, dialkylamino, aminocarbonyl, alkylaminocarbonyl, dialkylaminocarbonyl, cyano, cyanoalkylaminocarbonyl, alkoxy, alkenyloxy, hydroxy, hydroxyalkoxy, halo, carboxy, alkylcarbonylamino, alkylcarbonyloxy, alkyl-S(O)₀₋₂-_{,} alkenyl-S(O)₀₋₂-, aminosulfonyl, alkylaminosulfonyl, dialkylaminosulfonyl, alkylsulfonyl-NR^{c}- (where R^{c} is hydrogen, alkyl, optionally substituted alkenyl, hydroxy, alkoxy, alkenyloxy, or cyanoalkyl), alkylaminocarbonyloxy, dialkylaminocarbonyloxy, alkylaminoalkyloxy, dialkylaminoalkyloxy, alkoxycarbonyl, alkenyloxycarbonyl, alkoxycarbonylamino, alkylaminocarbonylamino, dialkylaminocarbonylamino, alkoxyalkyloxy, and -C(O)NR^{a}R^{b} (where R^{a} and R^{b} are independently hydrogen, alkyl, optionally substituted alkenyl, hydroxy, alkoxy, alkenyloxy, or cyanoalkyl).

"Optionally substituted alkenyl" means an alkyl radical, as defined herein, optionally substituted with one or more group(s), in another example one, two, three, four, or five groups, independently selected from alkylcarbonyl, alkenylcarbonyl, cycloalkylcarbonyl, alkylcarbonyloxy, alkenylcarbonyloxy, amino, alkylamino, dialkylamino, aminocarbonyl, alkylaminocarbonyl, dialkylaminocarbonyl, cyano, cyanoalkylaminocarbonyl, alkoxy, alkenyloxy, hydroxy, hydroxyalkoxy, halo, carboxy, alkylcarbonylamino, alkylcarbonyloxy, alkyl-S(O)₀₋₂-, alkenyl-S(O)₀₋₂-, aminosulfonyl, alkylaminosulfonyl, dialkylaminosulfonyl, alkylsulfonyl-NR^{c}- (where R^{c} is hydrogen, alkyl, optionally substituted alkenyl, hydroxy, alkoxy, alkenyloxy, or cyanoalkyl), alkylaminocarbonyloxy, dialkylaminocarbonyloxy, alkylaminoalkyloxy, dialkylaminoalkyloxy, alkoxycarbonyl, alkenyloxycarbonyl, alkoxycarbonylamino, alkylaminocarbonylamino, dialkylaminocarbonylamino, alkoxyalkyloxy, and -C(O)NR^{a}R^{b} (where R^{a} and R^{b} are independently hydrogen, alkyl, optionally substituted alkenyl, hydroxy, alkoxy, alkenyloxy, or cyanoalkyl).

"Optionally substituted amino" refers to the group -N(H)R or -N(R)R where each R is independently selected from the group: optionally substituted alkyl, optionally substituted alkoxy, optionally substituted aryl, optionally substituted heterocycloalkyl, optionally substituted heteroaryl, acyl, carboxy, alkoxycarbonyl, -S(O)₂-(optionally substituted alkyl), -S(O)₂-optionally substituted aryl), -S(O)₂-(optionally substituted heterocycloalkyl), -S(O)₂-(optionally substituted heteroaryl), and -S(O)₂-(optionally substituted heteroaryl). For example, "optionally substituted amino" includes diethylamino, methylsulfonylamino, and furanyl-oxy-sulfonamino.

"Optionally substituted aminoalkyl" means an alkyl group, as defined herein, substituted with at least one, in another example one or two, optionally substituted amino group(s), as defined herein.

"Optionally substituted aryl" means an aryl group, as defined herein, optionally substituted with one, two, or three substituents independently selected from acyl, acylamino, acyloxy, optionally substituted alkyl, optionally substituted alkenyl, alkoxy, alkenyloxy, halo, hydroxy, alkoxycarbonyl, alkenyloxycarbonyl, amino, alkylamino, dialkylamino, nitro, aminocarbonyl, alkylaminocarbonyl, dialkylaminocarbonyl, carboxy, cyano, alkylthio, alkylsulfinyl, alkylsulfonyl, aminosulfonyl, alkylaminosulfonyl, dialkylaminosulfonyl, alkylsulfonylamino, aminoalkoxy, or aryl is pentafluorophenyl. Within the optional substituents on "aryl", the alkyl and alkenyl, either alone or as part of another group (including, for example, the alkyl in alkoxycarbonyl), are independently optionally substituted with one, two, three, four, or five halo.

"Optionally substituted arylalkyl" means an alkyl group, as defined herein, substituted with optionally substituted aryl, as defined herein.

"Optionally substituted cycloalkyl" means a cycloalkyl group, as defined herein, substituted with one, two, or three groups independently selected from acyl, acyloxy, acylamino, optionally substituted alkyl, optionally substituted alkenyl, alkoxy, alkenyloxy, alkoxycarbonyl, alkenyloxycarbonyl, alkylthio, alkylsulfinyl, alkylsulfonyl, aminosulfonyl, alkylaminosulfonyl, dialkylaminosulfonyl, alkylsulfonylamino, halo, hydroxy, amino, alkylamino, dialkylamino, aminocarbonyl, alkylaminocarbonyl, dialkylaminocarbonyl, nitro, alkoxyalkyloxy, aminoalkoxy, alkylaminoalkoxy, dialkylaminoalkoxy, carboxy, and cyano. Within the above optional substitutents on "cycloalkyl", the alkyl and alkenyl, either alone or as part of another substituent on the cycloalkyl ring, are independently optionally substituted with one, two, three, four, or five halo, e.g. haloalkyl, haloalkoxy, haloalkenyloxy, or haloalkylsulfonyl.

"Optionally substituted cycloalkylalkyl" means an alkyl group substituted with at least one, in another example one or two, optionally substituted cycloalkyl groups, as defined herein.

"Optionally substituted heteroaryl" means a heteroaryl group optionally substituted with one, two, or three substituents independently selected from acyl, acylamino, acyloxy, optionally substituted alkyl, optionally substituted alkenyl, alkoxy, alkenyloxy, halo, hydroxy, alkoxycarbonyl, alkenyloxycarbonyl, amino, alkylamino, dialkylamino, nitro, aminocarbonyl, alkylaminocarbonyl, dialkylaminocarbonyl, carboxy, cyano, alkylthio, alkylsulfinyl, alkylsulfonyl, aminosulfonyl, alkylaminosulfonyl, dialkylaminosulfonyl, alkylsulfonylamino, aminoalkoxy, alkylaminoalkoxy, and dialkylaminoalkoxy. Within the optional substituents on "heteroaryl", the alkyl and alkenyl, either alone or as part of another group (including, for example, the alkyl in alkoxycarbonyl), are independently optionally substituted with one, two, three, four, or five halo.

"Optionally substituted heteroarylalkyl" means an alkyl group, as defined herein, substituted with at least one, in another example one or two, optionally substituted heteroaryl group(s), as defined herein.

"Optionally substituted heterocycloalkyl" means a heterocycloalkyl group, as defined herein, optionally substituted with one, two, or three substituents independently selected from acyl, acylamino, acyloxy, optionally substituted alkyl, optionally substituted alkenyl, alkoxy, alkenyloxy, halo, hydroxy, alkoxycarbonyl, alkenyloxycarbonyl, amino, alkylamino, dialkylamino, nitro, aminocarbonyl, alkylaminocarbonyl, dialkylaminocarbonyl, carboxy, cyano, alkylthio, alkylsulfinyl, alkylsulfonyl, aminosulfonyl, alkylaminosulfonyl, dialkylaminosulfonyl, alkylsulfonylamino, aminoalkoxy, or aryl is pentafluorophenyl. Within the optional substituents on "heterocycloalkyl", the alkyl and alkenyl, either alone or as part of another group (including, for example, the alkyl in alkoxycarbonyl), are independently optionally substituted with one, two, three, four, or five halo.

"Optionally substituted heterocycloalkylalkyl" means an alkyl group, as defined herein, substituted with at least one, in another example one or two, optionally substituted heterocycloalkyl group(s) as defined herein.

"Substituted aryl" means an aryl group, as defined herein, optionally substituted with one, two, or three substituents independently selected from acyl, acylamino, acyloxy, optionally substituted alkyl, optionally substituted alkenyl, alkoxy, alkenyloxy, halo, hydroxy, alkoxycarbonyl, alkenyloxycarbonyl, amino, alkylamino, dialkylamino, nitro, aminocarbonyl, alkylaminocarbonyl, dialkylaminocarbonyl, carboxy, cyano, alkylthio, alkylsulfinyl, alkylsulfonyl, aminosulfonyl, alkylaminosulfonyl, dialkylaminosulfonyl, alkylsulfonylamino, aminoalkoxy, or aryl is pentafluorophenyl. Within the optional substituents on "aryl", the alkyl and alkenyl, either alone or as part of another group (including, for example, the alkyl in alkoxycarbonyl), are independently optionally substituted with one, two, three, four, or five halo.

"Substituted cycloalkyl" means a cycloalkyl group, as defined herein, substituted with one, two, or three groups independently selected from acyl, acyloxy, acylamino, optionally substituted alkyl, optionally substituted alkenyl, alkoxy, alkenyloxy, alkoxycarbonyl, alkenyloxycarbonyl, alkylthio, alkylsulfinyl, alkylsulfonyl, aminosulfonyl, alkylaminosulfonyl, dialkylaminosulfonyl, alkylsulfonylamino, halo, hydroxy, amino, alkylamino, dialkylamino, aminocarbonyl, alkylaminocarbonyl, dialkylaminocarbonyl, nitro, alkoxyalkyloxy, aminoalkoxy, alkylaminoalkoxy, dialkylaminoalkoxy, carboxy, and cyano. Within the above optional substitutents on "cycloalkyl", the alkyl and alkenyl, either alone or as part of another substituent on the cycloalkyl ring, are independently optionally substituted with one, two, three, four, or five halo, e.g. haloalkyl, haloalkoxy, haloalkenyloxy, or haloalkylsulfonyl.

"Substituted heteroaryl" means a heteroaryl group optionally substituted with one, two, or three substituents independently selected from acyl, acylamino, acyloxy, optionally substituted alkyl, optionally substituted alkenyl, alkoxy, alkenyloxy, halo, hydroxy, alkoxycarbonyl, alkenyloxycarbonyl, amino, alkylamino, dialkylamino, nitro, aminocarbonyl, alkylaminocarbonyl, dialkylaminocarbonyl, carboxy, cyano, alkylthio, alkylsulfinyl, alkylsulfonyl, aminosulfonyl, alkylaminosulfonyl, dialkylaminosulfonyl, alkylsulfonylamino, aminoalkoxy, alkylaminoalkoxy, and dialkylaminoalkoxy. Within the optional substituents on "heteroaryl", the alkyl and alkenyl, either alone or as part of another group (including, for example, the alkyl in alkoxycarbonyl), are independently optionally substituted with one, two, three, four, or five halo.

"Substituted heterocycloalkyl" means a heterocycloalkyl group, as defined herein, optionally substituted with one, two, or three substituents independently selected from acyl, acylamino, acyloxy, optionally substituted alkyl, optionally substituted alkenyl, alkoxy, alkenyloxy, halo, hydroxy, alkoxycarbonyl, alkenyloxycarbonyl, amino, alkylamino, dialkylamino, nitro, aminocarbonyl, alkylaminocarbonyl, dialkylaminocarbonyl, carboxy, cyano, alkylthio, alkylsulfinyl, alkylsulfonyl, aminosulfonyl, alkylaminosulfonyl, dialkylaminosulfonyl, alkylsulfonylamino, aminoalkoxy, or aryl is pentafluorophenyl. Within the optional substituents on "heterocycloalkyl", the alkyl and alkenyl, either alone or as part of another group (including, for example, the alkyl in alkoxycarbonyl), are independently optionally substituted with one, two, three, four, or five halo.

"Yield" for each of the reactions described herein is expressed as a percentage of the theoretical yield.

"AKT inhibitor" includes, for example, LY294002, PKC 412, perifosine, compounds in Table 2a, compounds in Table 2b, and compounds described in WO 2006/071819 and WO05/117909 These references also describe in vitro assays that can be used to determine the inhibitory activity of AKT.

"Alkylating agent" includes, for example, one or more of the following: Chlorambucil, Chlormethine, Cyclophosphamide, Ifosfamide, Melphalan, Carmustine, Streptozocin, Fotemustine, Lomustine, Streptozocin, Carboplatin, Cisplatin, Oxaliplatin, BBR3464, Busulfan, Dacarbazine, Mechlorethamine, Procarbazine, Temozolomide, ThioTEPA, and Uramustine.

"Antibody" includes, for example, one or more of the following: an IGF1R antibody (including, for example, ^{α}IGF-1R A12 MoAb, 19D12, h7C10 and CP-751871), an EGFR antibody (including, for example, Cetuximab (Erbitux®) and Panitumumab), an ErbB2 antibody (including, for example, Trastuzumab (Herceptin®)), a VEGF antibody (including, for example, Bevacizumab (Avastin®)), an IgG1 antibody (including, for example, Ibritumomab (tiuxetan)), a CD20 antibody (including, for example, Rituximab and Tositumomab), a CD33 antibody (including, for example, Gemtuzumab and Gemtuzumab ozogamicin), and a CD52 antibody (including, for example, Alemtuzumab).

"Antimetabolite" includes, for example, methotrexate, Pemetrexed, Raltitrexed, Cladribine, Clofarabine, Fludarabine, Mercaptopurine, Thioguanine, Capecitabine, Cytarabine, fluorouracil (administered with or without leucovorin or folinic acid), and Gemcitabine.

"Antimicrotubule agent" includes, for example, Vincristine,Vinblastine, Vinorelbine, Vinflunine, and Vindesine.

"Aromatase inhibitor" includes, for example, one or more of the following: Aminoglutethimide, Anastrozole (Arimidex®), Letrozole (Femara®), Exemestane (Aromasin®), and Formestane (Lentaron®).

"Cancer" refers to cellular-proliferative disease states, including but not limited to: Cardiac: sarcoma (angiosarcoma, fibrosarcoma, rhabdomyosarcoma, liposarcoma), myxoma, rhabdomyoma, fibroma, lipoma and teratoma; Lung: bronchogenic carcinoma (squamous cell, undifferentiated small cell, undifferentiated large cell, adenocarcinoma), alveolar (bronchiolar) carcinoma, bronchial adenoma, sarcoma, lymphoma, chondromatous hanlartoma, inesothelioma; Gastrointestinal: esophagus (squamous cell carcinoma, adenocarcinoma, leiomyosarcoma, lymphoma), stomach (carcinoma, lymphoma, leiomyosarcoma), pancreas (ductal adenocarcinoma, insulinorna, glucagonoma, gastrinoma, carcinoid tumors, vipoma), small bowel (adenocarcinoma, lymphoma, carcinoid tumors, Karposi's sarcoma, leiomyoma, hemangioma, lipoma, neurofibroma, fibroma), large bowel (adenocarcinoma, tubular adenoma, villous adenoma, hamartoma, leiomyoma); Genitourinary tract: kidney (adenocarcinoma, Wilm's tumor [nephroblastoma], lymphoma, leukemia), bladder and urethra (squamous cell carcinoma, transitional cell carcinoma, adenocarcinoma), prostate (adenocarcinoma, sarcoma), testis (seminoma, teratoma, embryonal carcinoma, teratocarcinoma, choriocarcinoma, sarcoma, interstitial cell carcinoma, fibroma, fibroadenoma, adenomatoid tumors, lipoma); Liver: hepatoma (hepatocellular carcinoma), cholangiocarcinoma, hepatoblastoma, angiosarcoma, hepatocellular adenoma, hemangioma; Bone: osteogenic sarcoma (osteosarcoma), fibrosarcoma, malignant fibrous histiocytoma, chondrosarcoma, Ewing's sarcoma, malignant lymphoma (reticulum cell sarcoma), multiple myeloma, malignant giant cell tumor chordoma, osteochronfroma (osteocartilaginous exostoses), benign chondroma, chondroblastoma, chondromyxofibroma, osteoid osteoma and giant cell tumors; Nervous system: skull (osteoma, hemangioma, granuloma, xanthoma, osteitis defornians), meninges (meningioma, meningiosarcoma, gliomatosis), brain (astrocytoma, medulloblastoma, glioma, ependymoma, germinoma [pinealoma], glioblastoma multiform, oligodendroglioma, schwannoma, retinoblastoma, congenital tumors), spinal cord neurofibroma, meningioma, glioma, sarcoma); Gynecological: uterus (endometrial carcinoma), cervix (cervical carcinoma, pre-tumor cervical dysplasia), ovaries (ovarian carcinoma [serous cystadenocarcinoma, mucinous cystadenocarcinoma, unclassified carcinoma], granulosa-thecal cell tumors, Sertoli-Leydig cell tumors, dysgerminoma, malignant teratoma), vulva (squamous cell carcinoma, intraepithelial carcinoma, adenocarcinoma, fibrosarcoma, melanoma), vagina (clear cell carcinoma, squamous cell carcinoma, botryoid sarcoma (embryonal rhabdomyosarcoma], fallopian tubes (carcinoma); Hematologic: blood (myeloid leukemia [acute and chronic], acute lymphoblastic leukemia, chronic lymphocytic leukemia, myeloproliferative diseases, multiple myeloma, myelodysplastic syndrome), Hodgkin's disease, non-Hodgkin's lymphoma [malignant lymphoma]; Skin: malignant melanoma, basal cell carcinoma, squamous cell carcinoma, Karposi's sarcoma, moles dysplastic nevi, lipoma, angioma, dermatofibroma, keloids, psoriasis; Adrenal Glands: neuroblastoma; and breast cancer. Thus, the term "cancerous cell" as provided herein, includes a cell afflicted by any one of the above-identified conditions.

"Chemotherapeutic agent" includes, but is not limited to, an AKT inhibitor, an alkylating agent, an antimetabolite, an antimicrotubule agent, an aromatase inhibitor, a c-KIT inhibitor, a cMET inhibitor, an EGFR inhibitor, an ErbB2 inhibitor, a Flt-3 inhibitor, an HSP90 inhibitor, an IGF1R inhibitor, a platin, a Raf inhibitor, rapamycin, a Rapamycin analogue, a Receptor Tyrosine Kinase inhibitor, a taxane, a topoisomerase inhibitor, a SRC and/or ABL kinase inhibitor, and a VEGFR inhibitor. A pharmaceutically acceptable salt, solvate, and/or hydrate of a chemotherapeutic agent can be prepared by one of ordinary skill in the art and such salt, solvate, and/or hydrates thereof can be used to practice the invention.

"c-KIT inhibitor" includes, for example, imatinib, sunitinib, nilotinib, AMG 706, sorafenib, compounds in Table 3b, compounds in Table 3c, compounds in Table 8, compounds in Table 9, and compounds described in WO 2006/108059, WO/2005/020921, WO/2006/033943, and WO 2005/030140.

"cMET inhibitor" includes, for example, compounds in Table 3a, compounds in Table 3b, compounds in Table 3c, compounds described in WO06/108059, WO 2006/014325, and WO 2005/030140.

"EGFR inhibitor" includes, for example, one or more of the following: pelitinib, lapatinib (Tykerb®), gefitinib (Iressa®), erlotinib (Tarceva®), Zactima (ZD6474, vandetinib), AEE788 and HKI-272, EKB-569, CI-1033, N-(3,4-dichloro-2-fluorophenyl)-7-({[(3a*R*,5r,6a*S*)-2-methyloctahydrocyclopenta[c]pyrrol-5-yl]methyl}oxy)-6-(methyloxy)quinazolin-4-amine *N*-(4-bromo-3-chloro-2-fluorophenyl)-7-({[(3a*R*,5r,6a*S*)-2-methyloctahydrocyclo-penta[c]pyrrol-5-yl]methyl}oxy)-6-(methyloxy)quinazolin-4-amine, *N*-(3,4-dichloro-2-fluorophenyl)-7-({[(3a*R*,5s,6a*S*)-2-methyloctahydrocyclopenta[c]pyrrol-5-yl]methyl}oxy)-6-(methyloxy)quinazolin-4-amine, *N*-(4-bromo-3-chloro-2-fluorophenyl)-7-({[(3a*R*,5s,6a*S*)-2-methyloctahydrocyclo-penta[c]pyrrol-5-yl]methyl}oxy)-6-(methyloxy)quinazolin-4-amine, compounds in Table 4, compounds in Table 7, and compounds described in WO 2004/006846 and WO 2004/050681.

"ErbB2 inhibitor" includes, for example, lapatinib (GW572016), PKI-166, canertinib, CI-1033, HKI272, and EKB-569.

"Flt-3 inhibitor" includes, for example, CEP-701, PKC 412, MLN 518, sunitinib, sorafenib, compounds in Table 3a, compounds in Table 3b, compounds in Table 3c, compounds in Table 9, and compounds described in WO 2006/108059, WO/2006/033943, WO 2006/014325, and WO 2005/030140.

"Hormone therapy" and "hormonal therapy" include, for example, treatment with one or more of the following: steroids (e.g. dexamethasone), finasteride, tamoxifen, and an aromatase inhibitor.

"HSP90 inhibitor" includes, for example, 17-AAG, 17-DMAG, Geldanamycin, 5-(2,4-dihydroxy-5-isopropylphenyl)-N-ethyl-4-(4-(morpholinomethyl)phenyl)isoxazole-3-carboxamide [NVP-AUY922 (VER 52296)], 6-chloro-9-((4-methoxy-3,5-dimethylpyridin-2-yl)methyl)-9H-purin-2-amine (CNF2024, also named BIIB021), compounds disclosed in WO2004072051, compounds disclosed in WO2005028434, compounds disclosed in WO2007035620 and compounds disclosed in WO2006091963.

"IGF1R inhibitor" includes, for example, Tyrphostin AG 1024, compounds in Table 5a, compounds in Table 5b, and compounds described in WO06/074057.

"Kinase-dependent diseases or conditions" refer to pathologic conditions that depend on the activity of one or more lipid kinases. Kinases either directly or indirectly participate in the signal transduction pathways of a variety of cellular activities including proliferation, adhesion, migration, differentiation and invasion. Diseases associated with kinase activities include tumor growth, the pathologic neovascularization that supports solid tumor growth, and associated with other diseases where excessive local vascularization is involved such as ocular diseases (diabetic retinopathy, age-related macular degeneration, and the like) and inflammation (psoriasis, rheumatoid arthritis, and the like).

While not wishing to be bound to theory, phosphatases can also play a role in "kinase-dependent diseases or conditions" as cognates of kinases; that is, kinases phosphorylate and phosphatases dephosphorylate, for example lipid substrates. Therefore compounds of the invention, while modulating kinase activity as described herein, may also modulate, either directly or indirectly, phosphatase activity. This additional modulation, if present, may be synergistic (or not) to activity of compounds of the invention toward a related or otherwise interdependent kinase or kinase family. In any case, as stated previously, the compounds of the invention are useful for treating diseases characterized in part by abnormal levels of cell proliferation (*i.e.* tumor growth), programmed cell death (apoptosis), cell migration and invasion and angiogenesis associated with tumor growth.

"Metabolite" refers to the break-down or end product of a compound or its salt produced by metabolism or biotransformation in the animal or human body; for example, biotransformation to a more polar molecule such as by oxidation, reduction, or hydrolysis, or to a conjugate (see Goodman and Gilman, "The Pharmacological Basis of Therapeutics" 8.sup.th Ed., Pergamon Press, Gilman et al. (eds), 1990 for a discussion of biotransformation). As used herein, the metabolite of a compound of the invention or its salt may be the biologically active form of the compound in the body. In one example, a prodrug may be used such that the biologically active form, a metabolite, is released *in vivo.* In another example, a biologically active metabolite is discovered serendipitously, that is, no prodrug design *per se* was undertaken. An assay for activity of a metabolite of a compound of the present invention is known to one of skill in the art in light of the present disclosure.

"Patient" for the purposes of the present invention includes humans and other animals, particularly mammals, and other organisms. Thus the methods are applicable to both human therapy and veterinary applications. In another embodiment the patient is a mammal, and in another embodiment the patient is human.

A "pharmaceutically acceptable salt" of a compound means a salt that is pharmaceutically acceptable and that possesses the desired pharmacological activity of the parent compound. It is understood that the pharmaceutically acceptable salts are non-toxic. Additional information on suitable pharmaceutically acceptable salts can be found in Remington's Pharmaceutical Sciences, 17th ed., Mack Publishing Company, Easton, PA, 1985, which is incorporated herein by reference or S. M. Berge, et al., "Pharmaceutical Salts," J. Pharm. Sci., 1977;66:1-19.

Examples of pharmaceutically acceptable acid addition salts include those formed with inorganic acids such as hydrochloric acid, hydrobromic acid, sulfuric acid, nitric acid, phosphoric acid, and the like; as well as organic acids such as acetic acid, trifluoroacetic acid, propionic acid, hexanoic acid, cyclopentanepropionic acid, glycolic acid, pyruvic acid, lactic acid, oxalic acid, maleic acid, malonic acid, succinic acid, fumaric acid, tartaric acid, citric acid, benzoic acid, cinnamic acid, 3-(4-hydroxybenzoyl)benzoic acid, mandelic acid, methanesulfonic acid, ethanesulfonic acid, 1,2-ethanedisulfonic acid, 2-hydroxyethanesulfonic acid, benzenesulfonic acid, 4-chlorobenzenesulfonic acid, 2-naphthalenesulfonic acid, 4-toluenesulfonic acid, camphorsulfonic acid, glucoheptonic acid, 4,4'-methylenebis-(3-hydroxy-2-ene-1-carboxylic acid), 3-phenylpropionic acid, trimethylacetic acid, tertiary butylacetic acid, lauryl sulfuric acid, gluconic acid, glutamic acid, hydroxynaphthoic acid, salicylic acid, stearic acid, muconic acid, p-toluenesulfonic acid, and salicylic acid and the like.

Examples of a pharmaceutically acceptable base addition salts include those formed when an acidic proton present in the parent compound is replaced by a metal ion, such as sodium, potassium, lithium, ammonium, calcium, magnesium, iron, zinc, copper, manganese, aluminum salts and the like. Specific salts are the ammonium, potassium, sodium, calcium, and magnesium salts. Salts derived from pharmaceutically acceptable organic non-toxic bases include, but are not limited to, salts of primary, secondary, and *tertiary* amines, substituted amines including naturally occurring substituted amines, cyclic amines and basic ion exchange resins. Examples of organic bases include isopropylamine, trimethylamine, diethylamine, triethylamine, tripropylamine, ethanolamine, 2-dimethylaminoethanol, 2-diethylaminoethanol, dicyclohexylamine, lysine, arginine, histidine, caffeine, procaine, hydrabamine, choline, betaine, ethylenediamine, glucosamine, methylglucamine, theobromine, purines, piperazine, piperidine, N-ethylpiperidine, tromethamine, *N*-methylglucamine, polyamine resins, and the like. Exemplary organic bases are isopropylamine, diethylamine, ethanolamine, trimethylamine, dicyclohexylamine, choline, and caffeine."Platin(s)," and "platin-containing agents" include, for example, cisplatin, carboplatin, and oxaliplatin.

"Prodrug" refers to compounds that are transformed (typically rapidly) *in vivo* to yield the parent compound of the above formulae, for example, by hydrolysis in blood. Common examples include, but are not limited to, ester and amide forms of a compound having an active form bearing a carboxylic acid moiety. Examples of pharmaceutically acceptable esters of the compounds of this invention include, but are not limited to, alkyl esters (for example with between about one and about six carbons) the alkyl group is a straight or branched chain. Acceptable esters also include cycloalkyl esters and arylalkyl esters such as, but not limited to benzyl. Examples of pharmaceutically acceptable amides of the compounds of this invention include, but are not limited to, primary amides, and secondary and tertiary alkyl amides (for example with between about one and about six carbons). Amides and esters of the compounds of the present invention may be prepared according to conventional methods. A thorough discussion of prodrugs is provided in T. Higuchi and V. Stella, "Pro-drugs as Novel Delivery Systems," Vol 14 of the A.C.S. Symposium Series, and in Bioreversible Carriers in Drug Design, ed. Edward B. Roche, American Pharmaceutical Association and Pergamon Press, 1987.

"Raf inhibitor" includes, for example, sorafenib, RAF 265 (CHIR 265), compounds in Table 6, and compounds described in WO 2005/112932. These references also describe in vitro assays that can be used to determine the inhibitory activity of RAF.

"Rapamycin analogue" includes for example, CCI-779, AP 23573, RAD 001 , TAFA 93, and compounds described in WO 2004/101583 and US 7,160,867.

"Receptor Tyrosine Kinase inhibitor" includes, for example, inhibitors of AKT, EGFR, ErbB2, IGF1R, KIT, Met, Raf, and VEGFR2. Examples of receptor tyrosine kinase inhibitors can be found in WO 2006/108059 (US Nat'l Stage Application Serial No. 11/910,720), WO 2006/074057 (US Nat'l Stage Application Serial No. 11/722,719), WO 2006/071819 (US Nat'l Stage Application Serial No. 11/722,291), WO 2006/014325 (US Nat'l Stage Application Serial No. 11/571,140), WO 2005/117909 (US Nat'l Stage Application Serial No. 11/568,173), WO 2005/030140 (US Nat'l Stage Application Serial No. 10/573,336), WO 2004/050681 US Nat'l Stage Application Serial No. 10/533,555), WO 2005/112932 (US Nat'l Stage Application Serial No. 11/568,789), and WO 2004/006846 (US Nat'l Stage Application Serial No. 10/522,004). In particular, the applications cited in this paragraph are incorporated for the purpose of providing specific examples and generic embodiments (and the definitions associated with the terms used in the embodiments) of compounds that are useful in the practice of the invention. These references also describe in vitro assays useful in the practice of this invention.

"Taxane" includes, for example, one or more of the following: Paclitaxel (Taxol^{®}) and Docetaxel (Taxotere^{®}).

"Therapeutically effective amount" is an amount of a compound of the invention, that when administered to a patient, ameliorates a symptom of the disease. The amount of a compound of the invention which constitutes a "therapeutically effective amount" will vary depending on the compound, the disease state and its severity, the age of the patient to be treated, and the like. The therapeutically effective amount can be determined routinely by one of ordinary skill in the art having regard to their knowledge and to this disclosure.

"Topoisomerase inhibitor" includes, for example, one or more of the following: amsacrine, camptothecin, etoposide, etoposide phosphate, exatecan, irinotecan, lurtotecan, and teniposide, and topotecan.

"Treating" or "treatment" of a disease, disorder, or syndrome, as used herein, includes (i) preventing the disease, disorder, or syndrome from occurring in a human, i.e. causing the clinical symptoms of the disease, disorder, or syndrome not to develop in an animal that may be exposed to or predisposed to the disease, disorder, or syndrome but does not yet experience or display symptoms of the disease, disorder, or syndrome; (ii) inhibiting the disease, disorder, or syndrome, *i.e.*, arresting its development; and (iii) relieving the disease, disorder, or syndrome, *i.e.*, causing regression of the disease, disorder, or syndrome. As is known in the art, adjustments for systemic versus localized delivery, age, body weight, general health, sex, diet, time of administration, drug interaction and the severity of the condition may be necessary, and will be ascertainable with routine experimentation by one of ordinary skill in the art. In another embodiment, "treating" or "treatment" of a disease, disorder, or syndrome, as used herein, includes (i) inhibiting the disease, disorder, or syndrome, *i.e.*, arresting its development; and (ii) relieving the disease, disorder, or syndrome, *i.e.*, causing regression of the disease, disorder, or syndrome.

"SRC and/or ABL kinase inhibitor" includes, for example, dasatinib, imatinib (Gleevec®), and compounds described in WO 2006/074057.

"VEGFR inhibitor" includes, for example, one or more of the following: VEGF Trap, ZD6474 (vandetanib, Zactima), sorafenib, Angiozyme, AZD2171 (cediranib), pazopanib, sorafenib, axitinib, SU5416 (semaxanib), PTK787 (vatalanib), AEE778, RAF 265, sunitinib (Sutent), *N*-(3,4-dichloro-2-fluorophenyl)-7-({[(3a*R*,5r,6a*S*)-2-methyloctahydrocyclopenta[c]pyrrol-5-yl]methyl}oxy)-6-(methyloxy)quinazolin-4-amine, *N*-(4-bromo-3-chloro-2-fluorophenyl)-7-({[(3a*R*,5r,6a*S*)-2-methyloctahydrocyclopenta[c]pyrrol-5-yl]methyl}oxy)-6-(methyloxy)quinazolin-4-amine, *N-*(3,4-dichloro-2-fluorophenyl)-7-({[(3a*R*,5s,6a*S*)-2-methyloctahydrocyclopenta[c]pyrrol-5-yl]methyl}oxy)-6-(methyloxy)quinazolin-4-amine, *N*-(4-bromo-3-chloro-2-fluorophenyl)-7-({[(3a*R*,5s,6a*S*)-2-methyloctahydrocyclo-penta[c]pyrrol-5-yl]methyl}oxy)-6-(methyloxy)quinazolin-4-amine, compounds in Table 7, and compounds described in WO 2004/050681 and WO 2004/006846.

### Embodiments of the Invention

The following paragraphs present a number of embodiments of compounds that can be used to practice the invention. In each instance, the embodiment includes both of the recited compounds as well as individual isomers and mixtures of isomers. In addition, in each instance, the embodiment includes the pharmaceutically acceptable salts, hydrates, and/or solvates of the recited compounds and any individual isomers or mixture of isomers thereof.

One embodiment (A) of the Invention is directed to a Compound of Formula (III) of Formula (IV): 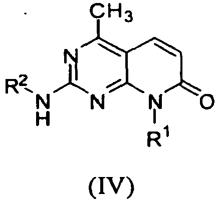 or where the Compound is optionally as a pharmaceutically acceptable salt and additionally optionally as a solvate and additionally optionally as hydrate thereof; where R¹ and R² are as defined in the Summary of the Invention for a Compound of Formula (III).

Another embodiment (B) of the Invention is directed to a Compound of Formula (III) of Formula (V): 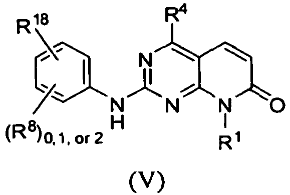 or where the Compound is optionally as a pharmaceutically acceptable salt and additionally optionally as a solvate and additionally optionally as hydrate thereof; where R¹, R⁴, R⁸, and R¹⁸ are as defined in the Summary of the Invention for a Compound of Formula (III).

Another embodiment (C) of the Invention is directed to a Compound of Formula (III) of Formula (VI): 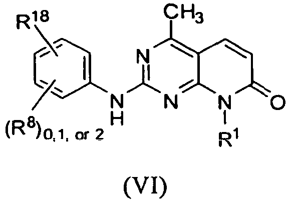 or where the Compound is optionally as a pharmaceutically acceptable salt and additionally optionally as a solvate and additionally optionally as hydrate thereof; where R¹, R⁸, and R¹⁸ are as defined in the Summary of the Invention for a Compound of Formula (III).

Another embodiment (D) of the Invention is directed to a Compound of Formula (III) of Formula (VII): 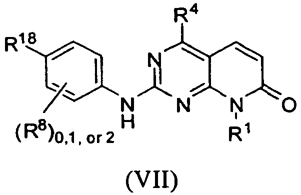 or where the Compound is optionally as a pharmaceutically acceptable salt and additionally optionally as a solvate and additionally optionally as hydrate thereof; where R¹, R⁴, R⁸, and R¹⁸ are as defined in the Summary of the Invention for a Compound of Formula (III).

Another embodiment (E) of the Invention is directed to a Compound of Formula (III) of Formula (VIII): 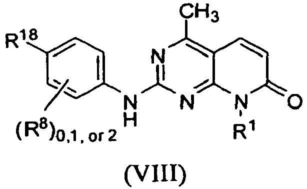 or where the Compound is optionally as a pharmaceutically acceptable salt and additionally optionally as a solvate and additionally optionally as hydrate thereof; where R¹, R⁸, and R¹⁸ are as defined in the Summary of the Invention for a Compound of Formula (III).

Another embodiment (F) of the Invention is directed to a Compound of Formula (III), (IV), (V), (VI), (VII), or (VIII), wherein R¹ is optionally substituted alkyl or optionally substituted cycloalkyl; or a single isomer thereof, where the Compound is optionally as a pharmaceutically acceptable salt and additionally optionally as a solvate and additionally optionally as hydrate thereof; and all other groups in Formula (III), (IV), (V), (VI), (VII), and (VIII) are as defined in the Summary of the Invention for a Compound of Formula (III).

Another embodiment (G) of the Invention is directed to a Compound of Formula (III), (IV), (V), (VI), (VII), or (VIII), or a single isomer thereof, where the Compound is optionally as a pharmaceutically acceptable salt and additionally optionally as a solvate and additionally optionally as hydrate thereof, and where
- R¹⁸: is -L-R⁹, -C(O)NR²³R^{23a}, or -N(R¹²)C(O)R¹³;
- L: is optionally substituted heterocycloalkyl;
- R⁹: is -C(O)NHR¹⁰, -C(O)R¹⁵, -S(O)₂R¹⁶, or -S(O)₂NR¹⁷R¹⁹;
- R¹⁰: is alkyl, optionally substituted cycloalkyl, optionally substituted cycloalkylalkyl, optionally substituted aryl, optionally substituted arylalkyl, optionally substituted heterocycloalkyl, optionally substituted heterocycloalkylalkyl, optionally substituted heteroaryl, or optionally substituted heteroarylalkyl;
- R¹²: is hydrogen or alkyl;
- R¹³, R¹⁵, R¹⁶, and R¹⁹: are optionally substituted alkyl;
- R¹⁷: is hydrogen or optionally substituted alkyl;
- R²³: is hydrogen or alkyl; and
- R^{23a}: is substituted cycloalkyl, substituted heterocycloalkyl, substituted heteroaryl, or substituted aryl;
and all other groups in Formula (III), (IV), (V), (VI), (VII), and (VIII) are as defined in the Summary of the Invention for a Compound of Formula (III).

Another embodiment (H) of the Invention is directed to a Compound of Formula (III), (IV), (V), (VI), (VII), or (VIII), wherein R¹⁸ is -L-R⁹ and L is optionally substituted heterocycloalkyl and R⁹ is -C(O)R¹⁵ where R¹⁵ is optionally substituted alkyl; or a single isomer thereof, where the Compound is optionally as a pharmaceutically acceptable salt and additionally optionally as a solvate and additionally optionally as hydrate thereof; and all other groups in Formula (III), (IV), (V), (VI), (VII), and (VIII) are as defined in the Summary of the Invention for a Compound of Formula (III).

Another embodiment (Q) of the Invention is directed to a Compound of Formula (III), (IV), (V), (VI), (VII), or (VIII), wherein R¹⁸ is -NHC(O)R¹³ and R¹³ is optionally substituted alkyl; or a single isomer thereof, where the Compound is optionally as a pharmaceutically acceptable salt and additionally optionally as a solvate and additionally optionally as hydrate thereof; and all other groups in Formula (III), (IV), (V), (VI), (VII), and (VIII) are was defined in the Summary of the Invention for a Compound of Formula (III).

Another embodiment (J) of the Invention is directed to a Compound of Formula (III), (IV), (V), (VI), (VII), or (VIII), where R¹⁸ is -C(O)NHR^{23a} where R^{23a} is substituted aryl, substituted heteroaryl or substituted heterocyloalkyl; or a single isomer thereof, where the Compound is optionally as a pharmaceutically acceptable salt and additionally optionally as a solvate and additionally optionally as hydrate thereof; and all other groups in Formula (III), (IV), (V), (VI), (VII), and (VIII) are as defined in the Summary of the Invention for a Compound of Formula (III).

Another embodiment (K) of the Invention is directed to a Compound of Formula (III), (IV), (V), (VI), (VII), or (VIII), wherein R¹ is optionally substituted alkyl or optionally substituted cycloalkyl; and R¹⁸ is -L-R⁹ and L is optionally substituted heterocycloalkyl and R⁹ is -C(O)R¹⁵ and R¹⁵ is optionally substituted alkyl; or a single isomer thereof, where the Compound is optionally as a pharmaceutically acceptable salt and additionally optionally as a solvate and additionally optionally as hydrate thereof; and all other groups in Formula (III), (IV), (V), (VI), (VII), and (VIII) are as defined in the Summary of the Invention for a Compound of Formula (III).

Another embodiment (R) of the Invention is directed to a Compound of Formula (III), (IV), (V), (VI), (VII), or (VIII), wherein R¹ is optionally substituted alkyl or optionally substituted cycloalkyl; and R¹⁸ is -N(H)C(O)R¹³ and R¹³ is optionally substituted alkyl; or a single isomer thereof, where the Compound is optionally as a pharmaceutically acceptable salt and additionally optionally as a solvate and additionally optionally as hydrate thereof; and all other groups in Formula (III), (IV), (V), (VI), (VII), and (VIII) are as defined in the Summary of the Invention for a Compound of Formula (III).

Another embodiment (L) of the Invention is directed to a Compound of Formula (III), (IV), (V), (VI), (VII), or (VIII), wherein R¹ is optionally substituted alkyl or optionally substituted cycloalkyl; and R¹⁸ is -C(O)NHR^{23a} where R^{23a} is substituted aryl, substituted heteroaryl or substituted heterocyloalkyl; or a single isomer thereof, where the Compound is optionally as a pharmaceutically acceptable salt and additionally optionally as a solvate and additionally optionally as hydrate thereof; and all other groups in Formula (III), (IV), (V), (VI), (VII), and (VIII)) are as defined in the Summary of the Invention for a Compound of Formula (III).

Also described is a Compound of Formula II where R³² is cyclopentyl; R²⁸ is R^{28a} and R^{28a} is thiazol-2-yl or thiazol-5-yl and R²⁹ is R^{29a} and R^{29a} is unsubstituted piperazinyl or piperidinyl; where the Compound is optionally as a single isomer thereof and additionally optionally as a pharmaceutically acceptable salt and additionally optionally as a solvate and additionally optionally as hydrate thereof. In another embodiment, the invention is directed to a Compound of Formula II where R³² is cyclopentyl; R²⁸ is R^{28a} and R^{28a} is thiazol-5-yl and R²⁹ is R^{29a} and R^{29a} is piperazinyl or piperidinyl where the piperazinyl and piperidinyl are optionally substituted with one group selected from alkyl and alkoxycarbonyl; where the Compound is optionally as a single isomer thereof and additionally optionally as a pharmaceutically acceptable salt and additionally optionally as a solvate and additionally optionally as hydrate thereof.

Also described is a Compound of Formula II where R³² is ethyl; R²⁸ is R^{28b} and R^{28b} is imidazoyl and R²⁹ is R^{29b} and R^{29b} is pyrazolyl; where the Compound is optionally as a single isomer thereof and additionally optionally as a pharmaceutically acceptable salt and additionally optionally as a solvate and additionally optionally as hydrate thereof.

Also described is a Compound of formula I where A is =CH- or =N-; R²⁰ is alkyl, cycloalkyl, or heterocycloalkyl; R²¹ is pyrazolyl, thiadiazolyl, -C(O)OH, -C(O)NHR²³ or -C(O)R²⁵; R²² is hydrogen or halo; R²³ is alkyl or phenylmethyl; and R²⁵ is piperidinyl or piperazinyl where the piperazinyl and piperidinyl are optionally substituted with one group selected from alkyl and alkoxycarbonyl; where the Compound is optionally as a single isomer thereof and additionally optionally as a pharmaceutically acceptable salt and additionally optionally as a solvate and additionally optionally as hydrate thereof.

Also described is a Compound of Formula I where A is =CH- or =N-; R²⁰ is alkyl, cycloalkyl, or heterocycloalkyl; R²¹ is pyrazolyl; and R²² is hydrogen or halo; where the Compound is optionally as a single isomer thereof and additionally optionally as a pharmaceutically acceptable salt and additionally optionally as a solvate and additionally optionally as hydrate thereof. Another embodiment of the invention is directed to a Compound of Formula I where A is =CH- or =N-; R²⁰ is ethyl or cyclopentyl; R²¹ is pyrazolyl; and R²² is hydrogen; where the Compound is optionally as a single isomer thereof and additionally optionally as a pharmaceutically acceptable salt and additionally optionally as a solvate and additionally optionally as hydrate thereof. Another embodiment of the invention is directed to a Compound of Formula I where A is =CH- or =N-; R²⁰ is cycloalkyl; R²¹ is pyrazolyl; and R²² is hydrogen or halo; where the Compound is optionally as a single isomer thereof and additionally optionally as a pharmaceutically acceptable salt and additionally optionally as a solvate and additionally optionally as hydrate thereof.

Also described is a Compound of Formula I where A is =CH- or N-; R²⁰ is alkyl, cycloalkyl, or heterocycloalkyl; R²¹ is thiadiazolyl; and R²² is hydrogen or halo; where the Compound is optionally as a single isomer thereof and additionally optionally as a pharmaceutically acceptable salt and additionally optionally as a solvate and additionally optionally as hydrate thereof. Another embodiment of the invention is directed to a Compound of Formula I where A is =CH- or N-; R²⁰ is cycloalkyl; R²¹ is thiadiazolyl; and R²² is hydrogen or halo; where the Compound is optionally as a single isomer thereof and additionally optionally as a pharmaceutically acceptable salt and additionally optionally as a solvate and additionally optionally as hydrate thereof.

Also described is a Compound of Formula I where A is =CH- or N-; R²⁰ is alkyl, cycloalkyl, or heterocycloalkyl; R²¹ is -C(O)OH; R²² is hydrogen or halo; where the Compound is optionally as a single isomer thereof and additionally optionally as a pharmaceutically acceptable salt and additionally optionally as a solvate and additionally optionally as hydrate thereof. Another embodiment of the invention is directed to a Compound of Formula I where A is =CH- or =N-; R²⁰ is ethyl, cyclopentyl, or tetrahydrofuranyl; R²¹ is -C(O)OH; R²² is hydrogen or halo; where the Compound is optionally as a single isomer thereof and additionally optionally as a pharmaceutically acceptable salt and additionally optionally as a solvate and additionally optionally as hydrate thereof.

Also described is a Compound of Formula I where A is =CH- or =N-; R²⁰ is alkyl, cycloalkyl, or heterocycloalkyl; R²¹ is -C(O)NHR²³; R²² is hydrogen or halo; and R²³ is alkyl or phenylmethyl; where the Compound is optionally as a single isomer thereof and additionally optionally as a pharmaceutically acceptable salt and additionally optionally as a solvate and additionally optionally as hydrate thereof. Another embodiment of the invention is directed to a Compound of Formula I where A is =CH- or =N-, R²⁰ is cycloalkyl, R²¹ is -C(O)NHR²³, R²² is hydrogen or halo, and R²³ is alkyl or phenylmethyl; where the Compound is optionally as a single isomer thereof and additionally optionally as a pharmaceutically acceptable salt and additionally optionally as a solvate and additionally optionally as hydrate thereof.

Also described is a Compound of Formula I where A is =CH- or =N-; R²⁰ is alkyl, cycloalkyl, or heterocycloalkyl; R²¹ is -C(O)R²⁵; R²² is hydrogen or halo; and R²⁵ is piperidinyl or piperazinyl where the piperazinyl and piperidinyl are optionally substituted with one group selected from alkyl and alkoxycarbonyl; where the Compound is optionally as a single isomer thereof and additionally optionally as a pharmaceutically acceptable salt and additionally optionally as a solvate and additionally optionally as hydrate thereof.

Also described is a Compound of Formula I where A is =CH- or =N-; R²⁰ is alkyl, cycloalkyl, or heterocycloalkyl; R²¹ is -C(Q)R²⁵; R²² is hydrogen or halo; and R²⁵ is piperazinyl where the piperazinyl is optionally substituted with one group selected from alkyl and alkoxycarbonyl; where the Compound is optionally as a single isomer thereof and additionally optionally as a pharmaceutically acceptable salt and additionally optionally as a solvate and additionally optionally as hydrate thereof. Another embodiment of the invention is directed to a Compound of Formula I where A is =CH- or N-; R²⁰ is cycloalkyl; R²¹ is -C(O)R²⁵; R²² is hydrogen or halo; and R²⁵ is piperazinyl where the piperazinyl is optionally substituted with one group selected from alkyl and alkoxycarbonyl; where the Compound is optionally as a single isomer thereof and additionally optionally as a pharmaceutically acceptable salt and additionally optionally as a solvate and additionally optionally as hydrate thereof. Another embodiment of the invention is directed to a Compound of Formula I where A is =CH- or =N-; R²⁰ is alkyl, cycloalkyl, or heterocycloalkyl; R²¹ is -C(O)R²⁵; R²² is hydrogen or halo; and R²⁵ is piperazinyl where the piperazinyl is optionally substituted with one group selected from methyl, ethyl, or tert-butoxycarbonyl; where the Compound is optionally as a single isomer thereof and additionally optionally as a pharmaceutically acceptable salt and additionally optionally as a solvate and additionally optionally as hydrate thereof.

Also described is a Compound of Formula I where A is =CH- or =N-; R²⁰ is alkyl, cycloalkyl, or heterocycloalkyl; R²¹ is -C(O)R²⁵; R²² is hydrogen or halo; and R²⁵ is piperidinyl where the piperidinyl is optionally substituted with one group selected from alkyl and alkoxycarbonyl; where the Compound is optionally as a single isomer thereof and additionally optionally as a pharmaceutically acceptable salt and additionally optionally as a solvate and additionally optionally as hydrate thereof. Another embodiment of the invention is directed to a Compound of Formula I where A is =CH- or N-, R²⁰ is cycloalkyl, R²¹ is -C(O)R²⁵, R²² is hydrogen, and R²⁵ is piperidinyl where the piperidinyl is optionally substituted with one group selected from alkyl and alkoxycarbonyl; where the Compound is optionally as a single isomer thereof and additionally optionally as a pharmaceutically acceptable salt and additionally optionally as a solvate and additionally optionally as hydrate thereof.

Also described is a Compound of Formula I where A is =CH- or =N-, R²⁰ is cycloalkyl or heterocycloalkyl, R²¹ is piperidinyl (where the piperidinyl is optionally substituted with one group selected from alkyl and alkoxycarbonyl), and R²² is hydrogen or halo; where the Compound is optionally as a single isomer thereof and additionally optionally as a pharmaceutically acceptable salt and additionally optionally as a solvate and additionally optionally as hydrate thereof. In another embodiment, the invention is directed to a Compound of Formula I where A is =CH- or N-, R²⁰ is cycloalkyl, R²¹ is piperidinyl (where the piperidinyl is optionally substituted with one group selected from alkyl and alkoxycarbonyl), and R²² is halo; where the Compound is optionally as a single isomer thereof and additionally optionally as a pharmaceutically acceptable salt and additionally optionally as a solvate and additionally optionally as hydrate thereof.

Also described is a Compound of Formula I where A is =CH- or =N-, R²⁰ is alkyl, R²¹ is piperidinyl (where the piperidinyl is optionally substituted with one group selected from alkyl and alkoxycarbonyl), and R²² is hydrogen; where the Compound is optionally as a single isomer thereof and additionally optionally as a pharmaceutically acceptable salt and additionally optionally as a solvate and additionally optionally as hydrate thereof.

Also described is a Compound of Formula I where A is =CH- or =N-; R²⁰ is alkyl, cycloalkyl, or heterocycloalkyl; R²¹ is imidazolyl; and R²² is bromo; where the Compound is optionally as a single isomer thereof and additionally optionally as a pharmaceutically acceptable salt and additionally optionally as a solvate and additionally optionally as hydrate thereof. In another embodiment, the invention is directed to a Compound of Formula I where A is =CH- or =N-; R²⁰ is alkyl; R²¹ is imidazolyl; and R²² is bromo; where the Compound is optionally as a single isomer thereof and additionally optionally as a pharmaceutically acceptable salt and additionally optionally as a solvate and additionally optionally as hydrate thereof.

Also described is a Compound of Formula I where A is =CH- or =N-; R²⁰ is alkyl or heterocycloalkyl; R²¹ is amino, alkylamino, or dialkylamino; and R²² is hydrogen or halo; where the Compound is optionally as a single isomer thereof and additionally optionally as a pharmaceutically acceptable salt and additionally optionally as a solvate and additionally optionally as hydrate thereof. In another embodiment, the invention is directed to a Compound of Formula I where A is =CH- or =N-; R²⁰ is alkyl; R²¹ is amino, alkylamino, or dialkylamino; and R²² is hydrogen; where the Compound is optionally as a single isomer thereof and additionally optionally as a pharmaceutically acceptable salt and additionally optionally as a solvate and additionally optionally as hydrate thereof.

Also described is a Compound of Formula I where A is =CH- or N-, R²⁰ is alkyl or heterocycloalkyl, R²¹ is imidazolyl, and R²² is hydrogen, where the Compound is optionally as a single isomer thereof and additionally optionally as a pharmaceutically acceptable salt and additionally optionally as a solvate and additionally optionally as hydrate thereof. In another embodiment, the invention is directed to a Compound of Formula I where A is =CH- or =N-, R²⁰ is alkyl, R²¹ is imidazolyl, and R²² is hydrogen; where the Compound is optionally as a single isomer thereof and additionally optionally as a pharmaceutically acceptable salt and additionally optionally as a solvate and additionally optionally as hydrate thereof.

Also described is a Compound of Formula I where A is =CH- or =N-; R²⁰ is cycloalkyl; R²¹ is amino, alkylamino, dialkylamino, or piperazinyl (optionally substituted with one group selected from alkyl and alkoxycarbonyl); and R²² is halo; where the Compound is optionally as a single isomer thereof and additionally optionally as a pharmaceutically acceptable salt and additionally optionally as a solvate and additionally optionally as hydrate thereof. In another embodiment, the invention is directed to a Compound of Formula I where A is =CH- or =N-; R²⁰ is cycloalkyl; R²¹ piperazinyl optionally substituted with one group selected from alkyl and alkoxycarbonyl; and R²² is halo; where the Compound is optionally as a single isomer thereof and additionally optionally as a pharmaceutically acceptable salt and additionally optionally as a solvate and additionally optionally as hydrate thereof. In another embodiment, the invention is directed to a Compound of Formula I where A is =CH- or =N-; R²⁰ is cyclopentyl; R²¹ piperazinyl optionally substituted with one group selected from methyl, ethyl, and *tert*-butoxycarbonyl; and R²² is bromo; where the Compound is optionally as a single isomer thereof and additionally optionally as a pharmaceutically acceptable salt and additionally optionally as a solvate and additionally optionally as hydrate thereof.

Also described is a Compound of Formula I where A is =CH- or =N-, R²⁰ is ethyl, R²¹ is piperazinyl optionally substituted with one group selected from alkyl and alkoxycarbonyl, and R²² is hydrogen; where the Compound is optionally as a single isomer thereof and additionally optionally as a pharmaceutically acceptable salt and additionally optionally as a solvate and additionally optionally as hydrate thereof. In another embodiment, the invention is directed to a Compound of Formula I where A is =CH- or =N-, and R²⁰ is ethyl, R²¹ is piperazinyl optionally substituted with one alkyl, and R²² is hydrogen; where the Compound is optionally as a single isomer thereof and additionally optionally as a pharmaceutically acceptable salt and additionally optionally as a solvate and additionally optionally as hydrate thereof.

Also described is a Compound of Formula I where A is =CH- or =N-, R²⁰ is heterocycloalkyl, R²¹ is piperazinyl optionally substituted with one group selected from alkyl and alkoxycarbonyl, and R²² is hydrogen; where the Compound is optionally a single isomer thereof and additionally optionally as a pharmaceutically acceptable salt and additionally optionally as a solvate and additionally optionally as hydrate thereof

Another embodiment (M) of the Invention provides a pharmaceutical composition which comprises a compound of the invention optionally as a pharmaceutically acceptable salt and additionally optionally as a solvate or hydrate thereof and a pharmaceutically acceptable carrier, excipient, or diluent.

Also described is a method of treating disease, disorder, or syndrome where the disease is associated with uncontrolled, abnormal, and/or unwanted cellular activities effected directly or indirectly by PI3Kα which method comprises administering to a human in need thereof a therapeutically effective amount of a compound of Formula I, II, (III), (IV), (V), (VI), (VII), or (VIII) or a compound selected from Table 1 and Table 2 and Table 3 where the compound is optionally as a pharmaceutically acceptable salt and additionally optionally as a solvate or hydrate thereof, or a pharmaceutical composition thereof. In another embodiment the Compound is selected from Table 2 and Table 3.

Also described as embodiment (P) is a method of treating a disease, disorder, or syndrome which method comprises administering to a patient a therapeutically effective amount of a compound of Formula I, II, (III), (IV), (V), (VI), (VII), or (VIII) or a compound selected from Table 1 and Table 2 and Table 3, where the Compound is optionally as a pharmaceutically acceptable salt, optionally as a solvate or hydrate, and optionally as a pharmaceutical composition thereof; and a pharmaceutically acceptable carrier, excipient, or diluent.

In another embodiment of (P), the disease is cancer. In another embodiment, the cancer is breast cancer, colon cancer, rectal cancer, endometrial cancer, gastric carcinoma (including gastrointestinal carcinoid tumors and gastrointestinal stromal tumors), glioblastoma, hepatocellular carcinoma, small cell lung cancer, non-small cell lung cancer, melanoma, ovarian cancer, cervical cancer, pancreatic cancer, prostate carcinoma, acute myelogenous leukemia (AML), chronic myelogenous leukemia (CML), non-Hodgkin's lymphoma, or thyroid carcinoma. In another embodiment, the cancer is ovarian cancer, cervical cancer, breast cancer, colon cancer, rectal cancer, or glioblastoma. In another embodiment the Compound is selected from Table 2 and Table 3.

Also described as embodiment (T) is a method of treating cancer which method comprises administering to a patient a therapeutically effective amount of a compound of Formula I, II, (III), (IV), (V), (VI), (VII), or (VIII) or a Compound selected from Table 1 and Table 2 and Table 3, optionally as a pharmaceutically acceptable salt and additionally optionally as a solvate or hydrate thereof, or a pharmaceutical composition comprising a therapeutically effective amount of a compound of Formula I, II, (III), (IV), (V), (VI), (VII), or (VIII) and a pharmaceutically acceptable carrier, excipient, or diluent in combination with one or more chemotherapeutic agents.

In another embodiment of embodiment (T), one or more of the chemotherapeutic agents are independently selected from rapamycin, a rapamycin analogue, an alkylating agent, a taxane, a platin, an EGFR inhibitor, and an ErbB2 inhibitor. In another embodiment, one or two of the chemotherapeutic agents is independently selected from rapamycin, temozolomide, paclitaxel, docetaxel, carboplatin, cisplatin, oxaliplatin, gefitinib (Iressa®), erlotinib (Tarceva®), Zactima (ZD6474), HKI-272, pelitinib, canertinib, and lapatinib. In another embodiment, one or two of the chemotherapeutic agents are independently selected from rapamycin, temozolomide, paclitaxel, docetaxel, carboplatin, trastuzumab, erlotinib, and lapatinib. In another embodiment, one or two of the chemotherapeutic agents are independently selected from rapamycin, paclitaxel, carboplatin, and erlotinib.

In another embodiment of embodiment (T), one or more of the chemotherapeutic agents are independently selected from a platin and a taxane. In another embodiment, one or two of the chemotherapeutic agents is independently selected from carboplatin, cisplatin, oxaliplatin, and paclitaxel.

In another embodiment of embodiment (T), one or more of the chemotherapeutic agents is an EGFR inhibitor. In another embodiment, one or two of the chemotherapeutic agents is an EGFR inhibitor. In another embodiment, one of the chemotherapeutic agent is an EGFR inhibitor selected from lapatinib (Tykerb®), gefitinib (Iressa®), erlotinib (Tarceva®), Zactima (ZD6474), AEE778, HKI-272, EKB-569, and CI1033.

In another embodiment of embodiment (T), one or more of the chemotherapeutic agents is an ErbB2 inhibitor. In another embodiment, one of the chemotherapeutic agents is an ErbB2 inhibitor selected from lapatinib, EXB-569, HKI272, CI1033, and PKI-166.

In another embodiment of embodiment (T), one or more of the chemotherapeutic agents is selected from rapamycin, CCI-779, AP23573, RAD 001, TAFA 93, PI103, and SF1126. In another embodiment, one of the chemotherapeutic agents is rapamycin.

Also described is a method of treating cancer which method comprises administering to a patient a therapeutically effective amount of a compound of Formula I, II, (III), (IV), (V), (VI), (VII), or (VIII) or a Compound selected from Table 1 and Table 2 and Table 3 optionally additionally as a pharmaceutically acceptable salt, solvate, and/or hydrate thereof, or a pharmaceutical composition comprising a therapeutically effective amount of a compound of Formula I, II, (III), (IV), (V), (VI), (VII), or (VIII) or a Compound selected from Table 1 and Table 2 and Table 3 and a pharmaceutically acceptable carrier, excipient, or diluent in combination with radiation.

Also described is a method of treating cancer which method comprises administering to a patient a therapeutically effective amount of a compound of Formula I, II, (III), (IV), (V), (VI), (VII), or (VIII) or a Compound selected from Table 1 and Table 2 and Table 3 optionally as a pharmaceutically acceptable salt, solvate, and/or hydrate thereof, or a pharmaceutical composition comprising a therapeutically effective amount of a compound of Formula I, II, (III), (IV), (V), (VI), (VII), or (VIII) or a Compound selected from Table 1 and Table 2 and Table 3 and a pharmaceutically acceptable carrier, excipient, or diluent in combination with one or more antibodies.

Also described is a method of treating cancer which method comprises administering to a patient a therapeutically effective amount of a compound of Formula I, II, (III), (IV), (V), (VI), (VII), or (VIII) or a Compound selected from Table 1 and Table 2 and Table 3 optionally as a pharmaceutically acceptable salt, solvate and/or hydrate thereof, or a pharmaceutical composition comprising a therapeutically effective amount of a compound of Formula I, II, (III), (IV), (V), (VI), (VII), or (VIII) or a Compound selected from Table 1 and Table 2 and Table 3 and a pharmaceutically acceptable carrier, excipient, or diluent in combination with surgery.

Also described is a method of treating cancer which method comprises administering to a patient a therapeutically effective amount of a compound of Formula I, II, (III), (IV), (V), (VI), (VII), or (VIII) or a Compound selected from Table 1 and Table 2 and Table 3 optionally as a pharmaceutically acceptable salt, solvate, and/or hydrate thereof, or a pharmaceutical composition comprising a therapeutically effective amount of a compound of Formula I, II, (III), (IV), (V), (VI), (VII), or (VIII) or a Compound selected from Table 1 and Table 2 and Table 3 and a pharmaceutically acceptable carrier, excipient, or diluent in combination with one or more hormone therapies.

Also described is a method of treating acute myelogenous leukemia which method comprises administering to a patient a therapeutically effective amount of a compound of Formula I, II, (III), (IV), (V), (VI), (VII), or (VIII) or a Compound selected from Table 1 and Table 2 and Table 3 optionally as a pharmaceutically acceptable salt, solvate, and/or hydrate thereof, or a pharmaceutical composition comprising a therapeutically effective amount of a compound of Formula I, II, (III), (IV), (V), (VI), (VII), or (VIII) or a Compound selected from Table 1 and Table 2 and Table 3 and a pharmaceutically acceptable carrier, excipient, or diluent; in combination with one or more of treatments selected from bone marrow or peripheral blood stem cell transplantation, radiation, one or more antibodies, and one or more chemotherapeutic agents. In another embodiment, the antibody is selected from Gemtuzumab ozogamicin (Mylotarg), ^{α}IGF-1R A12 MoAb, ^{α}IGF-1R 19D12 MoAb, ^{α}IGF-1R h7C10 MoAb, ^{α}IGF-1R CP-751871 MoAb and trastuzumab. In another embodiment, one or two of the chemotherapeutic agents is selected from Imatinib (i.e. Gleevec®), PKC412, CEP-701, daunorubicin, doxorubicin, cytarabine (ara-C), an anthracycline drug such as daunorubicin or idarubicin (Daunomycin, Idamycin), 6-thioguanine, and a granulocyte colony-stimulating factor such as Neupogen or Leukine.

Also described is a method of treating chronic myelogenous leukemia (CML) which method comprises administering to a patient a therapeutically effective amount of a compound of Formula I, II, (III), (IV), (V), (VI), (VII), or (VIII) or a Compound selected from Table 1 and Table 2 and Table 3 optionally as a pharmaceutically acceptable salt, solvate, and/or hydrate thereof, or a pharmaceutical composition comprising a therapeutically effective amount of a compound of Formula I, II, (III), (IV), (V), (VI), (VII), or (VIII) or a Compound selected from Table 1 and Table 2 and Table 3 and a pharmaceutically acceptable carrier, excipient, or diluent in combination with one or more of treatments selected from bone marrow or peripheral blood stem cell transplantation, radiation, one or more chemotherapeutic agents, immunotherapy, and one or more antibodies. In another embodiment, one or two of the chemotherapeutic agents is selected from Imatinib (i.e. Gleevec®), PKC412, hydroxyurea (Hydrea), cytosine, cytosine arabinoside, dasatinib, AMN107, VX680 (MK0457), and cytarabine (ara-C); in another embodiment, one or more of the chemotherapeutic agents is selected from Imatinib (i.e. Gleevec®) and dasatinib. In another embodiment, the immunotherapy is selected from interferon therapy such as interferon-α.

Also described is a method of treating prostate cancer which method comprises administering to a patient a therapeutically effective amount of a compound of Formula I, II, (III), (IV), (V), (VI), (VII), or (VIII) or a Compound selected from Table 1 and Table 2 and Table 3 optionally as a pharmaceutically acceptable salt, solvate, and/or hydrate thereof, or a pharmaceutical composition comprising a therapeutically effective amount of a compound of Formula I, II, (III), (IV), (V), (VI), (VII), or (VIII) or a Compound selected from Table 1 and Table 2 and Table 3 and a pharmaceutically acceptable carrier, excipient, or diluent in combination with one or more of treatments selected from surgery (including cryosurgery), radiation, one or more chemotherapeutic agents, one or more antibodies, and one or more hormone therapies. In another embodiment, one or two of the antibodies is ^{α}IGF-1R A12 MoAb, ^{α}IGF-1R 19D12 MoAb, ^{α}IGF-1R h7C10 MoAb, ^{α}IGF-1R CP-751871 MoAb. In another embodiment, one or two of the chemotherapeutic agents is selected from rapamycin, mitoxantrone, prednisone, docetaxel (Taxotere), doxorubicin, etoposide, vinblastine, paclitaxel, and carboplatin. In another embodiment, one or two of the hormone therapies is androgen deprivation therapy or androgen suppression therapy. In another embodiment, one or two of the treatments is a taxanes.

Also described is a method of treating melanoma which method comprises administering to a patient a therapeutically effective amount of a compound of Formula I, II, (III), (IV), (V), (VI), (VII), or (VIII) or a Compound selected from Table 1 and Table 2 and Table 3 optionally as a pharmaceutically acceptable salt, solvate, and/or hydrate thereof, or a pharmaceutical composition comprising a therapeutically effective amount of a compound of Formula I, II, (III), (IV), (V), (VI), (VII), or (VIII) or a Compound selected from Table 1 and Table 2 and Table 3 and a pharmaceutically acceptable carrier, excipient, or diluent in combination with one or more of treatments selected from surgery, radiation, one or more immunotherapies, one or more hormone therapies, and one or more chemotherapeutic agents. In another embodiment, one or two of the chemotherapeutic agents is independently selected from an alkylating agent, a taxane, a platin, and a Raf inhibitor. In another embodiment, one or two chemotherapeutic agent is selected from sorafenib, Paclitaxel (Taxol^{®}), Docetaxel (Taxotere^{®}), dacarbazine, rapamycin, imatinib mesylate (Gleevec®), sorafenib, cisplatin, carboplatin, dacarbazine (DTIC), carmustine (BCNU), vinblastine, temozolomide (Temodar), Melphalan, and imiquimod (Aldara). In another embodiment, one or two of the immunotherapies is selected from ipilimumab, interferon-alpha and/or interleukin-2. In another embodiment, one or two of the hormone therapies is tamoxifen.

Also described is a method of treating colon or rectal cancer which method comprises administering to a patient a therapeutically effective amount of a compound of Formula I, II, (III), (IV), (V), (VI), (VII), or (VIII) or a Compound selected from Table 1 and Table 2 and Table 3 optionally as a pharmaceutically acceptable salt, solvate, and/or hydrate thereof, or a pharmaceutical composition comprising a therapeutically effective amount of a compound of Formula I, II, (III), (IV), (V), (VI), (VII), or (VIII) or a Compound selected from Table 1 and Table 2 and Table 3 and a pharmaceutically acceptable carrier, excipient, or diluent in combination with one or more of treatments selected from surgery, radiation, one or more antibodies, and one or more chemotherapeutic agents. In another embodiment, the surgery is selected from local excision, electrofulguration, segmental colon resection, polypectomy, local transanal resection, low anterior resection, abdominoperineal resection, and pelvic exenteration. In another embodiment, one or two of the chemotherapeutic agents is selected from a platinum-containing compound (including cisplatin, oxaliplatin, and carboplatin), 5-fluorouracil (5-FU), leucovorin, capecitabine (Xeloda), irinotecan (Camptosar), FOLFOX (Folinic acid, 5-FU, Oxaliplatin), and leucovorin. In another embodiment, one or two of the antibodies is selected from cetuximab (Erbitux) and bevacizumab (Avastin).

Also described is a method of treating pancreatic cancer which method comprises administering to a patient a therapeutically effective amount of a compound of Formula I, II, (III), (IV), (V), (VI), (VII), or (VIII) or a Compound selected from Table 1 and Table 2 and Table 3 optionally as a pharmaceutically acceptable salt, solvate, and/or hydrate thereof, or a pharmaceutical composition comprising a therapeutically effective amount of a compound of Formula I, II, (III), (IV), (V), (VI), (VII), or (VIII) or a Compound selected from Table 1 and Table 2 and Table 3 and a pharmaceutically acceptable carrier, excipient, or diluent in combination with one or more of treatments selected from surgery, radiation, and one or more chemotherapeutic agents. In another embodiment, one or two of the chemotherapeutic agents is selected from platinum-containing compound (including cisplatin, oxaliplatin, and carboplatin), 5-fluorouracil (5-FU), gemcitabine, a taxane (including paclitaxel and docetaxel), topotecan, irinotecan, capecitabine, streptozocin, erlotinib (Tarceva), cetuximab, leucovorin, and capecitabine (Xeloda).

Also described is a method of treating breast cancer which method comprises administering to a patient a therapeutically effective amount of a compound of Formula I, II, (III), (IV), (V), (VI), (VII), or (VIII) or a Compound selected from Table 1 and Table 2 and Table 3 optionally as a pharmaceutically acceptable salt, solvate, and/or hydrate thereof, or a pharmaceutical composition comprising a therapeutically effective amount of a compound of Formula I, II, (III), (IV), (V), (VI), (VII), or (VIII) or a Compound selected from Table 1 and Table 2 and Table 3 and a pharmaceutically acceptable carrier, excipient, or diluent in combination with one or more of treatments selected from surgery, radiation, one or more chemotherapeutic agents, one or more hormone therapies, and one or more antibodies. In another embodiment, one or two of the chemotherapeutic agents is selected from lapatinib (Tykerb^{®}), Paclitaxel (Taxol^{®}), docetaxel, capecitabine, Cyclophosphamide (Cytoxan), CMF (cyclophosphamide, fluoruracil, and methotrexate), methotrexate, fluorouracil, doxorubicin, epirubicin, gemcitabine, carboplatin (Paraplatin), cisplatin (Platinol), vinorelbine (Navelbine), capecitabine (Xeloda), pegylated liposomal doxorubicin (Doxil), albumin-bound paclitaxel (Abraxane), AC (adriamycin and Cyclophosphamide), adriamyclin, and pamidronate or zoledronic acid (to treat bone weakness). In another embodiment, one or two of the hormone therapyies is selected from tamoxifen, Toremifene (Fareston), Fulvestrant (Faslodex), Megestrol acetate (Megace), ovarian ablation, Raloxifene, a luteinizing hormone-releasing hormone (LHRH) analogs (including goserelin and leuprolide), Megestrol acetate (Megace), and one or two aromatase inhibitor(s); in another embodiment, one or two of the aromatase inhibitor(s) is selected from letrozole (Femara), anastrozole (Arimidex), and exemestane (Aromasin). In another embodiment, one or more of the antibodyies is selected from ^{α}IGF-1R A12 MoAb, ^{α}IGF-1R 19D12 MoAb, ^{α}IGF-1R h7C10 MoAb, ^{α}IGF-1R CP-751871 MoAb, bevacizumab (Avastin), and trastuzumab.

Also described is a method of treating breast cancer which method comprises administering to a patient a therapeutically effective amount of a compound of Formula I, II, (III), (IV), (V), (VI), (VII), or (VIII) or a Compound selected from Table 1 and Table 2 and Table 3 optionally as a pharmaceutically acceptable salt, solvate, and/or hydrate thereof, or a pharmaceutical composition comprising a therapeutically effective amount of a compound of Formula I, II, (III), (IV), (V), (VI), (VII), or (VIIII) or a Compound selected from Table 1 and Table 2 and Table 3 and a pharmaceutically acceptable carrier, excipient, or diluent in combination with one or more of treatments selected from one or more chemotherapeutic agents and one or more antibodies. In another embodiment, one or two of the chemotherapeutic agents is selected from rapamycin, lapatinib, and erlotinib. In another embodiment, one of the antibodies is trastuzumab.

Also described is a method of treating non-small cell lung cancer which method comprises administering to a patient a therapeutically effective amount of a compound of Formula I, II, (III), (IV), (V), (VI), (VII), or (VIII) or a Compound selected from Table 1 and Table 2 and Table 3 optionally as a pharmaceutically acceptable salt, solvate, and/or hydrate thereof, or a pharmaceutical composition comprising a therapeutically effective amount of a compound of Formula I, II, (III), (IV), (V), (VI), (VII), or (VIII) or a Compound selected from Table 1 and Table 2 and Table 3 and a pharmaceutically acceptable carrier, excipient, or diluent in combination with one or more of treatments selected from surgery, radiation, one or more antibodies, and one or more chemotherapeutic agents. In another embodiment, one or two of the chemotherapeutic agents is independently selected from cisplatin, oxaliplatin, carboplatin, Zactima (ZD6474), Paclitaxel, Docetaxel (Taxotere®), Gemcitabine (Gemzar®), Vinorelbine, Irinotecan, Etoposide, Vinblastine, Erlotinib (Tarceva®), gefitinib (Iressa), and Pemetrexed. In another embodiment, one of the antibodies is Bevacizumab. In another embodiment, the chemotherapeutic agent is selected from cisplatin, oxaliplatin, carboplatin, Paclitaxel, Docetaxel (Taxotere®), and erlotinib (Tarceva®).

Also described is a method of treating small cell lung cancer which method comprises administering to a patient a therapeutically effective amount of a compound of Formula I, II, (III), (IV), (V), (VI), (VII), or (VIII) or a Compound selected from Table 1 and Table 2 and Table 3 optionally as a pharmaceutically acceptable salt, solvate, and/or hydrate thereof, or a pharmaceutical composition comprising a therapeutically effective amount of a compound of Formula I, II, (III), (IV), (V), (VI), (VII), or (VIII) or a Compound selected from Table 1 and Table 2 and Table 3 and a pharmaceutically acceptable carrier, excipient, or diluent in combination with one or more of treatments selected from surgery, radiation, and one or more chemotherapeutic agents. In another embodiment, one or two of the chemotherapy agents is selected from a platin (such as cisplatin, oxaliplatin, and carboplatin), gefitinib, vinorelbine, docetaxel, paclitaxel, etoposide, fosfamide, ifosfamide, cyclophosphamide, cyclophosphamide/doxorubicin/vincristine (CAV), doxorubicin, vincristine, gemcitabine, paclitaxel, vinorelbine, topotecan, irinotecan, inethotrexate, and docetaxel.

Also described is a method of treating papillary or anaplastic thyroid cancer which method comprises administering to a patient a therapeutically effective amount of a compound of Formula I, II, (III), (IV), (V), (VI), (VII), or (VIII) or a Compound selected from Table 1 and Table 2 and Table 3 optionally as a pharmaceutically acceptable salt, solvate, and/or hydrate thereof, or a pharmaceutical composition comprising a therapeutically effective amount of a compound of Formula I, II, (III), (IV), (V), (VI), (VII), or (VIII) or a Compound selected from Table 1 and Table 2 and Table 3 and a pharmaceutically acceptable carrier, excipient, or diluent in combination with one or more of treatments selected from surgery, radiation, radioactive iodine therapy, one or more hormone therapies, and one or more chemotherapeutic agents. In another embodiment, one or two of the chemotherapeutic agents is selected from thyroid hormone pills, Doxorubucin and a platin. In another embodiment, one of the hormone therapies is radioiodine ablation.

Also described is a method of treating endometrial cancer which method comprises administering to a patient a therapeutically effective amount of a compound of Formula I, II, (III), (IV), (V), (VI), (VII), or (VIII) or a Compound selected from Table I and Table 2 and Table 3 optionally as a pharmaceutically acceptable salt, solvate, and/or hydrate thereof, or a pharmaceutical composition comprising a therapeutically effective amount of a compound of Formula I, II, (III), (IV), (V), (VI), (VII), or (VIII) or a Compound selected from Table 1 and Table 2 and Table 3 and a pharmaceutically acceptable carrier, excipient, or diluent in combination with one or more of treatments selected from surgery, radiation, one or more hormone therapies, and one or more chemotherapeutic agents. In another embodiment, one or two of the hormone therapies is selected from megestrol acetate, Tamoxifen, and a progestin including medroxyprogesterone acetate (Provera) and megestrol acetate (Megace). In another embodiment, one or two of the chemotherapeutic agents is selected from a platinum-containing compound (including cisplatin, oxaliplatin, and carboplatin; in another example, cisplatin), a taxane (including paclitaxel), doxorubicin (Adriamycin), cyclophosphamide, fluorouracil (5-FU), methotrexate, and vinblastine.

Also described is a method of treating ovarian cancer which method comprises administering to a patient a therapeutically effective amount of a compound of Formula I, II, (III), (IV), (V), (VI), (VII), or (VIII) or a Compound selected from Table 1 and Table 2 and Table 3 optionally as a pharmaceutically acceptable salt, solvate, and/or hydrate thereof, or a pharmaceutical composition comprising a therapeutically effective amount of a compound of Formula I, II, (III), (IV), (V), (VI), (VII), or (VIII) or a Compound selected from Table 1 and Table 2 and Table 3 and a pharmaceutically acceptable carrier, excipient, or diluent in combination with one or more of treatments selected from surgery, radiation, one or more antibodies, and one or more chemotherapeutic agents. In another embodiment, one of the antibodies is selected from bevacizumab. In another embodiment, one or two of the chemotherapeutic agents is selected from a platinum-containing compound (including cisplatin, oxaliplatin and carboplatin), a taxane (including paclitaxel and docetaxel), topotecan, an anthracyclines (including doxorubicin and liposomal doxorubicin), gemcitabine, cyclophosphamide, vinorelbine (Navelbine), hexamethylmelamine, ifosfamide, etoposide, bleomycin, vinblastine, ifosfamide, vincristine, and cyclophosphamide. In another embodiment, one or more of the treatments is selected from one or two chemotherapeutic agents where the chemotherapeutic agents are independently selected from a platin and a taxane.

Also described is a method of treating glioblastoma which method comprises administering to a patient a therapeutically effective amount of a compound of Formula I, II, (III), (IV), (V), (VI), (VII), or (VIII) or a Compound selected from Table 1 and Table 2 and Table 3 optionally as a pharmaceutically acceptable salt, solvate, and/or hydrate thereof, or a pharmaceutical composition comprising a therapeutically effective amount of a compound of Formula I, II, (III), (IV), (V), (VI), (VII), or (VIII) or a Compound selected from Table 1 and Table 2 and Table 3 and a pharmaceutically acceptable carrier, excipient, or diluent in combination with one or more of treatments selected from surgery, radiation, one or more chemotherapeutic agents, one or more anti-seizure agents, and one or more agents to reduce swelling. In another embodiment, the radiation treatment is selected from external beam radiation, interstitial radiotherapy, and stereotactic radiosurgery. In another embodiment, one or two of the chemotherapeutic agents is selected from carmustine (BCNU), Erlotinib (Tarceva), bevacizumab, gefitinib (Iressa), rapamycin, temozolomide, cisplatin, BCNU, lomustine, procarbazine, and vincristine. In another embodiment, one or two of the anti-seizure agents is selected from diphenylhydantoin (Dilantin). In another embodiment, one or two of the agents to reduce swelling is selected from dexamethasone (Decadron). In another embodiment, one of the treatments is one or two chemotherapeutic agents; in another embodiment, one or two of the chemotherapeutic agents are independently selected from erlotinib and temozolomide.

Also described is a method of treating cervical cancer which method comprises administering to a patient a therapeutically effective amount of a compound of Formula I, II, (III), (IV), (V), (VI), (VII),or (VIII) or a Compound selected from Table 1 and Table 2 and Table 3 optionally as a pharmaceutically acceptable salt, solvate, and/or hydrate thereof, or a pharmaceutical composition comprising a therapeutically effective amount of a compound of Formula I, II, (III), (IV), (V), (VI), (VII), or (VIII) or a Compound selected from Table 1 and Table 2 and Table 3 and a pharmaceutically acceptable carrier, excipient, or diluent in combination with one or more of treatments selected from surgery, radiation, and one or more chemotherapeutic agents. In another embodiment, the surgery is selected from cryosurgery, laser surgery, loop electrosurgical excision, conization, simple hysterectomy, and radical hysterectomy and pelvic lymph node dissection. In another embodiment, the radiation is selected from called external beam radiation therapy and brachytherapy. In another embodiment, one or two of the chemotherapeutic agents is selected from a platinum compound (such as cisplatin, carboplatin, and oxaliplatin), paclitaxel, topotecan, ifosfamipe, gemcitabine, vinorelbine, and fluorouracil.

Also described is a method of treating gastrointestinal carcinoid tumor which method comprises administering to a patient a therapeutically effective amount of a compound of Formula I, II, (III), (IV), (V), (VI), (VII), or (VIII) or a Compound selected from Table 1 and Table 2 and Table 3 optionally as a pharmaceutically acceptable salt, solvate, and/or hydrate thereof, or a pharmaceutical composition comprising a therapeutically effective amount of a compound of Formula I, II, (III), (IV), (V), (VI), (VII), or (VIII) or a Compound selected from Table 1 and Table 2 and Table 3 and a pharmaceutically acceptable carrier, excipient, or diluent in combination with one or more of treatments selected from surgery, radiation, immunotherapy, and one or more chemotherapeutic agents. In another embodiment, the surgery is selected from excision and electrofulguration. In another embodiment, one or two of the chemotherapeutic agents is selected from cyproheptadine, SOM230, octreotide and lanreotide. In another embodiment, the immunotherapy is an interferon.

Also described is a method of treating gastrointestinal stromal tumor which method comprises administering to a patient a therapeutically effective amount of a compound of Formula I, II, (III), (IV), (V), (VI), (VII), or (VIII) or a Compound selected from Table 1 and Table 2 and Table 3 optionally as a pharmaceutically acceptable salt, solvate, and/or hydrate thereof, or a pharmaceutical composition comprising a therapeutically effective amount of a compound of Formula I, II, (III), (IV), (V), (VI), (VII), or (VIII) or a Compound selected from Table 1 and Table 2 and Table 3 and a pharmaceutically acceptable carrier, excipient, or diluent in combination with one or more of treatments selected from surgery, radiation, and one or more chemotherapeutic agents. In another embodiment, one or two of the chemotherapeutic agents are independently selected from imatinib mesylate (Gleevec), sunitinib (Sutent), and nilotinib (AMN107).

Also described is a method of treating hepatocellular carcinoma which method comprises administering to a patient a therapeutically effective amount of a compound of Formula I, II, (III), (IV), (V), (VI), (VII), or (VIII) or a Compound selected from Table 1 and Table 2 and Table 3 optionally as a pharmaceutically acceptable salt, solvate, and/or hydrate thereof, or a pharmaceutical composition comprising a therapeutically effective amount of a compound of Formula I, II, (III), (IV), (V), (VI), (VII), or (VIII) or a Compound selected from Table 1 and Table 2 and Table 3 and a pharmaceutically acceptable carrier, excipient, or diluent in combination with one or more of treatments selected from surgery, radiofrequency ablation, ethanol ablation, cryosurgery, hepatic artery embolization, chemoembolization, radiation, and one or more chemotherapeutic agents. In another embodiment, the surgery is selected from resection and transplantation. In another embodiment, one or two of the chemotherapeutic agents are independently selected from sorafenib, 5-fluorouracil and cisplatin.

Also described is a method of treating non-Hodgkin's lymphoma which method comprises administering to a patient a therapeutically effective amount of a compound of Formula I, II, (III), (IV), (V), (VI), (VII), or (VIII) or a Compound selected from Table 1 and Table 2 and Table 3 optionally as a pharmaceutically acceptable salt, solvate, and/or hydrate thereof, or a pharmaceutical composition comprising a therapeutically effective amount of a compound of Formula I, II, (III), (IV), (V), (VI), (VII), or (VIII) or a Compound selected from Table 1 and Table 2 and Table 3 and a pharmaceutically acceptable carrier, excipient, or diluent in combination with one or more of treatments selected from radiation, one or more chemotherapeutic agents, interferon therapy, one or more antibodies, and bone marrow or peripheral blood stem cell transplantation. In another embodiment, one or two of the chemotherapeutic agents are independently selected from CHOP (cyclophosphamide, doxorubicin, vincristine and prednisone), chlorambucil, fludarabine, and etoposide. In another embodiment, the antibody is selected from rituximab, ibritumomab tiuxetan, tositumomab, and alemtuzumab; in another embodiment, the antibody is rituximab

Also described is a method of treating cancer which method comprises administering to a patient a therapeutically effective amount of a compound of Formula I, II, (III), (IV), (V), (VI), (VII), or (VIII) or a Compound selected from Table 1 and Table 2 and Table 3 optionally as a pharmaceutically acceptable salt, solvate, and/or hydrate thereof, or a pharmaceutical composition comprising a therapeutically effective amount of a compound of Formula I, II, (III), (IV), (V), (VI), (VII), or (VIII) or a Compound selected from Table 1 and Table 2 and Table 3 and a pharmaceutically acceptable carrier, excipient, or diluent in combination with radiation and surgery.

Also described is a method of treating cancer which method comprises administering to a patient a therapeutically effective amount of a compound of Formula I, II, (III), (IV), (V), (VI), (VII), or (VIII) or a Compound selected from Table 1 and Table 2 and Table 3 optionally as a pharmaceutically acceptable salt, solvate, and/or hydrate thereof, or a pharmaceutical composition comprising a therapeutically effective amount of a compound of Formula I, II, (III), (IV), (V), (VI), (VII), or (VIII) or a Compound selected from Table 1 and Table 2 and Table 3 and a pharmaceutically acceptable carrier, excipient, or diluent in combination with radiation and one or two chemotherapeutic agents.

Also described is a method of treating cancer which method comprises administering to a patient a therapeutically effective amount of a compound of Formula I, II, (III), (IV), (V), (VI), (VII), or (VIII) or a Compound selected from Table 1 and Table 2 and Table 3 optionally as a pharmaceutically acceptable salt, solvate, and/or hydrate thereof, or a pharmaceutical composition comprising a therapeutically effective amount of a compound of Formula I, II, (III), (IV), (V), (VI), (VII), or (VIII) or a Compound selected from Table 1 and Table 2 and Table 3 and a pharmaceutically acceptable carrier, excipient, or diluent in combination with surgery and one or two chemotherapeutic agents.

Also described is a method of inhibiting proliferative activity in a cell, the method comprising administering to a cell or a plurality of cells an effective amount of a compound of Formula I, II, (III), (IV), (V), (VI), (VII), or (VIII) or a Compound selected from Table 1 and Table 2 and Table 3 optionally as a pharmaceutically acceptable salt, solvate, and/or hydrate thereof, and additionally optionally as pharmaceutical composition thereof.

### Representative Compounds

Representative compounds of Formula III are depicted below. The examples are merely illustrative and do not limit the scope of the invention in any way. Compounds of the invention are named according to systematic application of the nomenclature rules agreed upon by the International Union of Pure and Applied Chemistry (IUPAC), International Union of Biochemistry and Molecular Biology (IUBMB), and the Chemical Abstracts Service (CAS). Names were generated using ACD/Labs naming software 8.00 release, product version 8.08. Each of the recited compounds in Table 1 can be prepared as an individual isomer (where applicable) as well as a pharmaceutically acceptable salt, solvate, and/or hydrate, thereof.

**Table 1**

| **Example** | **Structure** | **Name** |
|---|---|---|
| 1 | 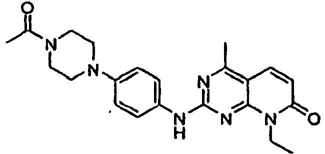 | 2-{[4-(4-acetylpiperazin-1-yl)phenyl]amino}-8-ethyl-4-methylpyrido[2,3-d]pyrimidin-7(8H)-one |
| 2 | 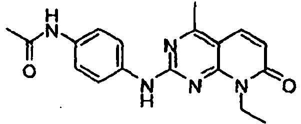 | *N*-(4-[(8-ethyl-4-methyl-7-oxo-7,8-dihydropyrido[2,3-d]pyrimidin-2-yl)amino]phenyl}acetamide |
| 3 | 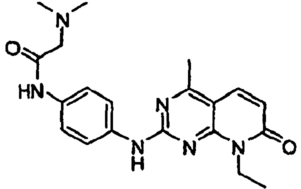 | *N*-(4-[(8-ethyl-4-methyl-7-oxo-7,8-dihydropyrido[2,3-d]pyrimidin-2-yl)amino]phenyl}-*N~*2~,*N~*2~-dimethylglycinamide |
| 4 | 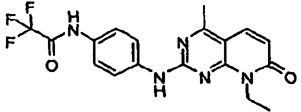 | *N*-{4-[(8-ethyl-4-methyl-7-oxo-7,8-dihydropyrido[2,3-d]pyrimidin-2-yl)amino]phenyl}-2,2,2-trifluoroacetamide |
| 5 | 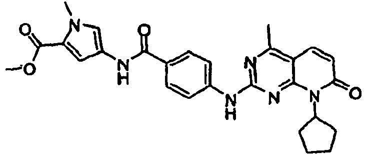 | methyl 4-[({4-[(8-cyclopentyl-4-methyl-7-oxo-7,8-dihydropyrido[2,3-d]pyrimidin-2-yl)amino]phenyl}carbonyl)amino]-1-methyl-1*H*-pyrrole-2-carboxylate |
| 6 | 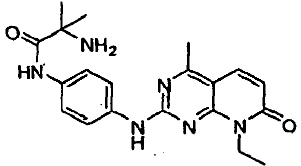 | *N*-{4-[(8-ethyl-4-methyl-7-oxo-7,8-dihydropyrido[2,3-d]pyrimidin-2-yl)amino]phenyl}-2-methylalaninamide |

### Other Compounds

Representative Compounds of Formula II are depicted below and are named according to systematic application of the nomenclature rules agreed upon by the International Union of Pure and Applied Chemistry (IUPAC), International Union of Biochemistry and Molecular Biology (IUBMB), and the Chemical Abstracts Service (CAS). Names were generated using ACD/Labs naming software 8.00 release, product version 8.08. Each of the recited compounds in Table 2 can be prepared as an individual isomer (where applicable) as well as a pharmaceutically acceptable salt, solvate, and/or hydrate, thereof.

**Table 2**

| **Example** | **Structure** | **Name** |
|---|---|---|
| 1 | 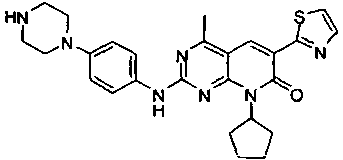 | 8-cyclopentyl-4-methyl-2-[(4-piperazin-1-ylphenyl)amino]-6-(1,3-thiazol-2-yl)pyrido[2,3-d]pyrimidin-7(8*H*)-one |
| 2 | 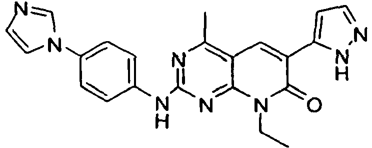 | 8-ethyl-2-{[4-(1*H*-imidazol-1-yl)phenyl]amino}-4-methyl-6-(1*H*-pyrazol-5-yl)pyrido[2,3-d]pyrimidin-7(8*H*)-one |
| 3 | 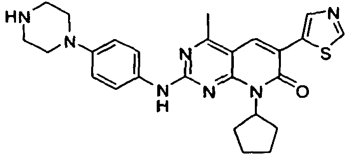 | 8-cyclopentyl-4-methyl-2-[(4-piperazin-1-ylphenyl)amino]-6-(1,3-thiazol-5-yl)pyrido[2,3-d]pyrimidin-7(8*H*)-one |
| 4 | 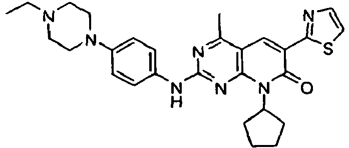 | 8-cyclopentyl-2-{[4-(4-ethylpiperazin-1-yl)phenyl]amino}-4-methyl-6-(1,3-thiazol-2-yl)pyrido[2,3-d]pyrimidin-7(8*H*)-one; |
| 5 | 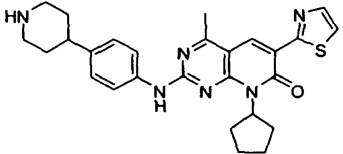 | 8-cyclopentyl-4-methyl-2-[(4-piperidin-4-ylphenyl)amino]-6-(1,3-thiazol-2-yl)pyrido[2,3-d]pyrimidin-7(8*H*)-one |

Representative compounds of Formula I are depicted below. The examples are merely illustrative and do not limit the scope of the invention in any way. Compounds of the invention are named according to systematic application of the nomenclature rules agreed upon by the International Union of Pure and Applied Chemistry (IUPAC), International Union of Biochemistry and Molecular Biology (IUBMB), and the Chemical Abstracts Service (CAS). Names were generated using ACD/Labs naming software 8.00 release, product version 8.08. Each of the recited compounds in Table 3 can be prepared as an individual isomer (where applicable) as well as a pharmaceutically acceptable salt, solvate, and/or hydrate, thereof.

**Table 3**

| **Example** | **Structure** | **Name** |
|---|---|---|
| 1 | 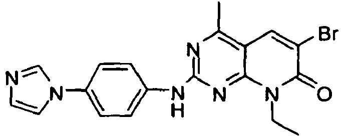 | 6-bromo-8-ethyl-2-{[4-(1*H*-imidazol-1-yl)phenyl]amino}-4-methylpyrido[2,3-*d*]Pyrimidin-7(8*H*)-one |
| 2 | 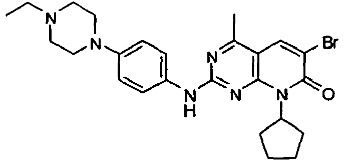 | 6-bromo-8-cyclopenryl-2-{[4-(4-ethylpiperazin-1-yl)phenyl]amino}-4-methylpyrido[2,3-d)pyrimidin-7(8*H*)-one |
| 3 | 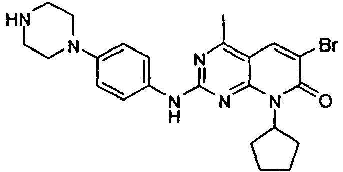 | 6-bromo-8-cyclopentyl-4-methyl-2-[(4-piperazin-1-ylpheny)amino]pyrido[2,3-*d*]pyrimidin-7(8*H*)-one |
| 4 | 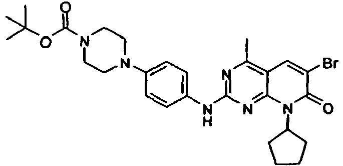 | 1,1-dimethylethy 4-{4-[(6-bromo-8-cyclopentyl-4-methyl-7-oxo-7,8-dihydropyrido[2,3-*d*]pyrimidin-2-yl)amino]phenyl}piperazine-1-carboxylate |
| 5 | 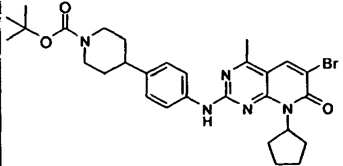 | 1,1-dimethylethyl 4-{4-[(6-bromo-8-cyclopentyl-4-methyl-7-oxo-7,8-dihydropyrido[2,3-d]pyrimidin-2-yl)amino]phenyl}piperidine-1-carboxylate |
| 6 | 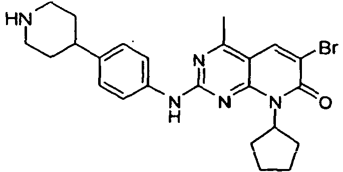 | 6-bromo-8-cyclopentyl-4-methyl-2-[(4-piperidin-4-ylphenyl)amino]pyrido[2,3-*d*]pyrimidin-7(8*H*)-one |
| 7 | 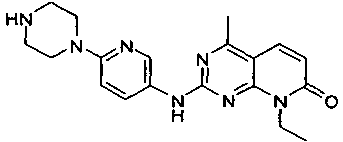 | 8-ethyl-4-methyl-2-[(6-piperazin-1-ylpyridin-3-yl)amino]pyrido[2,3-*d*]pyrimidin-7(8*H*)-one |
| 8 | 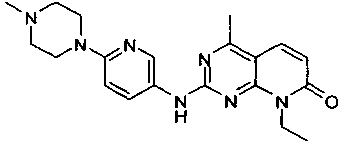 | 8-ethyl-4-methyl-2-{[6-(4-methylpiperazin-1-yl)pyridin-3-yl]amino}pyrido[2,3-*d*]pyrimidin-7(8*H*)-one |
| 9 | 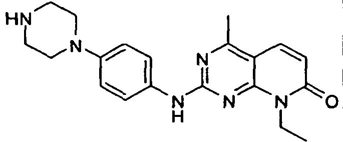 | 8-ethyl-4-methyl-2-[(4-piperazin-1-ylphenyl)amino]pyrido[2,3-*d*]pyrimidin-7(8*H*)-one |
| 10 | 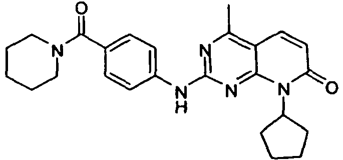 | 8-cyclopentyl-4-methyl-2-{[4-(piperidin-1-ylcarbonyl)phenyl]amino}pyrido[2,3-*d*]pyrimidin-7(8*H*)-one |
| 11 | 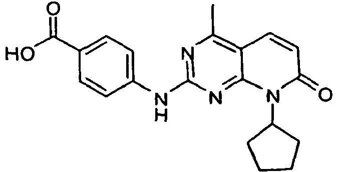 | 4-[(8-cyclopentyl-4-methyl-7-oxo-7,8-*d*ihydropyrido[2,3-*d*]pyrimidin-2-yl)amino]benzoic acid |
| 12 | 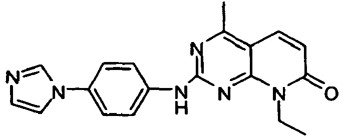 | 8-ethyl-2-{[4-(1*H*-imidazol-1-yl)phenyl]amino}-4-methylpyrido[2,3-*d*]pyrimidin-7(8*H*)-one |
| 13 | 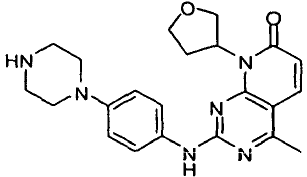 | 4-methyl-2-[(4-piperazin-1-ylphenyl)amino]-8-(tetrahydrofuran-3-yl)pyrido[2,3-*d*]pyrimidin-7(8*H*)-one |
| 14 | 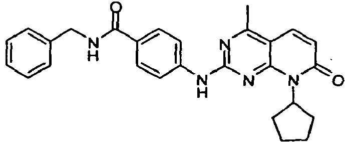 | 4-[(8-cyclopentyl-4-methyl-7-oxo-7,8-dihydropyrido[2,3-*d*]pyrimidin-2-yl)amino]-*N-*(phenylmethyl)benzamide |
| 15 | 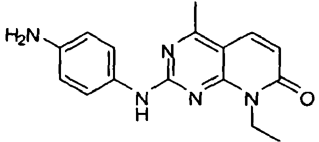 | 2-[(4-aminophenyl)amino]-8-ethyl-4-methylpyrido[2,3-*d*]pyrimidin-7(8*H*)-one |
| 16 | 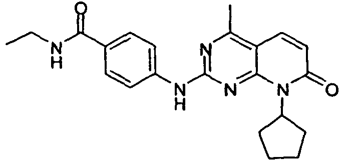 | 4-[(8-cyclopentyl-4-methyl-7-oxo-7,8-dihydropyrido[2,3-d]pyrimidin-2-yl)aminol-*N-*ethylbenzamide |
| 17 | 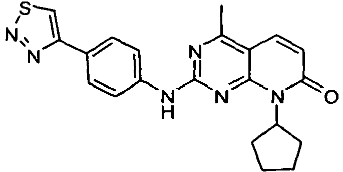 | 8-cyclopentyl-4-methyl-2-{(4-(1,2,3-thiadiazol-4-yl)phenyl]amino}pyrido[2,3-*d*]pyrimidin-7(8*H*)-one |
| 18 | 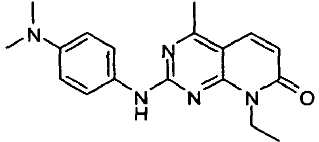 | 2-{[4-(dimethylamino)phenyl]amino}-8-ethyl-4-methylpyrido[2,3-*d*]pyrimidin-7(8*H*)-one |
| 19 | 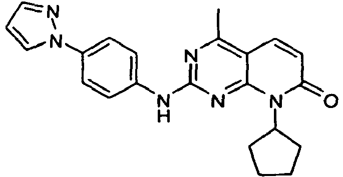 | 8-cyclopentyl-4-methyl-2-{[4-(1*H*-pyrazol-1-y])phenyl]amino}pyrido[2,3-*d*]pyrimidin-7(8*H*)-one |
| 20 | 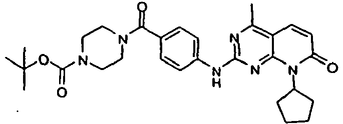 | 1,1-dimethylethyl 4-({4-[(8-cyclopentyl-4-methyl-7-oxo-7,8-*d*ihydropyrido[2,3-*d*]pyrimidin-2-yl)amino]phenyl}carbonyl)piperazine-1-carboxylate |
| 21 | 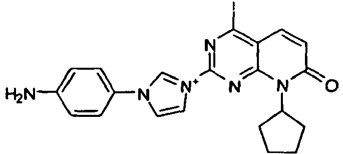 | 1-(4-aminophenyl)-3-(8-cyclopentyl-4-methyl-7-oxo-7,8-dihydropyrido[2,3-*d*]pyrimidin-2-yl)-1*H-*imidazol-3-ium |
| 22 | 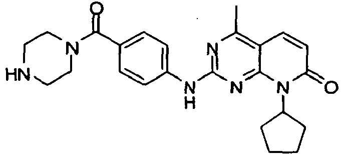 | 8-cyclopentyl-4-methyl-2-{[4-(piperazin-1-ylcarbonyl)phenyl]amino}pyrido[2,3-*d*]pyrimidin-7(8*H*)-one |

### General Administration

In one aspect, the invention provides pharmaceutical compositions comprising an inhibitor of PI3K according to the invention and a pharmaceutically acceptable carrier, excipient, or diluent. In certain other specific embodiments, administration is by the oral route. Administration of the compounds of the invention, or their pharmaceutically acceptable salts, in pure form or in an appropriate pharmaceutical composition, can be carried out via any of the accepted modes of administration or agents for serving similar utilities. Thus, administration can be, for example, orally, nasally, parenterally (intravenous, intramuscular, or subcutaneous), topically, transdermally, intravaginally, intravesically, intracistemally, or rectally, in the form of solid, semi-solid, lyophilized powder, or liquid dosage forms, such as for example, tablets, suppositories, pills, soft elastic and hard gelatin capsules, powders, solutions, suspensions, or aerosols, or the like, specifically in unit dosage forms suitable for simple administration of precise dosages.

The compositions will include a conventional pharmaceutical carrier or excipient and a compound of the invention as the/an active agent, and, in addition, may include carriers and adjuvants, etc.

Adjuvants include preserving, wetting, suspending, sweetening, flavoring, perfuming, emulsifying, and dispensing agents. Prevention of the action of microorganisms can be ensured by various antibacterial and antifungal agents, for example, parabens, chlorobutanol, phenol, sorbic acid, and the like. It may also be desirable to include isotonic agents, for example sugars, sodium chloride, and the like. Prolonged absorption of the injectable pharmaceutical form can be brought about by the use of agents delaying absorption, for example, aluminum monostearate and gelatin.

If desired, a pharmaceutical composition of the invention may also contain minor amounts of auxiliary substances such as wetting or emulsifying agents, pH buffering agents, antioxidants, and the like, such as, for example, citric acid, sorbitan monolaurate, triethanolamine oleate, butylalted hydroxytoluene, etc.

The choice of formulation depends on various factors such as the mode of drug administration (e.g., for oral administration, formulations in the form of tablets, pills or capsules) and the bioavailability of the drug substance. Recently, pharmaceutical formulations have been developed especially for drugs that show poor bioavailability based upon the principle that bioavailability can be increased by increasing the surface area i.e., decreasing particle size. For example, U.S. Pat. No. 4,107,288 describes a pharmaceutical formulation having particles in the size range from 10 to 1,000 nm in which the active material is supported on a crosslinked matrix of macromolecules. U.S. Pat. No. 5,145,684 describes the production of a pharmaceutical formulation in which the drug substance is pulverized to nanoparticles (average particle size of 400 nm) in the presence of a surface modifier and then dispersed in a liquid medium to give a pharmaceutical formulation that exhibits remarkably high bioavailability.

Compositions suitable for parenteral injection may comprise physiologically acceptable sterile aqueous or nonaqueous solutions, dispersions, suspensions or emulsions, and sterile powders for reconstitution into sterile injectable solutions or dispersions. Examples of suitable aqueous and nonaqueous carriers, diluents, solvents or vehicles include water, ethanol, polyols (propyleneglycol, polyethyleneglycol, glycerol, and the like), suitable mixtures thereof, vegetable oils (such as olive oil) and injectable organic esters such as ethyl oleate. Proper fluidity can be maintained, for example, by the use of a coating such as lecithin, by the maintenance of the required particle size in the case of dispersions and by the use of surfactants.

One specific route of administration is oral, using a convenient daily dosage regimen that can be adjusted according to the degree of severity of the disease-state to be treated.

Solid dosage forms for oral administration include capsules, tablets, pills, powders, and granules. In such solid dosage forms, the active compound is admixed with at least one inert customary excipient (or carrier) such as sodium citrate or dicalcium phosphate or (a) fillers or extenders, as for example, starches, lactose, sucrose, glucose, mannitol, and silicic acid, (b) binders, as for example, cellulose derivatives, starch, alignates, gelatin, polyvinylpyrrolidone, sucrose, and gum acacia, (c) humectants, as for example, glycerol, (d) disintegrating agents, as for example, agar-agar, calcium carbonate, potato or tapioca starch, alginic acid, croscarmellose sodium, complex silicates, and sodium carbonate, (e) solution retarders, as for example paraffin, (f) absorption accelerators, as for example, quaternary ammonium compounds, (g) wetting agents, as for example, cetyl alcohol, and glycerol monostearate, magnesium stearate and the like (h) adsorbents, as for example, kaolin and bentonite, and (i) lubricants, as for example, talc, calcium stearate, magnesium stearate, solid polyethylene glycols, sodium lauryl sulfate, or mixtures thereof. In the case of capsules, tablets, and pills, the dosage forms may also comprise buffering agents.

Solid dosage forms as described above can be prepared with coatings and shells, such as enteric coatings and others well known in the art. They may contain pacifying agents, and can also be of such composition that they release the active compound or compounds in a certain part of the intestinal tract in a delayed manner. Examples of embedded compositions that can be used are polymeric substances and waxes. The active compounds can also be in microencapsulated form, if appropriate, with one or more of the above-mentioned excipients.

Liquid dosage forms for oral administration include pharmaceutically acceptable emulsions, solutions, suspensions, syrups, and elixirs. Such dosage forms are prepared, for example, by dissolving, dispersing, etc., a compound(s) of the invention, or a pharmaceutically acceptable salt thereof, and optional pharmaceutical adjuvants in a carrier, such as, for example, water, saline, aqueous dextrose, glycerol, ethanol and the like; solubilizing agents and emulsifiers, as for example, ethyl alcohol, isopropyl alcohol, ethyl carbonate, ethyl acetate, benzyl alcohol, benzyl benzoate, propyleneglycol, 1,3-butyleneglycol, dimethylformamide; oils, in particular, cottonseed oil, groundnut oil, corn germ oil, olive oil, castor oil and sesame oil, glycerol, tetrahydrofurfuryl alcohol, polyethyleneglycols and fatty acid esters of sorbitan; or mixtures of these substances, and the like, to thereby form a solution or suspension.

Suspensions, in addition to the active compounds, may contain suspending agents, as for example, ethoxylated isostearyl alcohols, polyoxyethylene sorbitol and sorbitan esters, microcrystalline cellulose, aluminum metahydroxide, bentonite, agar-agar and tragacanth, or mixtures of these substances, and the like.

Compositions for rectal administrations are, for example, suppositories that can be prepared by mixing the compounds of the present invention with for example suitable nonirritating excipients or carriers such as cocoa butter, polyethyleneglycol or a suppository wax, which are solid at ordinary temperatures but liquid at body temperature and therefore, melt while in a suitable body cavity and release the active component therein.

Dosage forms for topical administration of a compound of this invention include ointments, powders, sprays, and inhalants. The active component is admixed under sterile conditions with a physiologically acceptable carrier and any preservatives, buffers, or propellants as may be required. Ophthalmic formulations, eye ointments, powders, and solutions are also contemplated as being within the scope of this invention.

Compressed gases may be used to disperse a compound of this invention in aerosol form. Inert gases suitable for this purpose are nitrogen, carbon dioxide, etc.

Generally, depending on the intended mode of administration, the pharmaceutically acceptable compositions will contain about 1% to about 99% by weight of a compound(s) of the invention, or a pharmaceutically acceptable salt thereof, and 99% to 1% by weight of a suitable pharmaceutical excipient. In one example, the composition will be between about 5% and about 75% by weight of a compound(s) of the invention, or a pharmaceutically acceptable salt thereof, with the rest being suitable pharmaceutical excipients.

Actual methods of preparing such dosage forms are known, or will be apparent, to those skilled in this art; for example, see Remington's Pharmaceutical Sciences, 18th Ed., (Mack Publishing Company, Easton, Pa., 1990). The composition to be administered will, in any event, contain a therapeutically effective amount of a compound of the invention, or a pharmaceutically acceptable salt thereof, for treatment of a disease-state in accordance with the teachings of this invention.

The compounds of the invention, or their pharmaceutically acceptable salts or solvates, are administered in a therapeutically effective amount which will vary depending upon a variety of factors including the activity of the specific compound employed, the metabolic stability and length of action of the compound, the age, body weight, general health, sex, diet, mode and time of administration, rate of excretion, drug combination, the severity of the particular disease-states, and the host undergoing therapy. The compounds of the present invention can be administered to a patient at dosage levels in the range of about 0.1 to about 1,000 mg per day. For a normal human adult having a body weight of about 70 kilograms, a dosage in the range of about 0.01 to about 100 mg per kilogram of body weight per day is an example. The specific dosage used, however, can vary. For example, the dosage can depend on a number of factors including the requirements of the patient, the severity of the condition being treated, and the pharmacological activity of the compound being used. The determination of optimum dosages for a particular patient is well known to one of ordinary skill in the art.

If formulated as a fixed dose, such combination products employ the compounds of this invention within the dosage range described above and the other pharmaceutically active agents within its approved dosage range. Compounds of the instant invention may alternatively be used sequentially with known pharmaceutically acceptable agents when a combination formulation is inappropriate.

### UTILITY

Compounds in Table 1, 2, and 3 have been tested using the assay described in Biological Example 1 and have been determined to be PI3K inhibitors. As such compounds of Formula III are useful for treating diseases, particularly cancer in which PI3K activity contributes to the pathology and/or symptomatology of the disease. For example, cancer in which PI3K activity contributes to its pathology and/or symptomatology include breast cancer, colon cancer, rectal cancer, endometrial cancer, gastric carcinoma, glioblastoma, hepatocellular carcinoma, small cell lung cancer, non-small cell lung cancer, melanoma, ovarian cancer, cervical cancer, pancreatic cancer, prostate carcinoma, acute myelogenous leukemia (AML), chronic myelogenous leukemia (CML), and thyroid carcinoma.

Suitable *in vitro* assays for measuring PI3K activity and the inhibition thereof by compounds are known in the art. For further details of an *in vitro* assay for measuring PI3K activity see Biological Examples, Example 1 *infra.* Following the examples disclosed herein, as well as that disclosed in the art, a person of ordinary skill in the art can determine the inhibitory activity of a compound of this invention.

Assays for measuring cell-based activity are described in Biological Examples, Example 2, 3, and 4 *infra* and are also known in the art..

Suitable *in vivo* models for cancer are known to those of ordinary skill in the art. For further details of *in vivo* models for prostate adenocarcinoma, glioblastoma, lung carcinoma, and breast adenocarcinoma, see Biological Examples 5, 6, 7, 8, 9, and 10, *infra.*

### General Synthesis

Compounds of this invention can be made by the synthetic procedures described below. The starting materials and reagents used in preparing these compounds are either available from commercial suppliers such as Aldrich Chemical Co. (Milwaukee, Wis.), or Bachem (Torrance, Calif.), or are prepared by methods known to those skilled in the art following procedures set forth in references such as Fieser and Fieser's Reagents for Organic Synthesis, Volumes 1-17 (John Wiley and Sons, 1991); Rodd's Chemistry of Carbon Compounds, Volumes 1-5 and Supplementals (Elsevier Science Publishers, 1989); Organic Reactions, Volumes 1-40 (John Wiley and Sons, 1991), March's Advanced Organic Chemistry, (John Wiley and Sons, 4th Edition) and Larock's Comprehensive Organic Transformations (VCH Publishers Inc., 1989). These schemes are merely illustrative of some methods by which the compounds of this invention can be synthesized, and various modifications to these schemes can be made and will be suggested to one skilled in the art having referred to this disclosure. The starting materials and the intermediates of the reaction may be isolated and purified if desired using conventional techniques, including but not limited to filtration, distillation, crystallization, chromatography and the like. Such materials may be characterized using conventional means, including physical constants and spectral data.

Unless specified to the contrary, the reactions described herein take place at atmospheric pressure and over a temperature range from about -78 °C to about 150 °C, in another embodiment from about 0 °C. to about 125 °C and in another embodiment at about room (or ambient) temperature, e.g., about 20 °C. Unless otherwise stated (as in the case of an hydrogenation), all reactions are performed under an atmosphere of nitrogen.

Prodrugs can be prepared by techniques known to one skilled in the art. These techniques generally modify appropriate functional groups in a given compound. These modified functional groups regenerate original functional groups by routine manipulation or *in vivo.* Amides and esters of the compounds of the present invention may be prepared according to conventional methods. A thorough discussion of prodrugs is provided in T. Higuchi and V. Stella, "Pro-drugs as Novel Delivery Systems," Vol 14 of the A.C.S. Symposium Series, and in Bioreversible Carriers in Drug Design, ed. Edward B. Roche, American Pharmaceutical Association and Pergamon Press, 1987.

The compounds of the invention, or their pharmaceutically acceptable salts, may have asymmetric carbon atoms or quatemized nitrogen atoms in their structure. Compounds of the Invention that may be prepared through the syntheses described herein may exist as single stereoisomers, racemates, and as mixtures of enantiomers and diastereomers. The compounds may also exist as geometric isomers. All such single stereoisomers, racemates and mixtures thereof, and geometric isomers are intended to be within the scope of this invention. Some of the compounds of the invention may exist as tautomers. For example, where a ketone or aldehyde is present, the molecule may exist in the enol form; where an amide is present, the molecule may exist as the imidic acid; and where an enamine is present, the molecule may exist as an imine. All such tautomers are within the scope of the invention.

The present invention also includes N-oxide derivatives and protected derivatives of compounds of the Invention. For example, when compounds of the Invention contain an oxidizable nitrogen atom, the nitrogen atom can be converted to an N-oxide by methods well known in the art. When compounds of the Invention contain groups such as hydroxy, carboxy, thiol or any group containing a nitrogen atom(s), these groups can be protected with a suitable "protecting group" or "protective group". A comprehensive list of suitable protective groups can be found in T.W. Greene, Protective Groups in Organic Synthesis, John Wiley & Sons, Inc. 1991. The protected derivatives of compounds of the Invention can be prepared by methods well known in the art.

Methods for the preparation and/or separation and isolation of single stereoisomers from racemic mixtures or non-racemic mixtures of stereoisomers are well known in the art. For example, optically active (R)- and (S)- isomers may be prepared using chiral synthons or chiral reagents, or resolved using conventional techniques. Enantiomers (R- and S-isomers) may be resolved by methods known to one of ordinary skill in the art, for example by: formation of diastereoisomeric salts or complexes which may be separated, for example, by crystallization; via formation of diastereoisomeric derivatives which may be separated, for example, by crystallization, selective reaction of one enantiomer with an enantiomer-specific reagent, for example enzymatic oxidation or reduction, followed by separation of the modified and unmodified enantiomers; or gas-liquid or liquid chromatography in a chiral environment, for example on a chiral support, such as silica with a bound chiral ligand or in the presence of a chiral solvent. It will be appreciated that where a desired enantiomer is converted into another chemical entity by one of the separation procedures described above, a further step may be required to liberate the desired enantiomeric form. Alternatively, specific enantiomer may be synthesized by asymmetric synthesis using optically active reagents, substrates, catalysts or solvents or by converting on enantiomer to the other by asymmetric transformation. For a mixture of enantiomers, enriched in a particular enantiomer, the major component enantiomer may be further enriched (with concomitant loss in yield) by recrystallization.

In addition, the compounds of the present invention can exist in unsolvated as well as solvated forms with pharmaceutically acceptable solvents such as water, ethanol, and the like. In general, the solvated forms are considered equivalent to the unsolvated forms for the purposes of the present invention.

The chemistry for the preparation of the compounds of this invention is known to those skilled in the art. In fact, there may be more than one process to prepare the compounds of the invention. For specific examples, see M. Barvian et al. J. Med. Chem. 2000, 43, 4606-4616; S. N. VanderWei et al. J. Med. Chem. 2005, 48, 2371-2387; P. L. Toogood et al. J. Med. Chem. 2005, 48, 2388-2406; J. Kasparec et al. Tetrahedron Letters 2003, 44, 4567-4570; and references cited therein. See also U.S. Pre-grant publication US2004/0009993 A1 (M. Angiolini et al.). The following examples illustrate but do not limit the invention. All references cited herein are incorporated by reference in their entirety.

A Compound of Formula II where R' is R³² and R²⁸, R²⁹, and R³² are as defined for a Compound of Formula II in the Summary of the Invention can be prepared according to Scheme 1.

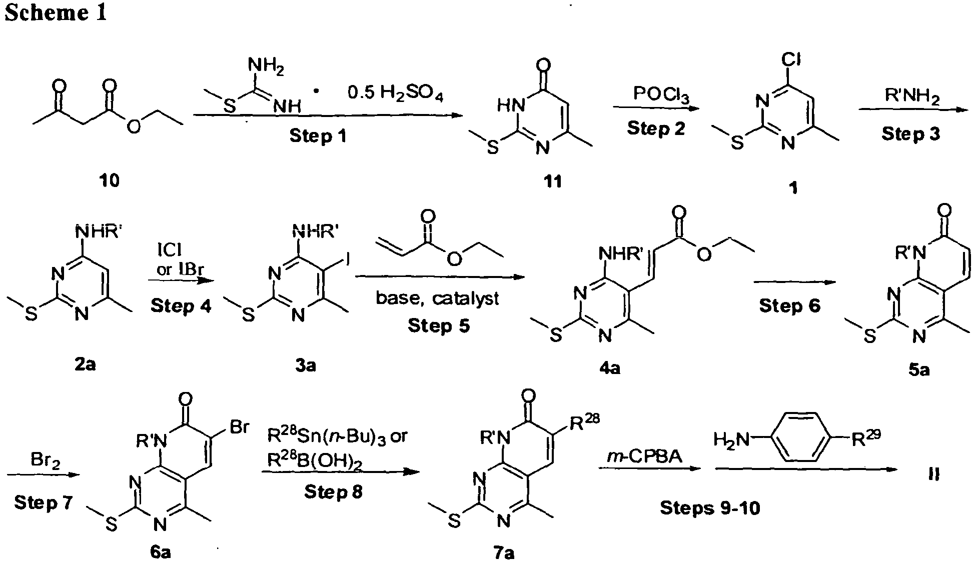

To a solution of commercially available 2-methyl-2-thiopseudourea sulfate in a solvent such as water is added a base such as sodium carbonate and an intermediate of formula **10** at room temperature. The reaction mixture is stirred for overnight or less. After neutralizing, **11** is collected through filtration and followed by drying under vacuum. **11** is then treated with POCl₃ and the reaction is heated to reflux for approximately 2 h and then concentrated under vacuum to dryness. **1** can be used directly in the next reaction without further purification.

An intermediate of formula **2a** is prepared by reacting an intermediate of formula 1 with a primary amine R'NH₂ in a solvent such as water and with heating. **2a** is then treated with iodine monochloride or iodine monobromide in a solvent such as methanol at around 0 °C and allowed to react for approximately overnight or less as needed for the reaction to go to completion to form **3a.** After completion the residue is triturated with acetone. Alternatively, after completion, the reaction mixture can be poured into 0.2 N sodium thiosulfate to quench excess iodine.

The intermediate **3a** is then reacted in a solvent, such as DMA or DMF, with ethyl acrylate in the presence of a base, such as triethylamine, and in the presence of a catalyst, such as Pd(OAc)₂, and (+)BINAP. The reaction is heated to approximately 95-100 °C and allowed to react for approximately overnight or less as needed for the reaction to go to completion to form **4a. 4a** is then optionally purified by column chromatography.

**5a** is prepared by treating **4a** with DBU optionally in the presence of a base such as DIEA at about room temperature. The reaction mixture is then heated to reflux or about 170 °C and allowed to proceed until completion, approximately 5-15 h. After evaporation of the solvent, the residue is triturated with acetone and collected by filtration to yield 5. Alternatively, the reaction is allowed to cool to room temperature and then purified directly by column chromatography.

**6a** is prepared by reacting **5a** with a brominating agent such as Br₂ in a solvent such as DCM at about room temperature. Then the reaction mixture is stirred for approximately overnight. The resulting product is filtered and then suspended in a solvent such as DCM and treated with a base such as triethylamine. The mixture is then washed with water and dried over a drying agent such as Na₂SO₄ to yield **6a.** Alternatively, after completion, the reaction mixture is partially concentrated and acetone is added, followed by concentrating and precipitating in a solvent such as ethyl acetate which can then be collected by filtration to yield **6a.**

A Suzuki coupling can be performed on **6** using a boronic acid (or ester) of formula R²⁸B(OH)₂ in a solvent such as a DME-H₂O mixture or such as a dioxane-H₂O mixture, in the presence of a catalyst such as Pd(dpppf)₂ and in the presence of a base such as triethylamine. The reaction mixture is heated to reflux or aobut 95-100 °C for approximately 4 h. After cooling to room temperature, the reaction mixture is partitioned with water and ethyl acetate. After separation, the organic layer is dried over a drying agent such as Na₂SO₄ to yield a compound of formula **7a.**

Alternatively, a Stille coupling can be performed on **6a** (either as the free base or as a salt such as an HBr salt) using a tin reagent of formula R²⁸Sn(*n*-Bu)₃, in a solvent such as toluene, in the presence of a catalyst such as Pd(PPh₃)₄, and optionally in the presence of a base such as triethylamine. The reaction is heated at about 110 °C for about four hours. After cooling to room temperature, the reaction mixture can be purified by column chromatography to yield a Compound of Formula II. Alternatively, after cooling to room temperature, 40% KF on alumina is added. The mixture is then filtered through Celite to remove the alumina and the Celite is then washed with a solvent such as ethyl acetate. The resulting filtrate can then be washed with 1 M aqueous KF and brine. The organic layers are dried over a drying agent such as MgSO₄, filtered and concentrated in vacuo. The residue can then be triturated with methylene chloride and hexane to yield a compound of formula **7a.**

The methylthio group of **7a** is then oxidized with m-CPBA in a solvent such as DCM at room temperature allowing to stir for approximately 4 h. After removal of the solvent under reduced pressure, the product is treated with with an amine of formula 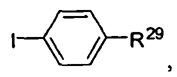 in a solvent such as dioxane and stirred at room temperature for approximately overnight to yield a Compound of the Invention of Formula II.

A Compound of Formula I, II, or III can be prepared according to Scheme 3 where R' is R¹ for a Compound of Formula III (as defined in the Summary of the Invention) and R' is R²⁰ for a Compound of Formula I (as defined in the Summary of the Invention) and R' is R³² for a Compound of Formula II (as defined in the Summary of the Invention); R² is as defined in the Summary of the Invention for a Compound of Formula III; R²¹ and R²² are as defined in the Summary of the Invention for a Compound of Formula I; R²⁸ and R²⁹ are as defined in the Summary of the Invention for a Compound of Formula II.

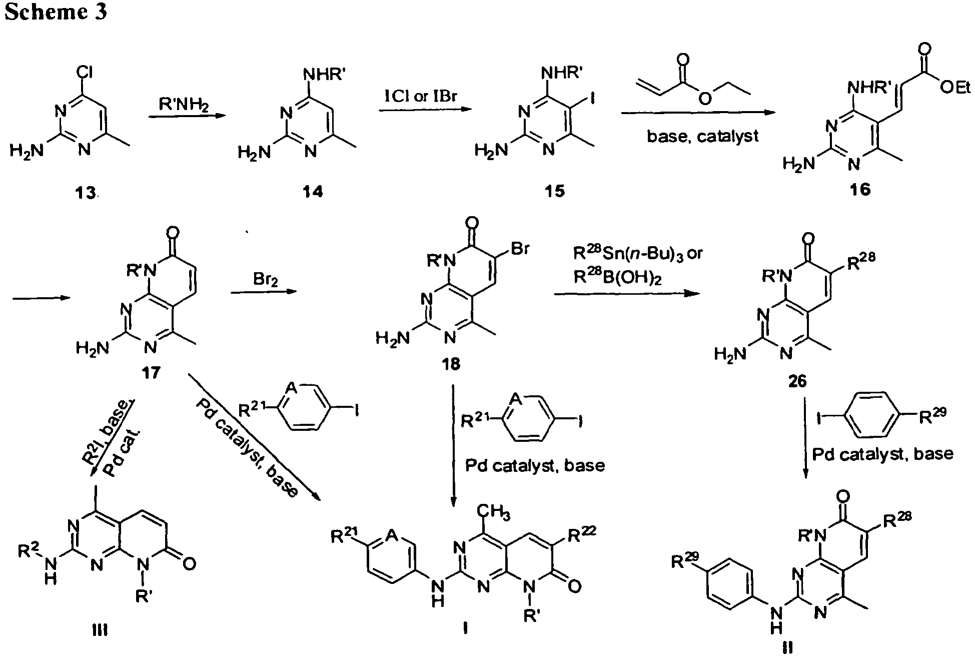

An intermediate of formula **14** is prepared by reacting an intermediate of formula **13** with a primary amine R'H₂ in a solvent such as water and with heating. **14** is then treated with iodine monochloride in a solvent such as methanol at around 0 °C and allowed to react for approximately overnight or less as needed for the reaction to go to completion to form **15.** After completion the residue is triturated with acetone. The intermediate **15** is then reacted in a solvent, such as DMA, with ethyl acrylate in the presence of a base, such as triethylamine, and in the presence of a catalyst, such as Pd(OAc)₂, and (+)BINAP. The reaction is heated to approximately 100 °C and allowed to react for approximately overnight or less as needed for the reaction to go to completion to form **16. 16** is then optionally purified by column chromatography. **26** can then be prepared from **16** by using the same reaction conditions as described in Scheme 1 (starting at the point of the preparation of **5a** from **4a**).

The intermediate **26** is then reacted with R²I, 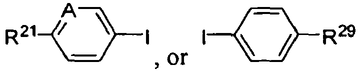 in the presence of a palladium (II) catalyst such as acetato(2'-di-*t*-butylphosphino-1,1'-biphenyl-2-yl)palladium (II) or chloro(di-2-norbornylphosphino)(2-dimethylaminomethylferrocen-1-yl)palladium (II) and a base such as sodium t-butoxide to provide a Compound of the Invention.

A compound of the invention where R' is R³², R²⁰, or R¹ where each is as defined in the Summary of the Invention for a Compound of Formula II, I, and III, respectively; R²⁸ is as defined in the Summary of the Invention for a Compound of Formula II; and where R^{b} is 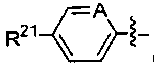 (for a Compound of Formula I) where R²¹ are as defined in the Summary of the Invention for a Compound of Formula I, or R^{b} is R² as defined in the Summary of the Invention for a Compound of Formula III, or where R^{b} is 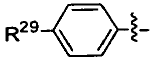 can alternatively be prepared according to Scheme 5.

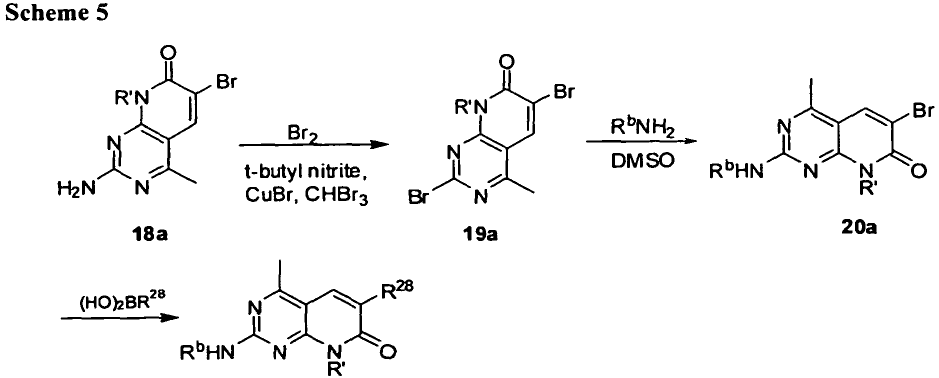

An intermediate of formula **18a** is prepared using the procedures described above in Scheme 3 can be brominated as depicted in Scheme 5. The intermediate **19a** is then reacted with an amine of formula R^{b}NH₂ to yield **20a** (a Compound of Formula I or III). **20a** is then reacted with a boronic acid to formula R²⁸ B(OH)₂ via a Suzuki couplingto yield a Compound of the Invention of Formula II.

A compound of Formula I or III where R' is R' as defined in the Summary of the Invention for a Compound of Formula III or R' is R²⁰ as defined in the Summary of the Invention for a Compound of Formula I and A is aryl or heteroaryl can alternatively be prepared according to Scheme 6.

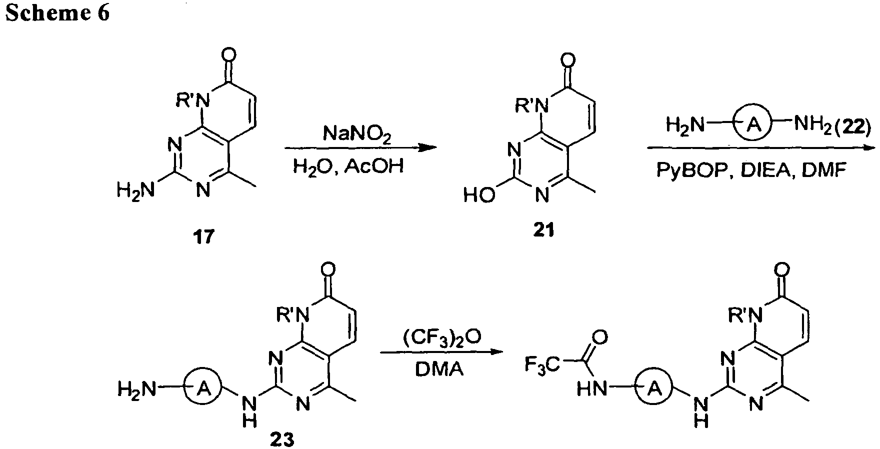

An intermediate of formula **17** is prepared as described above in Scheme 3 can be transformed to intermediate **21** which is then reacted with a diamine of formula **22** to yield an intermediate of formula **23** which then can be reacted to yield a Compound of Formula III.

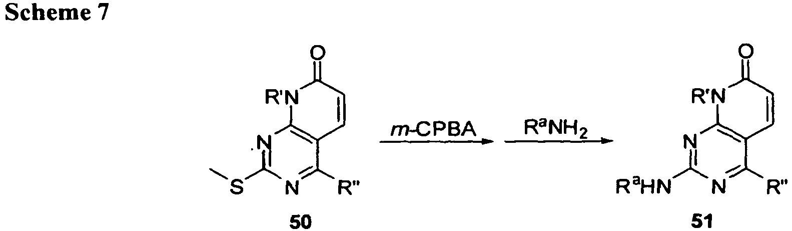

Subsequently, **50** can be oxidized to the corresponding sulfone with *m*-CPBA at room temperature and displaced with an amine of formula R^{a}NH₂ where to provide **51.**

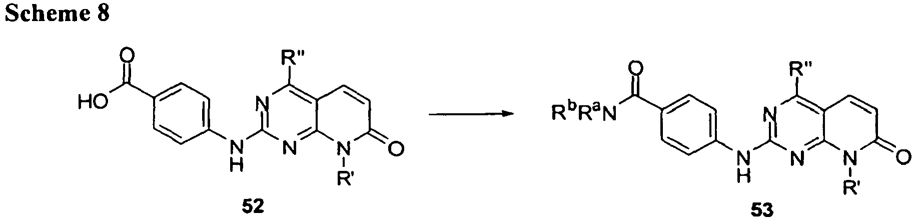

An intermediate of formula **52** can be treated with an amine of formula NHR^{a}R^{b} using standard amide formation conditions, for example with EDCI and HOBt, andin a solvent such as THF/H₂O.

### Synthetic Examples

### Synthesis 1

### 2-amino-8-ethyl-4-methyl-6-(1H-pyrazol-3-yl)pyrido[2,3-d]pyrimidin-7(8H)-one

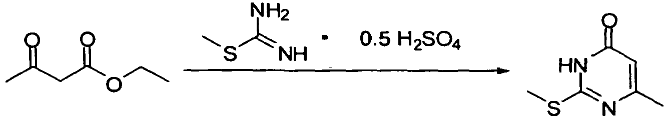

To a solution of 2-methyl-2-thiopseudourea sulfate (Aldrich, 58.74 g, 0.422 mol) in water (1000 mL) were added sodium carbonate (81.44 g, 0.768 mol) and ethyl acetoacetate (50 g, 0.384 mol) at room temperature. The reaction mixture was stirred overnight. After neutralizing to pH 8, the solid was collected through filtration followed by drying under vacuum overnight to afford 6-methyl-2-(methylthio)pyrimidin-4(3*H*)-one (57.2 g, 95% yield) of product. ¹H NMR (400 MHz, DMSO-d6): δ 12.47 (bs, 1H), 5.96 (bs, 1H), 2.47(s, 3H), 2.17 (s, 3H).

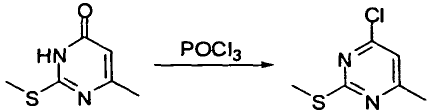

To the round bottom flask containing 6-methyl-2-(methylthio)pyrimidin-4(3*H*)-one (19 g, 121.6 mmol) was added POCl₃ (30 mL). The reaction mixture was heated to reflux for 2 h and then concentrated on a rotary evaporator to dryness. The crude 4-chloro-6-methyl-2-(methylthio)pyrimidine was used directly in the next reaction without further purification.

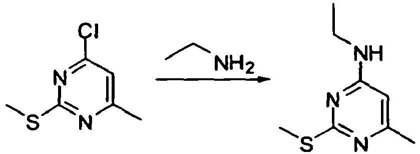

To the 4-chloro-6-methyl-2-(methylthio)pyrimidine from above was added 30 mL of a solution of 70% ethylamine in water. The reaction mixture was heated to 50 °C for 3 h. After completion, excess ethylamine was evaporated on rotary evaporator under vacuum. The solid was filtered and dried under vacuum to afford *N*-ethyl-6-methyl-2-(methylthio)pyrimidin-4-amine (20 g, 90% yield).

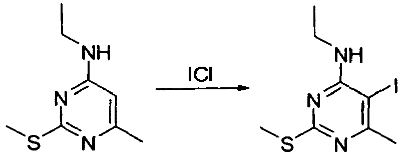

To the solution of *N*-ethyl-6-methyl-2-(methylthio)pyrimidin-4-amine (20 g, 121.6 mmol) in methanol was added iodine monochloride (26.58 g, 163.7 mmol) in small portions at 0 °C. Then the reaction mixture was stirred overnight. After evaporation of solvent, the residue was triturated with acetone. The product *N*-ethyl-5-iodo-6-methyl-2-(methylthio)pyrimin-4-amine (25.2 g, 75% yield) was collected by filtration. ¹H NMR (400 MHz, CDCl₃): δ 5.37 (bs, 1H), 3.52 (q, *J* = 7.2 Hz, 1H), 2.50 (s, 3H), 1.26 (t, *J* = 7.2 Hz, 3H).

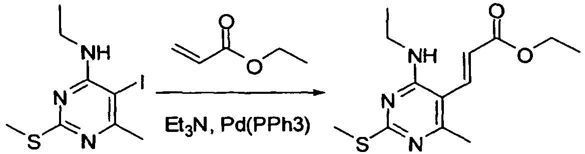

To the solution of *N*-ethyl-5-iodo-6-methyl-2-(methylthio)pyrimin-4-amine (25.2 g, 81.48 mmol) in DMA (260 mL) were added ethyl acrylate (12.23 g, 122.2 mmol), Pd(OAc)₂ (3.65 g, 16.25 mmol), (+)BINAP and triethyl amine (24.68 g, 244.4 mmol). Then the reaction mixture was heated to 100 °C and reacted overnight. After evaporation of solvent, the residue was diluted with water and the aqueous layer was extracted with ethyl acetate. The product (*E*)-ethyl-3-(4-(ethylamino)-6-methyl-2-(methylthio)pyrimidin-5-yl)acrylate (16.8 g, 73% yield) was isolated by silica gel column chromatography with 6-8% ethyl acetate in hexane as eluent. ¹H NMR (400 MHz, CDCl₃): δ 7.65 (d, *J* = 16.4Hz, 1H), 6.20 (d, *J* = 16.4Hz, 1H), 5.15 (bs, 1H), 4.28(q, *J* = 7.2 Hz, 2*H*), 3.54 (q, *J* = 7.2 Hz, 2*H*), 2.53 (s, 3H), 2.37 (s, 3H), 1.35 (t, *J* = 7.2 Hz, 3H), 1.24 (t, *J* = 7.2 Hz, 3H).

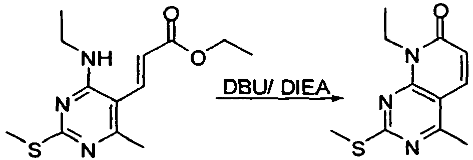

To a solution of (*E*)-ethyl-3-(4-(ethylamino)-6-methyl-2-(methylthio)pyrimidin-5-yl)acrylate (16.8 g, 59.8 mmol) in DIPEA was added 1,8-diazabicyclo[5.4.0]undec-7-ene (DBU, 18.21 g, 119.6 mmol) at room temperature. Then the reaction mixture was heated to reflux and reacted for 15 h. After evaporation of solvent, the residue was triturated with acetone. The product 8-ethyl-4-methyl-2-(methylthio)pyrido[2,3-d]pyrimidin-7(8*H*)-one (10.77 g, 77% yield) was collected by filtration. ¹H NMR (400 MHz, CDCl₃): δ 7.78 (d, *J* = 9.6 Hz, 1H), 6.63 (d, *J* = 9.6 Hz, 1H), 4.5(q, *J* = 7.2 Hz, 2H), 2.67 (s, 3H), 2.62 (s, 3H), 1.33 (t, *J* = 7.2 Hz, 3H).

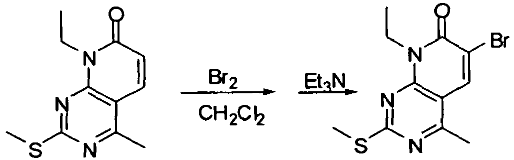

To a solution of 8-ethyl-4-methyl-2-(methylthio)pyrido[2,3-d]pyrimidin-7(8*H*)-one (6.31 g, 26.84 mmol) in DCM was added Br₂ (4.79 g, 29.52 mmol) dropwise at room temperature. Then the reaction mixture was stirred at room temperature overnight. After filtration the solid was suspended in DCM (100 mL), and triethylamine (20 mL) was added. The mixture was washed with water and dried with Na₂SO₄, and the product 6-bromo-8-ethyl-4-methyl-2-(methylthio)pyrido[2,3-d]pyrimidin-7(8*H*)-one (6.96 g, 83 % yield) was obtained after evaporation of DCM. ¹H NMR (400 MHz, CDCl₃): δ 8.22 (s, 1H), 4.56 (q, *J* = 7.2 Hz, 2H), 2.68 (s, 3H), 2.62 (s, 3H), 1.34 (t, *J* = 7.2Hz, 3H).

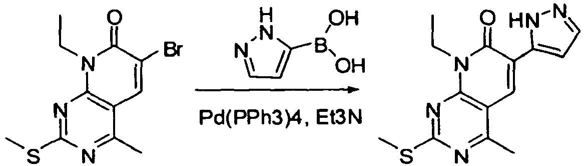

To a solution of 6-bromo-8-ethyl-4-methyl-2-(methylthio)pyrido[2,3-d]pyrimidin-7(8*H*)-one (0.765 g, 2.43 mmol) in DME-H₂O (10:1 11 mL) was added 1*H*-pyrazol-5-ylboronic acid (Frontier, 0.408 g, 3.65 mmol), [1,1'-bis(diphenylphosphino)ferrocene]dichloropalladium(II) complex with CH₂Cl₂ (Pd(dpppf),0.198 g, 0.243 mmol) and triethylamine (0.736 g, 7.29 mmol) at room temperature. Then the reaction mixture was heated to reflux and reacted for 4 h. After cooling down to room temperature, the reaction mixture was partitioned with water and ethyl acetate. After separation, the organic layer was dried with Na₂SO₄, and the product 8-ethyl-4-methyl-2-(methylthio)-6-(1H-pyrazol-5-yl)pyrido[2,3-d]pyrimidin-7(8*H*)-one (0.567 g, 77% yield) was obtained by silica gel column chromatography. ¹H NMR (400 MHz, CDCl₃): δ 13.3 (bs, 1H), 8.54 (s, 1H), 7.82-7.07 (m, 2H), 4.45 (q, *J* = 7.2 Hz, 2H), 2.71 (s, 3H), 2.60 (s, 3H), 1.26 (t, *J* = 7.2Hz, 3H).

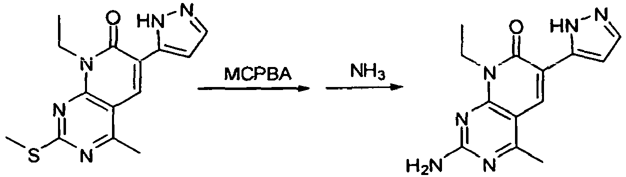

To the solution of 8-ethyl-4-methyl-2-(methylthio)-6-(1H-pyrazol-5-yl)pyrido[2,3-d]pyrimidin-7(8*H*)-one (0.123 g, 0.41mmol) in DCM (2 mL) was added MCPBA (0.176 g, 77%, 0.785 mmol) in a small portion at room temperature. Then the reaction mixture was stirred for 4 h. After evaporation of DCM, dioxane (1 mL) and liquid ammonia (1 mL) were introduced. The reaction was stirred at room temperature overnight. The product 2-amino-8-ethyl-4-methyl-6-(1H-pyrazol-3-yl)pyrido[2,3-d]pyrimidin-7(8*H*)-one (50.4 mg) was obtained by silica gel column chromatography. ¹H NMR (400 MHz, CD₃OD): δ 8.41 (s, 1H), 7.62 (d, *J* = 2.0 Hz, 1H), 6.96 (d, *J* = 2.0Hz, 1H), 4.51 (q, *J* = 7.2Hz, 2H), 2.64 (s, 3H), 1.29 (t, *J* = 7.2Hz, 3H); MS (EI) for C₁₃H₁₄N₆O: 271.3 (MH⁺).

### Synthesis 2

### Alternate route to (E)-ethyl-3-(4-(ethylamino)-6-methyl-2-(methylthio)pyrimidin-5-yl)acrylate

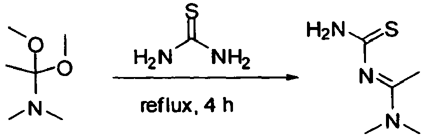

*N*,*N*-Dimethyl acetamide dimethyl acetal (75 g, 0.56 mole) was added to a suspension of thiourea (33.0 g, 0.43 mole) in methylene chloride. The mixture was heated under reflux for 4 h. The solvent was removed and the residue was crystallized from 5% MeOH and diethyl ether affording (1E)-*N*'-(aminocarbonothioyl)-*N*,*N-*dimethylethanimidamide (47.8 g, 76% yield).

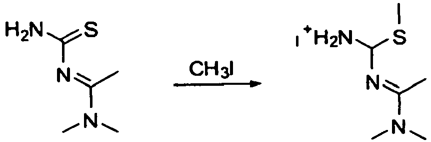

A suspension of (1E)-*N'*-(aminocarbonothioyl)-*N*,*N*-dimethylethanimidamide (47.8 g, 0.33 mole) in methyl iodide (150 mL) and THF (350 mL) was stirred for 18 h at room temperature. The mixture was evaporated under reduced pressure. After addition of 5% MeOH and diethyl ether, the compound precipitated and was collected by filtration affording (1E)-*N'*-[amino(methylthio)methyl]-*N*,*N*-dimethylethanimidamide hydrogen iodide salt (91.0 g, 96% yield).

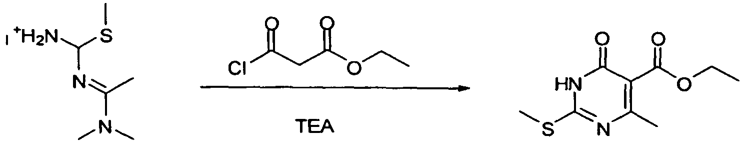

To a solution of (1E)-*N*'-[amino(methylthio)methyl]-*N*,*N-*dimethylethanimidamide hydrogen iodide salt (73.0 g, 0.26 mole) in dry dichloromethane (900 mL), was added ethyl 3-chloro-3-oxopropanoate (44 mL, 95% Lancaster, 0.34 mole) was added under a nitrogen atmosphere. The mixture was stirred for 4 h at room temperature, cooled to 0 °C then triethylamine (107 mL, 0.78 mole) was added. The reaction mixture was stirred overnight. The solvent was removed and H₂O was added. The pH was adjusted to pH = 5.0 with acetic acid and extracted with ethylacetate then evaporated and crystallized from the appropriate solvent (Ethylacetate-Hexanes mixture solvent, approximately 20% ethylacetate-Hexanes). This afforded ethyl 4-methyl-2-(methylthio)-6-oxo-1,6-dihydropyrimidine-5-carboxylate (36.5 g, 62% yield) after drying under vacuum.

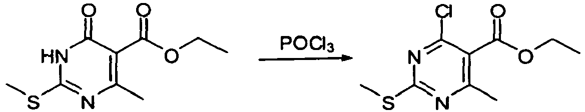

A solution of ethyl 4-methyl-2-(methylthio)-6-oxo-1,6-dihydropyrimidine-5-carboxylate (60 g, 0.26 mole) and phosphorous oxychloride (POCl₃, 320 mL) was heated under reflux for 4 to 5 h (monitor reaction by TLC using 30% ethylacetate and hexanes). After completion of reaction, phosphorous oxychloride was removed on a rotary evaporator. The residue was poured on to ice water and extracted with ethylacetate several times. The combined organic layers were evaporated, on a rotary evaporator, to give crude ethyl 4-chloro-6-methyl-2-(methylthio)pyrimidine-5-carboxylate (65 g). This compound was used without purification.

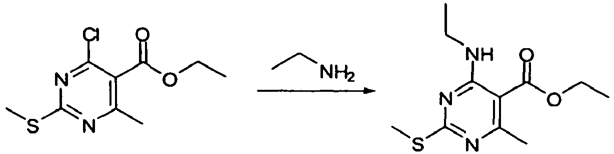

To a solution of ethyl 4-chloro-6-methyl-2-(methylthio)pyrimidine-5-carboxylate (65 g) in THF (1000 mL) and triethylamine (110 mL, 0.81 mole) was added ethylamine (2.0 M in THF, 0.81 mole) at 0 °C. This reaction mixture was stirred at room temperature overnight and then solvents were removed on a rotary evaporator. H₂O was added and the mixture extracted with ethyl acetate several times. Solvents from the combined organic layers were removed on a rotary evaporator affording 58 g (86% yield) of ethyl 4-(ethylamino)-6-methyl-2-(methylthio)pyrimidine-5-carboxylate. This material was used as such without further purification.

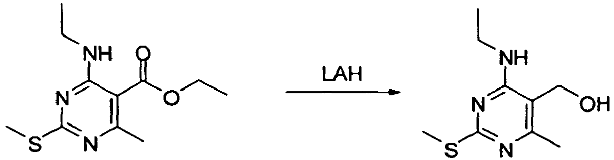

To a lithium aluminum hydride solution (LAH, 1.0 M solution in THF, Aldrich, 450 mL) was added a solution of ethyl 4-(ethylamino)-6-methyl-2-(methylthio)pyrimidine-5-carboxylate (57 g) in THF (1000 mL). The reaction mixture was stirred overnight. After cooling to 0 °C, the reaction mixture was cautiously quenched with a 1:9 mixture of H₂O/THF until gas evolution has ceased, then diluted with H₂O (500 mL) and stirred well for 2 h. The resulting slurry was extracted with ethylacetate several times. The aqueous layer was then filtered through Celite and washed with ethylacetate again. The combined organic layers were washed with brine, dried and concentrated under reduced pressure to give 41.0 g (85% yield) of [4-(ethylamino)-6-methyl-2-(methylthio)pyrimidin-5-yl]methanol as a light yellow crystal, which was used without purification in the next step.

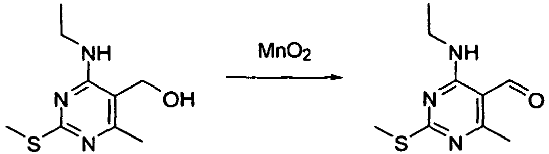

To a solution of [4-(ethylamino)-6-methyl-2-(methylthio)pyrimidin-5-yl]methanol (41.0 g) in chloroform (4000 mL) was added manganese oxide (125 g, 1.4 mole) and stirred for 4 h at room temperature. More manganese oxide was added until the disappearance of alcohol compound was observed. The reaction mixture was filtered through Celite and washed with some chloroform and evaporated all organic solvents to give 38 g (92% yield) of 4-(ethylamino)-6-methyl-2-(methylthio)pyrimidine-5-carbaldehyde as a colorless solid, which was used without purification in the next step.

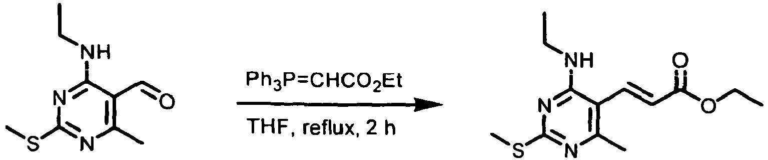

To a solution of 4-(ethylamino)-6-methyl-2-(methylthio)pyrimidine-5-carbaldehyde (38 g, 180 mmol) in THF (500 mL) was added (Carbethoxymethylene) triphenylphosphorane (95%, Aldrich, 85.18 g, 244 mmol). The reaction mixture was heated to reflux for 1.5 h and was monitered by TLC (4:1 hexanes/ethylacetate). The reaction was cooled to room temperature and was concentrated on a rotary evaporator. It was directly subjected to column chromatography (4:1 hexanes/ethylacetate) to give (*E*)-ethyl-3-(4-(ethylamino)-6-methyl-2-(methylthio)pyrimidin-5-yl)acrylate as a white crystal, 46.14 g (91% yield).

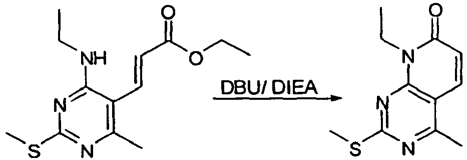

(*E*)-ethyl-3-(4-(ethylamino)-6-methyl-2-(methylthio)pyrimidin-5-yl)acrylate was converted to 8-ethyl-4-methyl-2-(methylthio)pyrido[2,3-d]pyrimidin-7(8*H*)-one as described above in Intermediate 1.

### Synthesis 3

### 2-Amino-6-bromo-8-ethyl-4-methylpyrido[2,3-d]pyrimidin-7(8H)-one

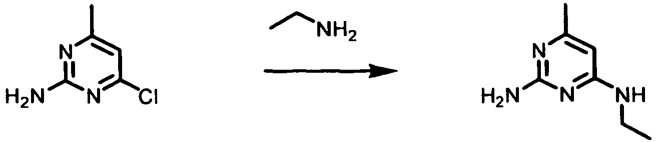

To a 3-necked 3-L flask, that was equipped with an overhead stirrer, was added in order 2-amino-4-chloro-6-methylpyrimidine (Aldrich, 100 g, 0.696 mol, 1 equiv.), ethylamine (70% ethylamine in water, Lancaster, 625 mL), 625 mL H₂O, and 125 mL TEA (0.889 mol, 1.28 equiv.). The mixture was stirred and heated at reflux for 20 h, during which time the reaction turned homogeneous. The reaction was allowed to cool to room temperature. The volatile ethylamine was removed on a rotary evaporator. A precipitate formed. The aqueous mixture containing the precipitate was allowed to stand at room temperature for 2 h and then filtered. After drying under vacuum, 106 g (100% yield) of 2-amino-6-ethylaminopyrimidine was obtained as a colorless solid. This material was used as such in the following reaction.

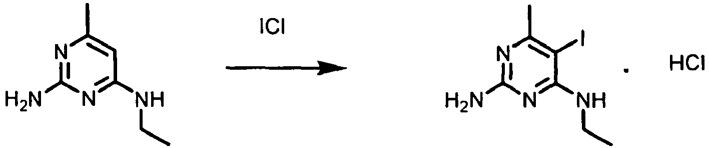

To a solution of 2-amino-6-ethylaminopyrimidine (98 g, 0.64 mol) in methanol (1.6 L) was added ICl (115.0 g, 0.71 mol) in a small portion at 15 °C. Then the reaction mixture was stirred at room temperature for 3 h (monitored by LC/MS). After evaporation of solvent by rotary evaporator, the residue was triturated with acetone. 2-amino-6-ethylamino-4-iodopyrimidine hydrochloride (188.5 g, 93% isolated yield) was obtained by vacuum filtration and drying. ¹H NMR (400 MHz, CD₃OD) δ 3.58 (q, 2H), 2.14 (s, 3H), 1.11 (t, 3H); MS (EI) for C₇H₁₁N4ClI: 279.1 (MH⁺).

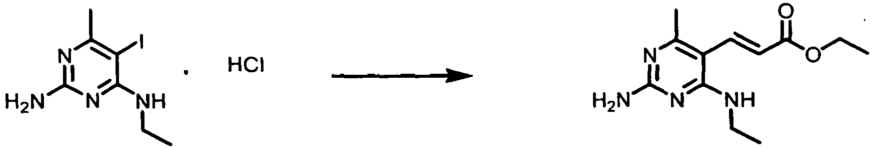

To a three-neck round bottom flask equipped with over-head mechanic stirrer were added 2-amino-6-ethylamino-4-iodopyrimidine hydrochloride (188.5 g, 0.60 mol), ethyl acrylate (221 mL, 2.0 mol), triethylamine (285 mL, 2.0 mol), DMF (1.3 L), and tetrakis(triphenylphosphine)palladium(0) (Pd(PPh₃)₄, 31.3 g, 0.027 mol). The reaction mixture was heated to 95 °C and stirred for 3 h (monitored by LC/MS). After reaction completion, the reaction mixture was evaporated about to 1/10 of original volume and partitioned with 500 mL of ethyl acetate and 1000 mL of water. The aqueous layer was extracted with ethyl acetate 5 times. (*E*)-Ethyl 3-(2-amino-4-(ethylamino)-6-methylpyrimidin-5-yl)acrylate (100 g, 67% yield) was obtained by recrystalization from acetone after evaporation of ethyl acetate. ¹H NMR (400 MHz, CD₃OD) δ 7.48 (dd, *J1* = 16.0 Hz, *J2* = 4.0 Hz, 1H), 6.20 (dd, *J1* = 16 Hz, *J2* = 4 Hz, 1H), 4.25 (q, *J* = 7.2 Hz, 2*H*), 3.51 (q, *J* = 7.6 Hz, 2H), 2.39 (s, 3H), 1.3 (t, *J* = 7.2 Hz, 3H), 1.2 (t, *J* = 7.6 Hz, 3H). MS (EI) for C₁₂H₁₈N₄O₂: 251.3 (MH⁺).

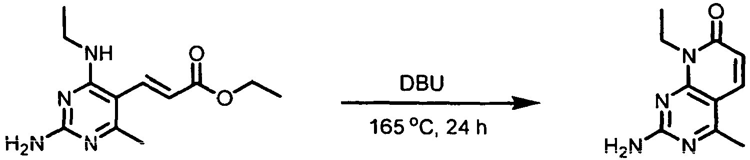

(*E*)-Ethyl 3-(2-amino-4-(ethylamino)-6-methylpyrimidin-5-yl)acrylate (4.50 g, 18.0 mmol) was added to DBU (10.95 g, 4.0 equiv.) and the mixture was heated to 165 °C and stirred for 24 h. After that, the mixture was cooled to 70 °C followed by the addition of H₂O (20 mL) to precipitate crystal and stirred for 1h at room temperature. The crystal was collected and washed with H₂O and acetone and dried under vacuum to afford 2.70 g (73.5% yield of 2-amino-8-ethyl-4-methylpyrido[2,3-d]pyrimidin-7(8*H*)-one as a light yellowish brown solid. LC/MS: Calculated for C₁₀H₁₂N₄O (204.2). Found: 205.31 (MH⁺); HPLC analytical purity: 98.5%.

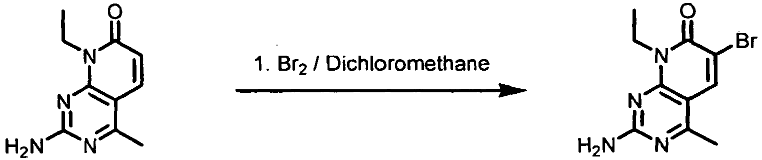

2-Amino-8-ethyl-4-methylpyrido[2,3-d]pyrimidin-7(8*H*)-one (2.70 g, 13.2 mmol) was added to dichloromethane (100 mL), and then bromine (0.75 mL, 1.10 equiv.) was added slowly. This reaction mixture was stirred for 3 h at room temperature. After that, the solvent was evaporated nearly 80% volume of reaction mixture under vacuum, and then acetone was added to give 3.54 g 2-Amino-6-bromo-8-ethyl-4-methylpyrido[2,3-d]pyrimidin-7(8*H*)-one as a tan solid. LC/MS: Calculated for C₁₀H₁₁BrN₄O (283.12). Found: 285.15 (M+2). HPLC analytical purity: 97.7%.

### Synthesis 4

### 8-isopropyl-4-methyl-2-(methylsulfonyl)pyrido[2,3-d]pyrimidin-7(8H)-one

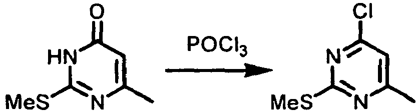

POCl₃ (250 mL) was added to a 500 mL round bottom flask charged with 15.0 g of 6-methyl-2-(methylthio)pyrimidin-4(3H)-one. The reaction mixture was topped with a Vigreux column and allowed to stir under nitrogen at 90 °C. After 4 h, LCMS indicated desired product had formed in high yield. POCl₃ was removed by rotary evaporation and azeotroped with 2 x 300 mL CHCl₃ and 2 x 300 mL toluene. 4-Chloro-6-methyl-2-(methylthio)pyrimidine as a thick yellow oil was placed under heavy vacuum for several hours, and then used immediately in the next reaction.

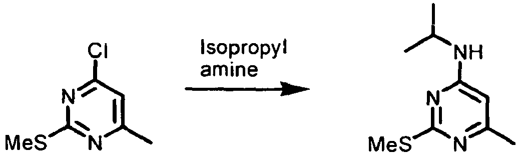

Isopropylamine (100 mL) was added very slowly to 22.0 g (125.96 mmol) of crude 4-chloro-6-methyl-2-(methylthio)pyrimidine in 70 mL of THF in a 350 mL pressure tube. NOTE: Residual POCl₃ caused smoking and bubbling, therefore the addition was done at 0 °C. Once acid was quenched, an additional 50 mL of isopropylamine was added and the pressure tube was sealed. The reaction vessel was brought to 60 °C and stirred overnight. The LCMS indicate very little starting material. Solvent was evaporated leaving *N*-isopropyl-6-methyl-2-(methylthio)pyrimidin-4-amine as a viscous yellow oil. The crude material was taken directly to the next step.

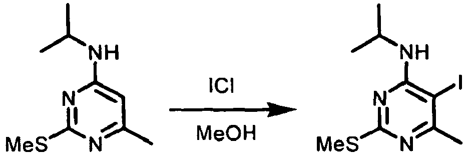

To a crude solution of *N*-isopropyl-6-methyl-2-(methylthio)pyrimidin-4-amine (44.6 g, 224 mmol), prepared using analogous procedures as described in Example 1, in 400 mL of methanol was added ICl (40.0 g, 246 mmol) in small portions at room temperature. The reaction mixture was then stirred at for 3 h monitoring by LC/MS. After evaporation of solvent by rotary evaporator, the residue was triturated with acetone to yield 5-iodo-*N*-isopropyl-6-methyl-2-(methylthio)pyrimidin-4-amine. ¹H NMR (400 MHz, CDCl₃) δ 6.37 (br m, 1H), 4.47 (m, 1H), 2.78 (s, 3H), 2.67 (s, 3H), 1.41 (d, *J* = 6.4, 6H).

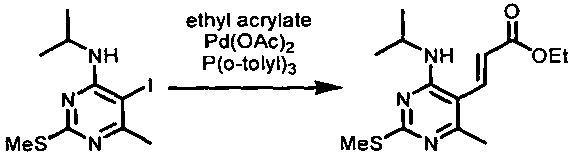

5-Iodo-*N*-isopropyl-6-methyl-2-(methylthio)pyrimidin-4-amine (8.1 g, 26.2 mmol), ethyl acrylate (5.24 g, 52.4 mmol), triethylamine (10.6 g, 105 mmol), palladium (II) acetate (1.17 g, 5.23 mmol), and tri-o-tolyl phosphine (1.59 g, 5.23 mmol) were added in that order to 10.8 mL of DMA in a pressure tube and sealed. The reaction mixture was heated to 100 °C and allowed to stir overnight. The reaction was quenched by filtration through a short silica plug washing with ACN. The solvent was evaporated and diluted with ethyl acetate then extracted with 10 % aqueous LiCl, followed by water and brine. NOTE: Extraction is necessary to remove all DMA giving resolution in chromatography. The sample was purified by silica gel column chromatography using 20 % ethyl acetate/hexane as eluent. Desired fractions were combined and reduced to afford 2.5 g (34 % yield) of ethyl (2E)-3-[4-(isopropylamino)-6-methyl-2-(methylthio)pyrimidin-5-yl]acrylate as a yellow/orange oil.

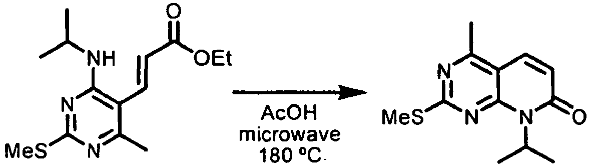

(*E*)-Ethyl 3-(4-(isopropylamino)-6-methyl-2-(methylthio)pyrimidin-5-yl)acrylate (2.5 g, 8.46 mmol) was dissolved in acetic acid by gentle warming. Sample was placed in microwave reactor for 6 h at 180 °C, 300 W, and 200 PSI. The product was purified by silica gel column chromatography eluting with 20 % ethyl acetate/hexane. Desired fractions were combined and reduced into 8-isopropyl-4-methyl-2-(methylthio)pyrido[2,3-d]pyrimidin-7(8*H*)-one as a yellow powder (1.20 g, 57 % yield) which was then dried under heavy vacuum overnight. ¹H NMR (400MHz, CDCl₃) δ 7.74 (d, *J* = 9.6, 1H), 6.58 (d, *J* = 9.6, 1H), 5.84 (br s, 1H), 2.65 (s, 3H), 2.63 (s, 3H), 1.63 (d, *J* = 6.8, 6H).

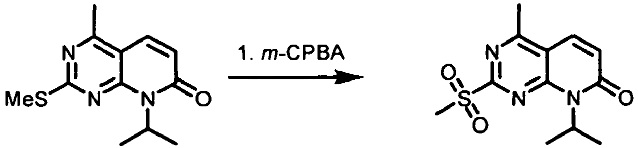

8-Isopropyl-4-methyl-2-(methylthio)pyrido[2,3-d]pyrimidin-7(8*H*)-one (5.38 g, 21.59 mmol) was dissolved in 100 mL DCM. To the stirring solution, m-CPBA (13.97 g, 64.78 mmol) was added. The reaction was allowed to stir for 2.5 h at room temperature. LCMS indicated reaction had gone to completion. Sample was diluted with 300 mL of DCM and 300 mL K₂CO₃, upon addition of base a white precipitate formed that dissolved in excess H₂O. Organic layer was extracted further with H₂O and brine, and then dried over Na₂CO₃. The solvent was evaporated to afford 8-isopropyl-4-methyl-2-(methylsulfonyl)pyrido[2,3-d]pyrimidin-7(8*H*)-one (6.0 g, 99 % yield) as a light yellow oil that was used immediately in the next reaction.

Using the same or analogous synthetic techniques and substituting with appropriate reagents, 8-cyclopentyl-4-methyl-2-(methylsulfonyl)pyrido[2,3-d]pyrimidin-7(8*H*)-one was prepared.

### Synthesis 5

### 2-amino-8-isopropyl-4-methylpyrido[2,3-d]pyrimidin-7(8H)-one

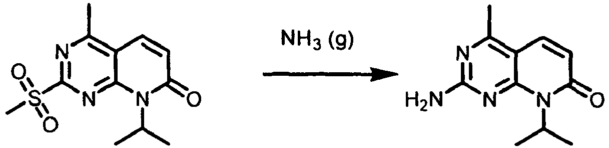

8-isopropyl-4-methyl-2-(methylsulfonyl)pyrido[2,3-d]pyrimidin-7(8*H*)-one (approximately 3.0 g) was dissolved in 50 mL THF, in a 350 mL pressure tube. While stirring, NH₃ (g) was bubbled in through solution for 1.5 minutes. A color change was observed form light yellow to olive green in about 120 seconds. The tube was sealed and stirred at room temperature overnight. A precipitate had formed. The reaction mixture, including precipitate, was reduced to near dryness, filtered and washed with a minimal volume of cold THF, affording 2.88 g of 2-amino-8-isopropyl-4-methylpyrido[2,3-d]pyrimidin-7(8*H*)-one.

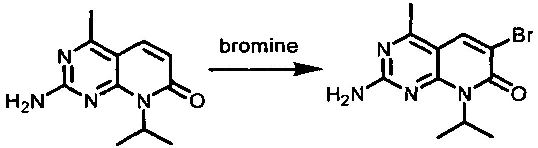

To a solution of 2-amino-8-isopropyl-4-methylpyrido[2,3-d]pyrimidin-7(8*H*)-one (2.88 g, 13.19 mmol) dissolved in 80 mL of DCM at 0 °C, (4.21 g, 26.39 mmol) bromine was added. Reaction vessel was removed from ice bath and allowed to react at room temperature over night. LCMS indicated complete conversion of starting material to product. Sample was evaporated to remove DCM and excess bromine. Orange solid was diluted in ethyl acetate and extracted with 10 % NaHSO₃, H₂O, and brine. Organic layer was dried over Na₂SO₄, filtered, and reduced to dryness yielding 2-amino-6-bromo-8-isopropyl-4-methylpyrido[2,3-d]pyrimidin-7(8*H*)-one as a light yellow powder (2.2 g, 56% yield). ¹H NMR (400MHz, CDCl₃) δ 8.08 (s, 1H), 5.83 (m, 1H), 5.69 (br s, 2H), 2.60 (s, 3H), 1.58 (d, *J* = 6.8, 6H).

Using the same or analogous synthetic techniques and substituting with appropriate reagents, the following intermediates were prepared: 6-bromo-8-isopropyl-4-methyl-2-(methylsulfonyl)pyrido[2,3-d]pyrimidin-7(8*H*)-one and 6-bromo-8-cyclopentyl-4-methyl-2-(methylsulfonyl)pyrido[2,3-d]pyrimidin-7(8*H*)-one.

### Synthesis 6

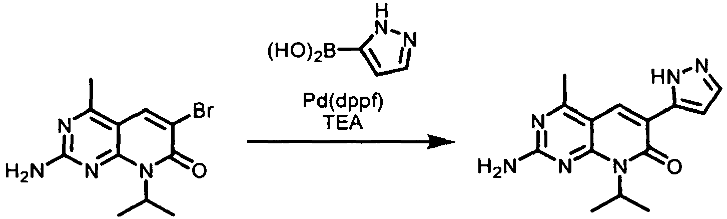

In a 350 mL pressure tube 2-amino-6-bromo-8-isopropyl-4-methylpyrido[2,3-d]pyrimidin-7(8*H*)-one (1.50 g, 5.05 mmol), 1*H*-pyrazol-3-yl boronic acid (1.12 g, 10.09 mmol), K₂CO₃ (336 mg, 15.1 mmol), and tetrakis(triphenylphosphine) palladium (0) (583 mg, 0.0504 mmol) were dissolved in 50 mL dioxane and 5 mL H₂O. The tube was sealed, heated to 100 °C and allowed to react overnight. A color change was observed. LCMS indicated no presence of starting material. The sample was filtered through a syringe filter and evaporated to dryness. The compound was dissolved in ethyl acetate and triturated in hexane. Light yellow powder of 2-amino-8-isopropyl-4-methyl-6-(1*H*-pyrazol-5-yl)pyrido[2,3-d]pyrimidin-7(8*H*)-one (195 mg, 13.7% yield) was found to be 98% pure by HPLC. ¹H NMR (400MHz, CDCl₃) δ 12.97 (br s, 1H), 8.35 (s, 1H), 7.60 (br s, 1H), 7.21 (s, 2*H*), 6.94 (s, 1H), 5.86 (br s, 1H), 2.50 (m, 6H), 1.54 (s, 3H), MS (EI) for C₁₄H₁₆N₆O: 285.0 (MH⁺).

### Synthesis 7

### 2-Amino-4-methyl-8-(phenylmethyl)-6-(1H-pyrazol-3-yl)pyrido[2,3-d]pyrimidin-7(8H)-one

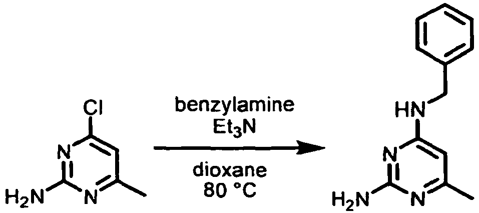

Triethylamine (3.4 mL, 24.6 mmol) was added to a suspension of 2-amino-4-chloro-6-methylpyrimidine (Aldrich, 1.77 g, 12.3 mmol) and benzylamine (1.98 g, 18.5 mmol) in anhydrous dioxane (20 mL). The reaction was heated to 80 °C and allowed to run for 12 h. Upon cooling to room temperature, a white precipitate formed which was collected by vacuum filtration. The solid was recrystallized from acetone: hexanes to afford N⁴-benzyl-6-methylpyrimidine-2,4-diamine (2.33 g, 89 % yield) as a white solid.

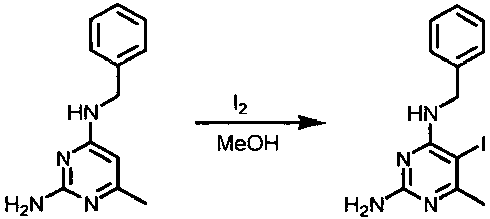

Iodine (3.04 g, 12.0 mmol) was added to a solution of *N⁴*-benzyl-6-methylpyrimidine-2,4-diamine (2.33 g, 10.9 mmol) in anhydrous MeOH (50 mL) at 0 °C. The reaction was allowed to warm to room temperature overnight. After 12 hours, an additional 0.5 equiv of iodine was added, and the reaction warmed to 50 °C. After four hours, the reaction was cooled to room temperature and concentrated in vacuo. The residue was diluted with ethyl acetate (200 mL) and washed with 10% NaHSO₃ (200 mL). The aqueous phase was separated and washed once more with ethyl acetate (200 mL). The organic phases were combined, washed with brine, separated and dried over Na₂SO₄, The filtrate was concentrated in vacuo to afford the product *N⁴*-benzyl-5-iodo-6-methylpyrimidine-2,4-diamine (3.14 g, 85 % yield).

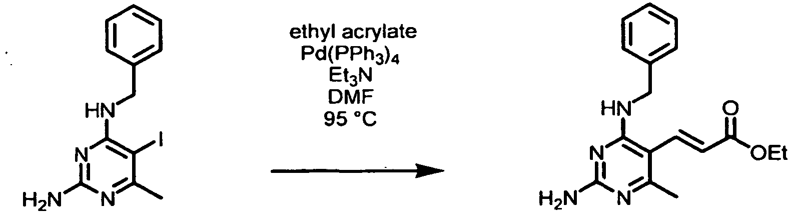

Triethylamine (7.60 mL, 54.5 mmol) was added to a suspension of *N⁴*-benzyl-5-iodo-6-methylpyrimidine-2,4-diamine (3.14 g, 10.9 mmol), ethyl acrylate (3.55 mL, 32.7 mmol) and Pd(PPh₃)₄ (629 mg, 0.545 mmol) in anhydrous DMF (20 mL). The reaction was heated to 95 °C under nitrogen. After 24 h, the reaction was allowed to cool to room temperature and concentrated in vacuo. The residue was poured into a 10% solution of LiCl and washed with ethyl acetate (100 mL). The organic phase was separated and washed with brine, separated and dried over Na₂SO₄. The filtrate was concentrated in vacuo and purified on SiO₂ (3:2 methylene chloride: ethyl acetate) to afford (*E*)-ethyl-3-(2-amino-4-(benzylamino)-6-methylpyrimidin-5-yl)acrylate (0.954 g, 28 % yield) as a light yellow solid.

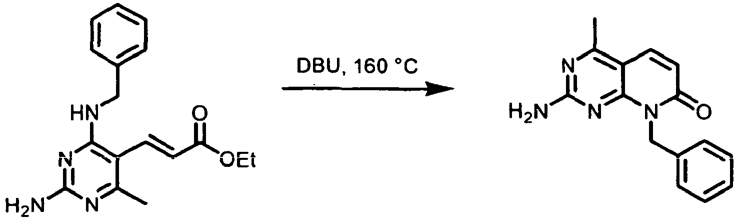

2-amino-4-methyl-8-(phenylmethyl)pyrido[2,3-*d*]pyrimidin-7(8*H*)-one Diazabicyclo[5.4.0]undec-7-ene (DBU) (1.83 mL, 12.2 mmol) was added to a flask charged with (*E*)-ethyl-3-(2-amino-4-(benzylamino)-6-methylpyrimidin-5-yl)acrylate (0.954 g, 3.05 mmol) and the reaction refluxed at 160 °C under a nitrogen atmosphere. After 20 hours, the reaction was cooled to room temperature and concentrated in vacuo. Purification on SiO₂ (1:1 methylene chloride: ethyl acetate) afforded the product (0.508 g, 62 % yield) as an off-white solid.

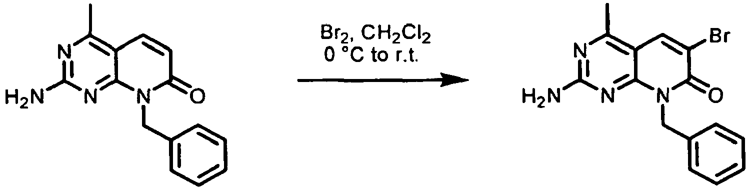

Bromine (72 µL, 1.40 mmol) was added to a suspension of 2-amino-4-methyl-8-(phenylmethyl)pyrido[2,3-*d*]pyrimidin-7(8*H*)-one (0.340 g, 1.27 mmol) in methylene chloride (20 mL) at 0 °C. The reaction was allowed to warm to room temperature over one hour and the resulting precipitate collected by vacuum filtration to afford 2-amino-6-bromo-4-methyl-(8-phenylmethyl)pyrido[2,3-*d*]pyrimidin-7(8*H*)-one (0.435 g, 99 % yield) after drying. The yellow solid was used in the next step without further purification.

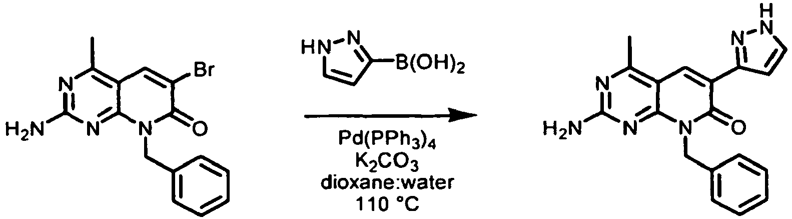

A 10:1 solution of dioxane and water (11 mL) was added to a flask charged with 2-ainino-6-bromo-4-methyl-(8-phenylmethyl)pyrido[2,3-*d*]pyrimidin-7(8*H*)-one (0.435 g, 1.27 mmol), 1*H*-pyrazole-5-boronic acid (0.284 g, 2.54 mmol), Pd(PPh₃)₄ (0.073 mg, 0.063 mmol), and K₂CO₃ (0.527 g, 3.81 mmol). The flask was flushed with nitrogen and fitted with a reflux condenser and heated to 110 °C. After 12 h the reaction was cooled to room temperature and diluted with ethyl acetate (100 mL) and washed with water. The aqueous phase was acidified to pH 1.0 and washed with ethyl acetate (100 mL). The organic phases were combined and washed with brine, separated and dried over Na₂SO₄, filtered and concentrated in vacuo. The residue was precipitated with ethyl acetate to give 2-Amino-4-methyl-8-(phenylmethyl)-6-(1H-pyrazol-3-yl)pyrido[2,3-*d*]pyrimidin-7(8*H*)-one (0.062 g, 15 % yield) as a yellow solid: ¹H NMR (400 MHz, DMSO-*d₆*): δ 13.10 (bs, 1H), 12.93 (bs, 1H), 8.47 (s, 1H), 7.76 (bs, 1H), 7.51 (bs, 1H), 7.28 (m, 5H), 6.97 (s, 1H), 5.55 (s, 2*H*), 2.55 (bs, 3H); MS (EI) for C_{18H16}N₆O: 333.1 (MH⁺).

### Synthesis 8

### 2-Amino-8-ethyl-4-methyl-6-(4-methyl-3-thienyl)pyrido[2,3-d]pyrimidin-7(8H)-one

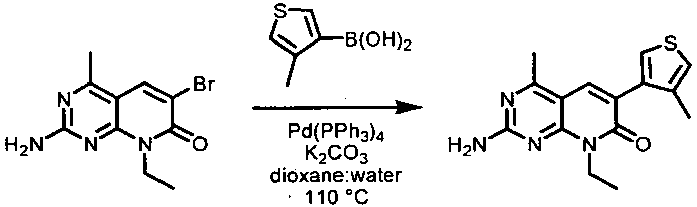

A 3:1 solution of dioxane and water (4 mL) was added to a flask charged with 2-amino-6-bromo-8-ethyl-4-methylpyrido[2,3-*d*]pyrimidin-7(8*H*)-one (0.140 g, 0.495 mmol) from above, 4-methylthiophene-3-boronic acid (0.140 g, 0.989 mmol), Pd(PPh₃)₄ (0.057 mg, 0.050 mmol), and K₂CO₃ (0.205 g, 1.48 mmol). The flask was flushed with nitrogen and fitted with a reflux condenser and heated to 100 °C. After 12 hours the reaction was cooled to room temperature and diluted with ethyl acetate (70 mL) and washed with water. The aqueous phase was separated and washed with an additional amount of ethyl acetate (70 mL). The organic phases were combined and washed with brine, separated and dried over Na₂SO₄, filtered and concentrated in vacuo. The residue was purified on SiO₂ (1:1 methylene chloride: ethyl acetate) to give 2-Amino-8-ethyl-4-methyl-6-(4-methyl-3-thienyl)pyrido[2,3-*d*]pyrimidin-7(8*H*)-one (0.081 g, 55 % yield) as an off-white solid: ¹H NMR (400 MHz, DMSO-*d₆*): δ 7.84 (s, 1H), 7.46 (d, *J* = 4.0 Hz, 1H), 7.19 (m, 3H), 4.32 (q, *J* = 8.0 Hz, 2H), 2.52 (s, 3H), 2.11 (bs, 3H), 1.19 (t, *J* = 8.0 Hz, 3H); MS (EI) for C₁₅H₁₆N₄OS: 301.1 (MH⁺).

Using the same or analogous synthetic techniques and substituting with appropriate reagents, the following intermediates were prepared:
**Intermediate 9:** 2-Amino-8-ethyl-4-methyl-6-(3-thienyl)pyrido[2,3-d]pyrimidin-7(8*H*)-one: ¹H NMR (400 MHz, CDCl₃): δ 8.11 (dd, *J* = 2.8, 1.2 Hz, 1H), 7.95 (s, 1H), 7.51 (dd, *J* = 5.2, 1.2 Hz, 1H), 7.37 (dd, *J =* 4.8, 3.2 Hz, 1H), 5.21, (bs, 2*H*), 4.48 (q, *J* = 6.8 Hz, 2*H*), 2.63 (s, 3H), 1.32 (t, *J* = 7.2 Hz, 3H); MS (EI) for C₁₄H₁₄OS: 287.0 (MH⁺).
**Intermediate 10:** 2-Amino-8-ethyl-6-furan-3-yl-4-methylpyrido[2,3-*d*]pyrimidin-7(8*H*)-one: ¹H NMR (400 MHz, CDCl₃): δ 8.47 (bs, 1H), 7.85 (s, 1H), 7.49 (t, *J* = 1.6 Hz, 1H), 6.77 (dd, *J* = 2.0, 0.8 Hz, 1H), 5.19, (bs, 2*H*), 4.48 (q, *J* = 6.8 Hz, 2*H*), 2.64 (s, 3H), 1.31 (t, *J* = 7.2 Hz, 3H); MS (EI) for C₁₄H₁₄N₄O₂: 271.1 (MH⁺).
**Intermediate 11:** 2-Amino-6-(3,5-dimethylisoxazol-4-yl)-8-ethyl-4-methylpyrido[2,3-*d*]pyrimidin-7(8*H*)-one: ¹H NMR (400 MHz, CDCl₃): δ 7.62 (s, 1H), 5.27, (bs, 2*H*), 4.44 (q, *J =* 7.2 Hz, 2*H*), 2.59 (s, 3H), 2.38 (s, 3H), 2.25 (s, 3H), 1.31 (t, *J* = 6.8 Hz, 3H); MS (EI) for C₁₅H₁₇N₅O₂: 300.1 (MH⁺).
**Intermediate 12:** 2-Amino-8-ethyl-6-isoxazol-4-yl-4-methylpyrido[2,3-*d*]pyrimidin-7(8*H*)-one: ¹H NMR (400 MHz, CDCl₃): δ 9.36 (s, 1H), 8.71 (s, 1H), 7.91 (s, 1H), 5.30, (bs, 2*H*), 4.48 (q, *J* = 7.2 Hz, 2*H*), 2.67 (s, 3H), 1.32 (t, *J* = 6.8 Hz, 3H); MS (EI) for C₁₃H₁₃NsO₂: 272.0 (MH⁺).
**Intermediate 13:** 2-Amino-8-ethyl-6-furan-2-yl-4-methylpyrido[2,3-*d*]pyrimidin-7(8*H*)-one: ¹H NMR (400 MHz, CDCl₃): δ 8.19 (s, 1H), 7.48 (d, *J* = 0.8 Hz, 1H), 7.37 (d, *J* = 3.6 Hz, 1H), 6.53 (dd, *J* = 3.6, 2.0 Hz 1H), 5.21, (bs, 2*H*), 4.48 (q, *J* = 7.2 Hz, 2*H*), 2.66 (s, 3H), 1.32 (t, *J* = 6.8 Hz, 3H); MS (EI) for C₁₄H₁₄N₄O₂: 271.0 (MH⁺).
**Intermediate 14:** 5-(2-Amino-8-ethyl-4-methyl-7-oxo-7,8-dihydropyndo[2,3-*d*]pyrimidin-6-yl)thiophene-2-carbonitrile: ¹H NMR (400 MHz, CDCl₃): δ 8.24 (s, 1H), 7.61 (d*, J* = 4.4 Hz, 1H), 7.55 (d, *J* = 4.4 Hz, 1H), 5.33, (bs, 2*H*), 4.48 (q, *J* = 7.2 Hz, 2*H*), 2.68 (s, 3H), 1.33 (t, *J* = 6.8 Hz, 3H); MS (EI) for C₁₅H₁₃N₅OS: 312.0 (MH⁺).
**Intermediate 15:** 2-Amino-8-ethyl-4-methyl-6-(1*H*-pyrazol-4-yl)pyrido[2,3-*d*]pyrimidin-7(8*H*)-one: ¹H NMR (400 MHz, DMSO-*d₆*): δ 12.88 (s, 1H), 8.38 (s, 1H), 8.17 (s, 2*H*), 7.10 (bs, 2*H*), 4.35 (q, *J* = 7.2 Hz, 2*H*), 2.59 (s, 3H), 1.20 (t, *J* = 7.2 Hz, 3H); MS (EI) for C₁₃H₁₄N₆O: 271.0 (MH⁺).
**Intermediate 16:** 2-Amino-8-ethyl-4-methyl-6-(1,3-thiazol-2-yl)pyrido[2,3-*d*]pyrimidin-7(8H)-one: ¹H NMR (400 MHz, CDCl₃): δ 8.94 (s, 1H), 7.94 (d, *J* = 3.2 Hz, 1H), 7.46 (d, *J* = 3.2 Hz, 1H), 5.34 (bs, *2H),* 4.54 (q, *J*= 7.2 Hz, *2H),* 2.73 (s, 3H), 1.35 (t, *J*= 7.2 Hz, 3H); MS (EI) for C₁₃H₁₃N₅OS: 288.0 (MH⁺).
**Intermediate 17:** 2-Amino-8-ethyl-4-methyl-6-(1-methyl-1*H-*pyrrol-2-yl)pyrido[2,3-*d*]pyrimidin-7(8*H*)-one: ¹H NMR (400 MHz, DMSO-*d₆*):δ 7.81 (s, 1H), 7.20 (bs, 2*H*), 6.81 6.11 (dd, *J* = 3.6, 2.0Hz, 1H), 6.02 (t, *J* = 3.2 Hz, 1H), 4.32 (q, *J* = 7.2 Hz, 2*H*), 3.49 (s, 3H), 2.52 (s, 3H), 1.19 (t, *J* = 7.2 Hz, 3H); MS (EI) for C₁₅H₁₇N₅O: 284.1 (MH⁺).
**Intermediate 18:** 2-Amino-8-ethyl-4-methyl-6-phenylpyrido[2,3-*d*]pyrimidin-7(8*H*)-one: ¹H NMR (400MHz, CDCl₃): δ 7.79 (s, 1H), 7.65 (d, *J =* 6.8 Hz, 2*H*), 7.43 (d, *J* = 7.2 Hz, 2*H*), 7.36 (d, *J* = 7.2 Hz, 1H), 5.24 (bs, 2*H*), 4.47 (q, *J =* 7.2 Hz, 2*H*), 2.60 (s, 3H), 1.31 (d, *J* = 7.2 Hz, 3H), MS (EI) for C₁₆H₁₆N₄O: 281.2 (MH⁺)
**Intermediate 19:** 2-Amino-8-ethyl-6-(4-methoxyphenyl)-4-methylpyrido[2,3-*d*]pyrimidin-7(8*H*)-one: ¹H NMR (400MHz, CDCl₃): δ 7.75 (s, 1H), 7.62 (d, *J*= 8.8 Hz, 2*H*), 6.96 (d, *J*= 8.8 Hz, 2*H)*, 5.17 (bs, 2*H*), 4.47 (q*, J=* 6.8 Hz, 2*H*), 3.85 (s, 3H), 2.60 (s, 3H), 1.31 (d, *J*= 7.2 Hz, 3H), MS (EI) for C₁₇H₁₈N₄O₂: 311.2 (MH⁺)
**Intermediate 20:** 2-Amino-8-ethyl-6-(2-methoxyphenyl)-4-methylpyrido[2,3-*d*]pyrimidin-7(8*H*)-one: ¹H NMR (400MHz, CDCl₃): δ 7.75 (m, 1H), 7.36 (m, 2*H*), 7.01 (m, 2*H*), 5.20 (bs, 2*H*), 4.45 (m, 2*H*), 3.82 (s, 3H), 2.56 (s, 3H), 1.31 (m, 3H), MS (EI) for C₁₇H₁₈N₄O₂: 311.2 (MH⁺)
**Intermediate 21:** 2-Amino-6-(4-chlorophenyl)-8-ethyl-4-methylpyrido[2,3-*d*]pyrimidin-7(8*H*)-one: ¹H NMR (400MHz, CDCl₃): δ 7.78 (s, 1H), 7.61 (d, *J*= 8.8 Hz, 2*H*), 7.39 (d, *J*= 8.8 Hz, 2*H*), 5.23 (bs, 2*H*), 4.46 (q, *J*= 7.2 Hz, 2*H*), 2.61 (s, 3H), 1.31 (d, *J*= 6.8 Hz, 3H), MS (EI) for C₁₆H₁₅ClN₄O: 315.1 (MH⁺)
**Intermediate 22:** 2-Amino-6-(3-chlorophenyl)-8-ethyl-4-methylpyrido[2,3-*d*]pyrimidin-7(8*H*)-one: ¹H NMR (400MHz, CDCl₃): δ 7.79 (s, 1H), 7.66 (m, 1H), 7.56 (m, 1H), 7.35 (m, 2*H*), 5.25 (bs, 2*H*), 4.46 (q, *J*= 5.6 Hz, 2*H*), 2.61 (s, 3H), 1.31 (d, *J =* 7.2 Hz, 3H), MS (EI) for C₁₆H₁₅ClN₄O: 315.1 (MH⁺)
**Intermediate 23:** 2-Amino-6-(2-chlorophenyl)-8-ethyl-4-methylpyrido[2,3-*d*]pyrimidin-7(8*H*)-one: ¹H NMR (400MHz, CDCl₃): δ 7.75 (s, 1H), 7.67 (m, 1H), 7.54 (m, 2*H*), 7.38 (m, 1H), 7.333 (m, 1H), 5.22 (bs, 2*H*), 4.46 (q, *J*= 6.8 Hz, 2*H*), 2.57 (s, 3H), 1.31 (d, *J* = 6.8 Hz, 3H), MS (EI) for C₁₆H₁₅ClN₄O: 315.1 (MH⁺)
**Intermediate 24:** 2-Amino-6-(2,4-dichlorophenyl)-8-ethyl-4-methylpyrido[2,3-*d*]Jpyrimidin-7(8*H*)-one: ¹H NMR (400MHz, CDCI₃): δ 7.77 (s, 1H), 7.67 (m, 1H), 7.49 (m, 1H), 7.32 (m, 1 H), 5.24 (bs, *2H),* 4.45 (q, *J* = 6.8 Hz, 2*H*), 2.58 (s, 3H), 1.30 (d, *J*= 7.2 Hz, 3H), MS (EI) for C₁₆H₁₄Cl₂N₄O: 349.1 (MH⁺)
**Intermediate 25:** 2-Amino-8-ethyl-4-methyl-6-(1*H-*pyrazol-5-yl)pyrido[2,3-*d*]pyrimidin-7(8*H*)-one: ¹H NMR (400 MHz, DMSO-*d*₆): δ 12.97 (bs, 1H), 8.37 (s, 1H), 7.60 (bs, 1H), 7.24 (bs, 2*H*), 6.95 (s, 1H), 4.33 (q, 2*H*), 2.55 (s, 3H), 1.81 (t, 3H); MS (EI) for C₁₃H₁₄N₆O: 271.3 (MH⁺).
**Intermediate 26:** 2-Amino-8-ethyl-4-methyl-6-(2-thienyl)pyrido[2,3-*d*]pyrimidin-7(8H)-one: ¹H NMR (400 MHz, DMSO-*d₆*):δ 8.39 (s, 1H), 7.85-7.13 (m, 5H), 4.37 (q, *J=* 7.2 Hz, 2*H*), 2.62 (s, 3H), 1.18 (t, *J*= 7.2 Hz, 3H); MS (EI) for C₁₄H₁₄N₄OS: 287.1 (MH⁺).
**Intermediate 27:** 2-Amino-8-ethyl-6-(4-fluorophenyl)-4-methylpyrido[2,3-*d*]pyrimidin-7(8*H*)-one: ¹H NMR (400 MHz, DMSO-*d₆*):δ 7.99 (s, 1H), 7.76-7.22 (m, 6H), 4.34 (q, *J* = 7.2*Hz*, 2*H*), 2.56 (s, 3H), 1.20 (t, *J* = 7.2 Hz, 3H); MS (EI) for C₁₆H₁₅FN₄O: 299.2 (MH⁺).
**Intermediate 28:** 2-Amino-8-ethyl-6-(3-fluorophenyl)-4-methylpyrido[2,3-*d*]pyrimidin-7(8*H*)-one: ¹H NMR (400 MHz, DMSO-*d*₆): δ 8.06 (s, 1H), 7.61-7.44 (m, 3H), 7.29 (bs, 2*H*), 7.20-7.15 (m, 1H), 4.34 (q, *J=* 7.2Hz, 2*H*), 2.58 (s, 3H), 1.20 (t, *J=* 7.2 Hz, 3H); MS (EI) for C₁₆H₁₅FN₄O: 299.2 (MH⁺).
**Intermediate 29:** 2-Amino-8-ethyl-6-(2-fluorophenyl)-4-methylpyrido[2,3-*d*]pyrimidin-7(8*H*)-one: ¹H NMR (400 MHz, DMSO-*d*₆): δ 7.96 (s, 1H), 7.50-7.23 (m, 6H), 4.32 (q, *J*= 6.8 Hz, 2*H*), 2.52 (s, 3H), 1.19 (t, *J* = 6.8 Hz, 3H); MS (EI) for C₁₆H₁₅FN₄O: 299.2 (MH⁺).
**Intermediate 30:** Methyl 3-(2-amino-8-ethyl-4-methyl-7-oxo-7,8-dihydropyrido[2,3-*d*]pyrimidin-6-yl)benzoate: ¹H NMR (400 MHz, DMSO-*d*₆): δ 8.34 (s, 1H), 8.06 (s, 1H), 7.95-7.55 (m, 3H), 7.28 (bs, 1H), 4.35 (q, *J* = 6.8 Hz, 2*H*), 3.89 (s, 3H), 2.58 (s, 3H), 1.21 (t, *J*= 6.8 Hz, 3H); MS (EI) for C_{18*H*18}N₄O₃: 339.2 (MH⁺).
**Intermediate 31:** 2-Amino-8-ethyl-4-methyl-6-pyrimidin-5-ylpyrido[2,3-*d*]pyrimidin-7(8*H*)-one: ¹H NMR (400 MHz, DMSO-*d*₆): δ 8.39 (s, 1H), 7.65-7.30 (m, 5H), 4.31 (q, *J*= 7.2 Hz, 2*H*), 2.50 (s, 3H), 1.17 (t, *J* = 7.2 Hz, 3H); MS (EI) for C₁₄H₁₄N₆O: 283.2 (MH⁺).

### Synthesis 32

### 2-Amino-8-ethyl-6-(1H-imidazol-5-yl)-4-methylpyrido[2,3-d]pyrimidin-7(8H)-one

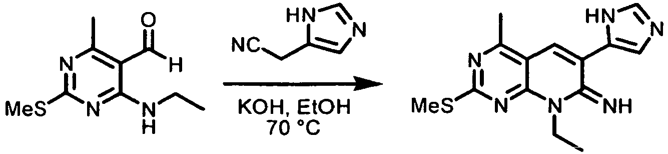

A solution of potassium hydroxide (0.139 g, 2.48 mmol) in absolute ethanol (3.0 mL) was added to a pressure tube charged with 4-(ethylamino)-6-methyl-2-(methylthio)pyrimidine-5-carbaldehyde (0.229 g, 1.08 mmol), prepared using procedures ismilar to those described for Intermediate 1, and 2-(1*H*-imidazol-5-yl)acetonitrile (0.174 g, 162 mmol) and heated to 70 °C. After 12 h, the reaction was allowed to cool to room temperature and concentrated in vacuo affording 8-ethyl-6-(1*H*-imidazol-5-yl)-4-methyl-2-(methylthio)pyrido[2,3-*d*]pyrimidin-7(8*H*)-imine as a solid. The product was used in the subsequent step without further purification.

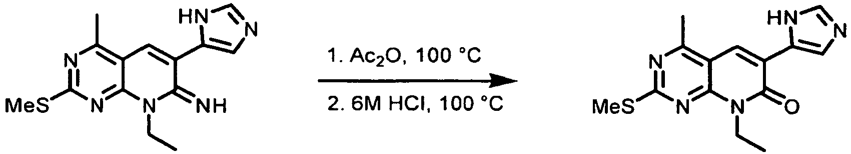

Acetic anhydride (15.0 mL) was added to a flask charged with crude 8-ethyl-6-(1*H*-imidazol-5-yl)-4-methyl-2-(methylthio)pyrido[2,3-*d*]pyrimidin-7(8*H*)-imine and heated to 100 °C. After 30 minutes, the reaction was allowed to cool to room temperature and concentrated in vacuo. The acetylated residue was then treated with 6 N HCl (16 mL) and heated to 95 °C for 30 minutes then transferred to a large flask. A saturated solution of NaHCO₃ (150 mL) was added at 0 °C to about pH = 8.0. The aqueous phase was washed thrice with ethyl acetate (100 mL) and the organic layers combined, then washed with brine and dried over Na₂SO₄. The drying agent was filtered off and the organic layers were concentrated in vacuo to afford crude 8-ethyl-6-(1*H*-imidazol-5-yl)-4-methyl-2-(methylthio)pyrido[2,3-*d*]pyrimidin-7(8*H*)-one which was used in the subsequent step without further purification.

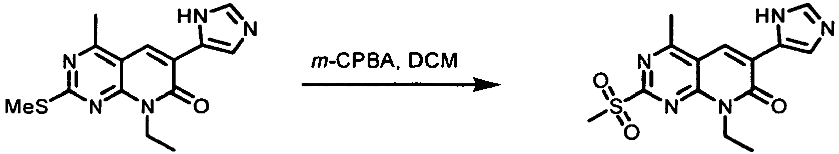

3-Chloroperbenzoic acid (0.299 g, 1.73 mmol) was added to a solution of crude 8-ethyl-6-(1*H*-imidazol-5-yl)-4-methyl-2-(methylthio)pyrido[2,3-*d*]pyrimidin-7(8*H*)-one (0.260g, 0.866 mmol) in dichloromethane (10.0 mL) at room temperature. After 1.5 h, the reaction was diluted with dichloromethane (50 mL) and washed twice with saturated NaHCO₃, followed by brine. The organic phase was separated and dried over Na₂SO₄, filtered, and concentrated in vacuo. The corresponding sulfone was used in the subsequent step without further purification.

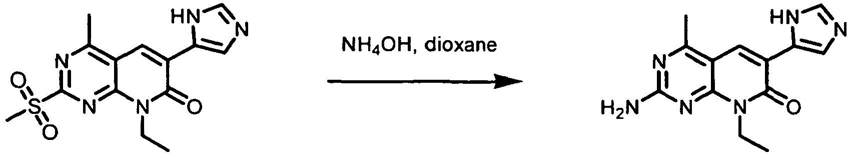

Concentrated aqueous ammonium hydroxide (400 µL) was added to a solution of the sulfone in dioxane (10 mL) at 0 °C. The reaction flask sealed, and allowed to warm to room temperature upon standing overnight. The reaction was concentrated in vacuo and purified on reverse phase HPLC (acetonitrile: water 0.1 % TFA, 20-60% gradient). The fractions containing product were collected and concentrated to one half volume and poured into saturated NaHCO₃ (50 mL). The aqueous phase was washed trice with ethyl acetate (50 mL) and dried over Na₂SO₄, filtered, and concentrated in vacuo. The residue was triturated with methylene chloride and ethyl acetate to afford 2-amino-8-ethyl-6-(1*H-*imidazol-5-yl)-4-methylpyrido[2,3-*d*]pyrimidin-7(8*H*)-one (29 mg, 12 % yield) as a light yellow solid: ¹H NMR (400 MHz, CH₃OH-*d₄*): δ 8.52 (bs, 1H), 7.88 (bs, 1H), 7.76 (s, 1H), 4.30 (q, *J* = 6.8 Hz, 2*H*), 2.65 (s, 3H), 1.29 (t, *J* = 6.8 Hz, 3H); MS (EI) for C₁₃H₁₄N₆O: 271.0 (MH⁺).

### Intermediate 33

### 2-Amino-8-ethyl-4-methyl-6-(1H-1,2,3-triazol-5-yl)pyrido[2,3-d]pyrimidin-7(8H)-one

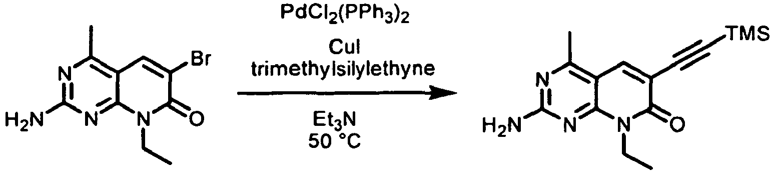

Trimethylsilylethyne (1.44 mL, 10.2 mmol) was added to a pressure tube charged with 2-amino-6-bromo-8-ethyl-4-methylpyrido[2,3-*d*]pyrimidin-7(8*H*)-one (1.58 g, 5.59 mmol) from above, CuI (0.053 g, 0.279 mmol), and PdCl₂(PPh₃)₂ (0.211 g, 0.279 mmol) in triethylamine (20 mL). The pressure tube was sealed under nitrogen and heated to 50 °C 96 h. The reaction was cooled to room temperature and poured into a saturated solution of NaHCO₃ (150 mL), then washed four times with ethyl acetate (50 mL). The organic layers were pooled and dried over Na₂SO₄, filtered and concentrated in vacuo. The residue was purified on SiO₂ (2:1, methylene chloride: ethyl acetate) to afford 2-amino-8-ethyl-4-methyl-6-((trimethylsilyl)ethynyl)pyrido[2,3-*d*]pyrimidin-7(8*H*)-one (1.09 g, 65 % yield) as an off white solid.

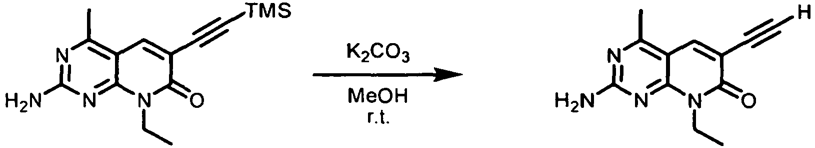

Potassium carbonate (1.00 g, 7.28 mmol) was added to a flask charged with 2-amino-8-ethyl-4-methyl-6-((trimethylsilyl)ethynyl)pyrido[2,3-*d*]pyrimidin-7(8*H*)-one (1.09 g, 3.64 mmol) in anhydrous methanol (15 mL). The reaction was stirred at room temperature under nitrogen for 16 h. The reaction was concentrated to one half volume and the yellow precipitate collected by vacuum filtration to afford 2-amino-8-ethyl -6-ethynyl-4-methylpyrido[2,3-*d*]pyrimidin-7(8H)-one.

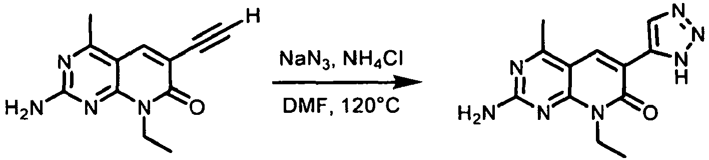

Anhydrous DMF (5.0 mL) was added to a flask charged with 2-amino-8-ethyl -6-ethynyl-4-methylpyrido[2,3-*d*]pyrimidin-7(8*H*)-one (0.204 g, 0.894 mmol), sodium azide (0.070 g, 1.07 mmol), and ammonium chloride (0.057 g, 1.07 mmol). The reaction was capped under nitrogen and heated to 120 °C. After 48 h, the reaction was cooled to room temperature and concentrated in vacuo. The residue was purified on reverse phase HPLC (acetonitrile: water 0.1 % TFA, 20-60% gradient). The fractions containing product were collected and concentrated to one half volume and poured into saturated NaHCO₃ (50 mL). The aqueous phase was washed trice with ethyl acetate (50 mL) and dried over Na₂SO₄, filtered, and concentrated in vacuo. The residue was triturated with methylene chloride and ethyl acetate to afford 2-amino-8-ethyl-4-methyl-6-(1*H*-1,2,3-triazol-5-yl)pyrido[2,3-*d*]pyrimidin-7(8*H*)-one (14 mg, 6 % yield) as a light yellow solid: ¹H NMR (400 MHz, DMSO-*d*₆): δ 8.55 (bs, 1H), 8.41 (bs, 1H), 7.32 (bs, *2H),* 4.37 (q, *J*= 7.2 Hz, *2H),* 2.60 (s, 3H), 1.21 (t, *J* = 7.2 Hz, 3H); MS (EI) for C_{12*H*13}N₇O: 272.0 (MH⁺).

### Intermediate 34

### 2-Amino-8-ethyl-4-methyl-6-(1H-tetrazol-5-yl)pyrido[2,3-d]pyrimidin-7(8H)-one

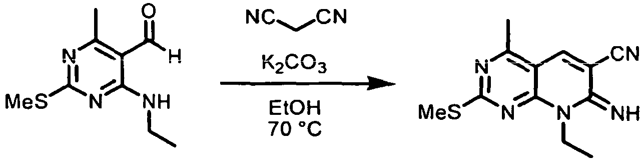

Potassium carbonate (0.539 g, 3.90 mmol) was added to a suspension of 4-(ethylamino)-6-methyl-2-(methylthio)pyrimidine-5-carbaldehyde (0.413 g, 1.95 mmol) from above, and malononitrile (0.194 g, 2.93 mmol) in absolute ethanol (15.0 mL) and heated to 70 °C. After one hour, the reaction was allowed to cool to room temperature and concentrated in vacuo. The residue was diluted with ethyl acetate (50 mL) and washed with saturated NaHCO₃ (50 mL), and brine. The organic phase was separated and concentrated in vacuo. The residue was precipitated with ethyl acetate and hexanes to give 8-ethyl-7-imino-4-methyl-2-(methylthio)-7,8-dihydropyrido[2,3-*d*]pyrimidine-6-carbonitrile as a brown solid that was used in the subsequent step without further purification.

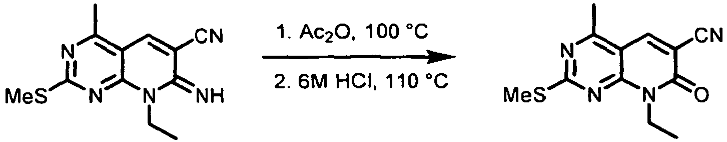

Acetic anhydride (10.0 mL) was added to a flask charged with 8-ethyl-7-imino-4-methyl-2-(methylthio)-7,8-dihydropyrido[2,3-*d*]pyrimidine-6-carbonitrile (0.506 g, 1.95 mmol) and heated to 100 °C. After one h, the reaction was allowed to cool to room temperature and concentrated in vacuo. The acetylated residue was then treated with 6 N HCl (40 mL) and heated to 95 °C for one hour then transferred to a large flask. A saturated solution of NaHCO₃ (500 mL) was added slowly at 0 °C until a ~pH 8.0 was achieved. The aqueous phase was washed thrice with ethyl acetate (100 mL) and the organic layers combined, then washed with brine and dried over Na₂SO₄. The drying agent was filtered and concentrated in vacuo to afford crude 8-ethyl-4-methyl-2-(methylthio)-7-oxo-7,8-dihydropyrido[2,3-*d*]pyrimidine-6-carbonitrile which was used in the subsequent step without further purification.

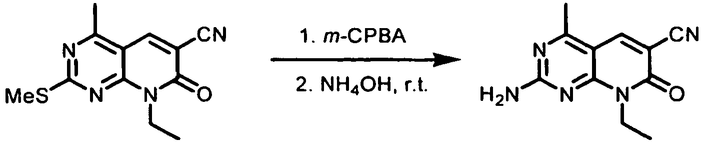

3-Chloroperbenzoic acid (1.00 g, 5.85 mmol) was added to a solution of crude 8-ethyl-4-methyl-2-(methylthio)-7-oxo-7,8-dihydropyrido[2,3-*d*]pyrimidine-6-carbonitrile (0.507 g, 1.95 mmol) in dichloromethane (30.0 mL) at room temperature. After 2.5 hours, the reaction was diluted with dichloromethane (50 mL) and washed twice with saturated NaHCO₃, followed by brine. The organic phase was separated and dried over Na₂SO₄, filtered, and concentrated in vacuo. 2-Amino-8-ethyl-4-methyl-7-oxo-7,8-dihydropyrido[2,3-*d*]pyrimidine-6-carbonitrile was used in the subsequent step without further purification.

Ammonium hydroxide (500 µL) was added to a solution of the above sulfone in dioxane (10 mL) at 0 °C. The reaction flask sealed, and allowed to warm to room temperature upon standing overnight. The reaction was concentrated in vacuo triturated with ethyl acetate to afford the product which was used in the subsequent step without further purification.

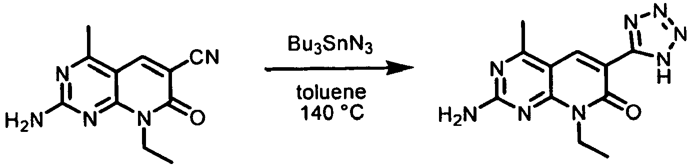

Tributyltin azide (660 µL, 2.41 mmol) was added to a flask charged with 2-amino-8-ethyl-4-methyl-7-oxo-7,8-dihydropyrido[2,3-*d*]pyrimidine-6-carbonitrile (0.184 g, 0.803 mmol) in anhydrous toluene (5.0 mL). The reaction was fitted with a reflux condenser and heated to 140 °C under a nitrogen atmosphere. After 20 h, the reaction was cooled to room temperature and the precipitate collected by vacuum filtration and washed with absolute ethanol to give 2-amino-8-ethyl-4-methyl-6-(1*H*-tetrazol-5-yl)pyrido[2,3-*d*]pyrimidin-7(8*H*)-one (98 mg, 45 % yield) as a light brown solid: ¹H NMR (400 MHz, 20 % DCI in D₂O): δ 6.97 (s, 1H), 2.42 (q, *J* = 7.2 Hz, *2H*), 0.953 (s, 3H), -0.73 (t, *J* = 7.2 Hz, 3H); MS (EI) for C₁₁H₁₁N₈O: 271.0 (MH⁺).

### Intermediate 35

### 8-(3-methoxypropyl)-4-methyl-2-(methylsulfonyl)pyrido[2,3-d]pyrimidin-7(8H)-one

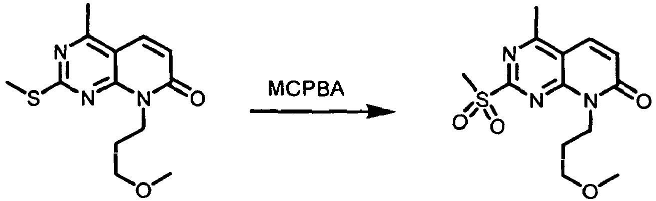

A mixture of 8-(3-methoxypropyl)-4-methyl-2-(methylthio)pyrido[2,3-d]pyrimidin-7(8*H*)-one (0.36 g, 1.29 mmol), prepared using procedures similar to those described in Example 1, dichloromethane (10 mL), and 77 % 3-chloroperbenzoic acid with water (0.723 g, 3.23 mmol) was stirred for 1 h. The mixture was diluted with dichloromethane, washed with sat. sodium bicarbonate (3 times), brine, dried over sodium sulfate, and DCM was removed under reduced pressure to give 8-(3-methoxypropyl)-4-methyl-2-(methylsulfonyl)pyrido[2,3-d]pyrimidin-7(8*H*)-one.

### Intermediate 36

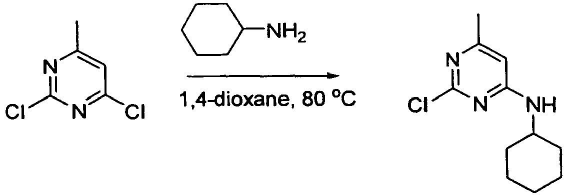

A mixture of 2,4-dichloro-6-methylpyrimidine (Aldrich, 5 g, 30 mmol), cyclohexylamine (3 g, 30 mmol) and DIEA (10 mL) was stirred at 80 °C for 12 h. The volatile material was removed under reduced pressure. The residue was loaded on a silica gel column, and was eluted with hexanes/ethyl acetate (3:1). 8-cyclohexyl-2-(ethylamino)-4-methyl-6-(thiophen-2-yl)pyrido[2,3-d]pyrimidin-7(8*H*)-one was obtained as colorless oil (2.8 g, 41 % yield).

### Example 1

### 8-Ethyl-2-{[4-(1H-imidazol-1-yl)phenyl]amino}-4-methyl-6-(1H-pyrazol-5-yl)pyrido[2,3-d]pyrimidin-7(8H)-one

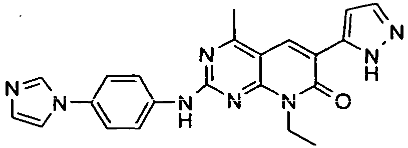

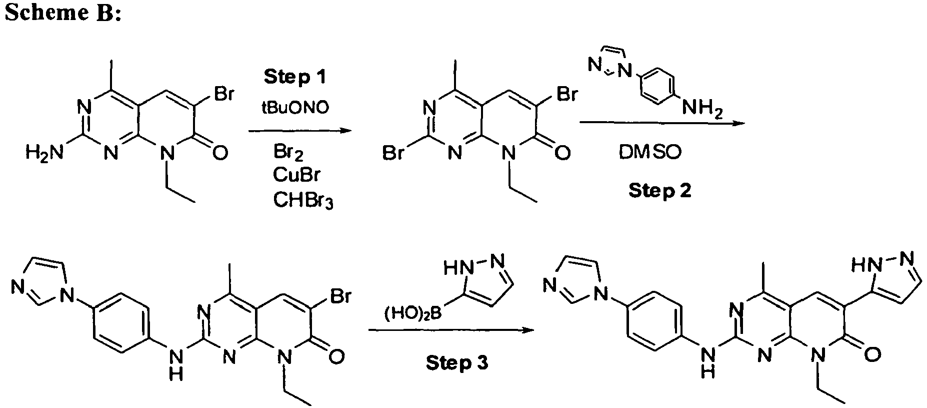

2,6-Dibromo-8-ethyl-4-methylpyrido[2,3-d]pyrimidin-7(8*H*)-one: 2-Amino-6-bromo-8-ethyl-4-methylpyrido[2,3-d]pyrimidin-7(8*H*)-one (4.51 g, 15.9 mmol) prepared using procedures similar to those described in Scheme 3, dry THF (100 mL), Cu(I)Br (2.89 g, 20.1 mmol), Br₂ (1.0 mL, 19.5 mmol), bromoform (18 mL), and t-butyl nitrite (8.0 mL, 67.3 mmol) were combined and stirred at 50 °C for 2 h. The reaction mixture was concentrated in vacuo. The resulting residue was suspended in CH₂Cl₂ and stirred at room temperature overnight. The solids were filtered, washed with CH₂Cl₂ and discarded. The filtrate was concentrated in vacuo and the resulting residue was purified by flash chromatography (4:1 hexanes:EtOAc) to give 2,6-dibromo-8-ethyl-4-methylpyrido[2,3-d]pyrimidin-7(8*H*)-one (2.83 g, 51% yield). ¹H-NMR (400MHz, d6-DMSO): 8.75 (s, 1H), 4.32 (q, 2H), 2.71 (s, 3H), 1.21 (t, 3H).

2-(4-(1*H*-Imidazol-1-yl)phenylamino)-6-bromo-8-ethyl-4-methylpyrido[2,3-d]pyrimidin-7(8*H*)-one: A DMSO solution (1.1 mL) of 200 mg (0.58 mmol) of 2,6-dibromo-8-ethyl-4-methylpyrido[2,3-d]pyrimidin-7(8*H*)-one and 110 mg (1.2 mmol) of 4-(1*H-*imidazol-1-yl)aniline is heated to 120 °C for 18 h. The product was isolated by preparative RP-HPLC (H₂O/ACN 0.1% TFA) gradient. The appropriate fractions were pooled and lyophilize to give a white solid: 77 mg (25%). LC-MS: 424.1, 426.1 (M+H) for C19H17BrN6O; ¹H NMR (400 MHz, CD₃OD) δ 9.38 (s, 1H), 8.37 (s, 1H), 8.03 (d, 2H), 7.98 (br s, 1H), 7.70 (br s, 1H), 7.63 (d, 2H), 4.52 (q, 2), 2.66 (s, 3H), 1.36 (t, 3H).

8-Ethyl-2-{[4-(1*H*-imidazol-1-yl)phenyl]amino}-4-methyl-6-(1*H*-pyrazol-5-yl)pyrido[2,3-d]pyrimidin-7(8*H*)-one: Prepared via the similar Suzuki coupling procedure using 3-pyrazoleboronic acid and 2-(4-(1*H-*imidazol-1-yl)phenylamino)-6-bromo-8-ethyl-4-methylpyrido[2,3-d]pyrimidin-7(8*H*)-one to give a white solid in 16% yield after preparative RP-HPLC using a (H₂O/ACN/ 0.1% TFA) gradient: ¹H NMR (400 MHz, CD3CN) δ 9.40 (s ,1H), 8.55 (s, 1H), 8.09 (d, 2H), 8.06 (br s, 1H), 7.77 (br s, 1H), 7.71 (d, 3H), 4.61 (br m, 2H), 2.76 (s, 3H), 1.40 (t, 3H); MS (EI) Calculated for C₂₂ H₂₀ N₈ O: 412.45; Found: 413.28 (MH⁺).

The compounds in Examples 15, 16, 17, and 18 can be prepared using procedures analogous to those in Example 1 (Steps 1 and 2).

**Example 15**: 6-bromo-8-cyclopentyl-4-methyl-2-[(4-piperazin-1-ylphenyl)amino]pyrido[2,3-*d*]pyrimidin-7(8H)-one.

**Example 16**: 1,1-dimethylethyl 4-{4-[(6-bromo-8-cyclopentyl-4-methyl-7-oxo-7,8-dihydropyrido[2,3-*d*]pyrimidin-2-yl)amino]phenyl}piperazine-1-carboxylate

**Example 17**: 1,1-dimethylethyl 4-{4-[(6-bromo-8-cyclopentyl-4-methyl-7-oxo-7,8-dihydropyrido[2,3-*d*]pyrimidin-2-yl)amino]phenyl}piperidine-1-carboxylate

**Example 18**: 6-bromo-8-cyclopentyl-4-methyl-2-[(4-piperidin-4-ylphenyl)amino]pyrido[2,3-*d*]pyrimidin-7(8*H*)-one

### Example 2

### N-{4-[(8-Ethyl-4-methyl-7-oxo-7,8-dihydropyrido[2,3-d]pyrimidin-2-yl)amino]phenyl}-2,2,2-trifluoroacetamide

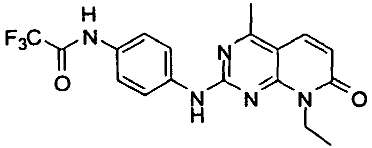

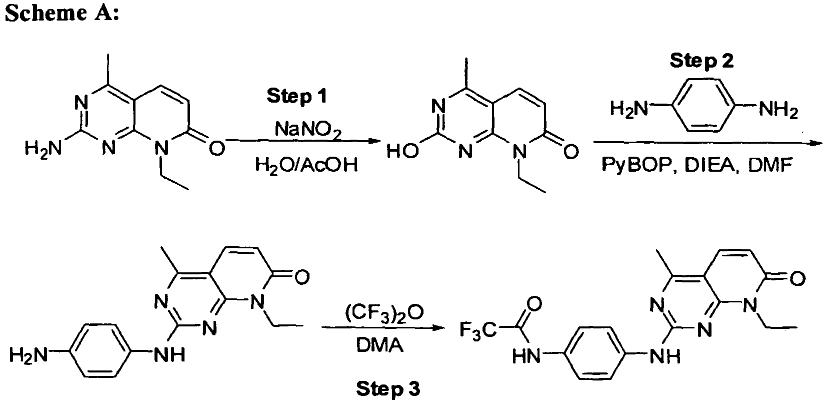

8-Ethyl-2-hydroxy-4-methylpyrido[2,3-d]pyrimidin-7(8*H*)-one: To 5.0 g (24.5 mmol) of 2-amino-8-ethyl-4-methylpyrido[2,3-d]pyrimidin-7(8*H*)-one, prepared using procedures similar to those described in Scheme 3a and 6.75 g (83.3 mmol) of sodium nitrite in a round bottom flask and slowly added a solution of 75 mL AcOH and 15 mL water. The reaction was heated at 50 °C for 18 h. LC/MS indicated mainly product. The reaction was cooled and the solid material collected by filtration and rinsed with water. This afforded 3.3 g (66% yield) of product. ¹H NMR (400 MHz, DMSO) δ 12.3 (br s, 1H), 7.82 (d, 1H), 6.22 (d, 1H), 4.16 (q, 2H), 1.12 (t, 3H); MS C₁₀H₁₁N₃O₂: 206.0 (MH⁺).

2-(4-Aminophenylamino)-8-ethyl-4-methylpyrido[2,3-d]pyrimidin-7(8*H*)-one: To 760 mg (3.70 mmol) of the 8-ethyl-2-hydroxy-4-methylpyrido[2,3-d]pyrimidin-7(8*H*)-one was added 3.47g (6.67 mmol) of PyBOP, 3.7 mL DMF, 400 mg (3.70 mmol) of p-phenylenediamine, and 1.9 mL (11.11 mmol) of DIEA. The reaction was stirred at room temperature 18 h, and afforded product. ¹H NMR (400 MHz, CD₃CN) δ 7.84 (d, 1H), 7.44 (d, 2H), 6.70 (d, 2H), 6.30 (d, 1H), 4.3 (q, 2H), 3.18 (q, 2H), 2.5 (s, 3H), 1.3 (t, 3H); MS C₁₆H₁₇N₅Ox C₂H₄O₂: 296.3 (MH⁺).

*N-*{4-[(8-Ethyl-4-methyl-7-oxo-7,8-dihydropyrido[2,3-d]pyrimidin-2-yl)amino]phenyl}-2,2,2-trifluoroacetamide: A mixture of 490 µL (0.49 mmol) of 2-(4-aminophenylamino)-8-ethyl-4-methylpyrido[2,3-d]pyrimidin-7(8*H*)-one, 490 µL of DMA, and 139 µL (0.98 mmol) trifluoroacetic anhydride was heated at 80 °C. Purification by preparatory HPLC, followed by lyophillization afforded 35 mg (18% yield) of pure product. ¹H NMR (400 MHz, CD3CN) δ 9.24 (br s, 1H), 8.52 (br s, 1H), 7.88 (d, 1H), 7.82 (d, 2*H*), 7.64 (d, 2H), 6.40 (d, 1H), 4.4 (q, 2H), 2.6 (s, 3H), 1.3 (t, 3H); Calculated for C₁₈ H₁₆ F₃ N₅ O₂: 391.35; Found: 392.22 (MH⁺).

Using the same or analogous synthetic techniques and substituting with appropriate reagents, Examples 3, 4, and 5 were prepared.

**Example 3:** *N-*{4-[(8-ethyl-4-methyl-7-oxo-7,8-dihydropyrido[2,3-d]pyrimidin-2-yl)amino]phenyl}acetamide. ¹H NMR (400 MHz, CD₃CN) δ 8.30 (br s, 1H), 8.06 (br s, 1H), 7.86 (d, 1H), 7.70 (d, 2H), 7.56 (d, 2H), 6.38 (d, 1H), 4.4 (q, 2H), 2.6 (s, 3H), 2.1 (s, 3H), 1.3 (t, 3H); MS (EI) Calculated for C₁₈H₁₉N₅O₂: 337.38; Found: 338.25 (MH⁺).

**Example 4:** *N*-{4-[(8-Ethyl-4-methyl-7-oxo-7,8-dihydropyrido[2,3-d]pyrimidin-2-yl)amino]phenyl}-*N*²,*N*²-dimethylglycinamide. ¹H NMR (400 MHz, CD₃CN) δ 9.44 (br s, 1H), 7.88 (d, 1H), 7.74 (d, 2H), 7.62 (d, 2H), 6.44 (d, 1H), 4.3 (q, 2H), 4.0 (s, 2H), 2.9 (s, 6H), 2.6 (s, 3H), 1.3 (t, 3H); Calculated for C₂₀ H₂₄ N₆ O₂: 380.45; Found: 381.1 (MH⁺).

**Example 5:** *N-*{4-[(8-Ethyl-4-methyl-7-oxo-7,8-dihydropyrido[2,3-d]pyrimidin-2-yl)amino]phenyl}-2-methylalaninamide. ¹H NMR (400 MHz, CD3CN) δ 9.4 (br s, 1H), 8.1 (s, 1H), 7.9 (d, 1H), 7.7 (d, 2H), 7.6 (d, 2H), 6.4 (d, 1H), 4.4 (q, 2H), 2.6 (s, 3H), 1.3 (s, 6H); Calculated for C₂₀ H₂₄ N₆ O₂: 380.45; Found: 381.1 (MH⁺).

### Example 6

### 2- {[4-(4-Acetylpiperazin-1-yl)phenyl]amino}-8-ethyl-4-methylpyrido[2,3-d]pyrimidin-7(8H)-one

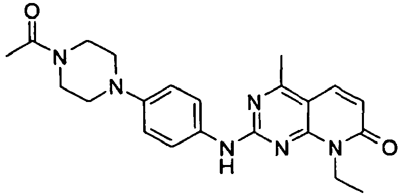

2-{[4-(piperazin-1-yl)phenyl]amino}-8-ethyl-4-methylpyrido[2,3-d]pyrimidin-7(8*H*)-one (which was prepared using procedures analogous to those described in Example 11) was acylated using procedures described in Example 2. ¹H NMR (400 MHz, CD3CN) δ 10.92 (br s, 1H), 7.84 (d, 1H), 7.67 (d, 2H), 7.38 (s, 1H), 7.12 (d, 2H), 6.47 (d, 1H), 4.33 (q, 2H), 3.71(dt, 4H), 3.22 (dt, 4H0, 2.69 (s, 3)H), 2.10 (s, 3H), 1.28 (t, 3H); MS (EI) Calculated for C₂₂ H₂₆ N₆ O₂: 406.48; Found: 407.1 (MH⁺).

### Example 7

### 8-cyclopentyl-4-methyl-2-[(4-piperazin-1-ylphenyl)amino]-6-(1,3-thiazol-2-yl)pyrido[2,3-d]pyrimidin-7(8H)-one 2HCl

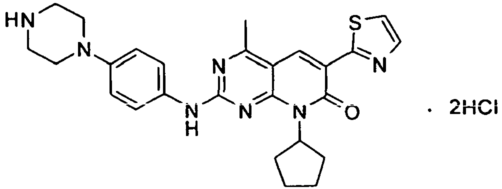

Using the same or analogous synthetic techniques as described in Scheme 3, and in particular the tin coupling 1,1-dimethylethyl 4-(4-{[8-cyclopentyl-4-methyl-7-oxo-6-(1,3-thiazol-2-yl)-7,8-dihydropyrido[2,3-*d*]pyrimidin-2-yl]amino}phenyl)piperazine-1-carboxylate was prepared. The Boc-protecting group was subsequently removed using procedures known to one of skill in the art to yield the title compound. MS (EI) Calculated for C₂₆H₂₉N₇OS: 487.22; Found: 488.1 (MH⁺).

### Example 8

### 8-cyclopentyl-2-{[4-(4-ethylpiperazin-1-yl)phenyl]amino}-4-methyl-6-(1,3-thiazol-2-yl)pyrido[2,3-d]pyrimidin-7(8H)-one 2

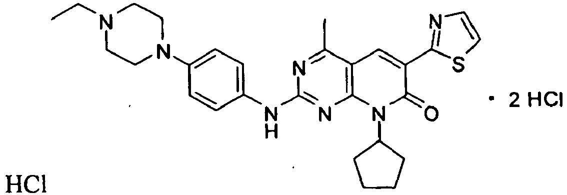

The title compound was prepared from 6-bromo-8-cyclopentyl-2-{[4-(4-ethylpiperazin-1-yl)phenyl]amino}-4-methylpyrido[2,3-*d*]pyrimidin-7(8*H*)-one (prepared using the the same or analogous synthetic techniques as described in Scheme 3) using the Stille coupling procedure described in Scheme 3. ¹H NMR (400MHz, CDCl₃) δ 8.93 (s, 1H), 7.95 (d, *J* = 2.8, 1 H), 7.54 (d, *J* = 8.0, 2H), 7.45 (d, J = 2.8, 1H), 6.96 (d, *J* = 8.4, 2H), 6.02 (q, *J* = 9.2, 1H), 3.76-3.50 (m, 4H), 3.19-2.90 (m, 4H), 2.76 (s, 3H), 2.50-2.37 (m, 2H), 2.08-1.95 (m, 2H), 1.95-1.82 (m, 2H), 1.79-1.46 (m, 4H), 1.53 (t, *J* = 7.2, 3H), MS (EI) Calculated for C₂₈H₃₃N₇OS: 515.25 Found: 516.3 (MH⁺).

### Example 9

### 8-cyclopentyl-4-methyl-2-[(4-piperidin-4-ylphenyl)amino]-6-(1,3-thiazol-2-yl)pyrido[2,3-d]pyrimidin-7(8H)-one 2HCl

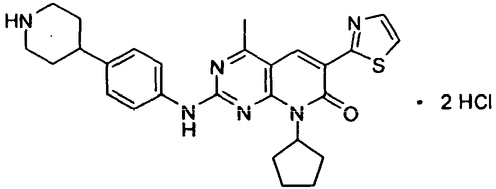

Using the same or analogous synthetic techniques as described in Scheme 3, and in particular the tin coupling, 1,1-dimethylethyl 4-(4-{[8-cyclopentyl-4-methyl-7-oxo-6-(1,3-thiazol-2-yl)-7,8-dihydropyrido[2,3-*d*]pyrimidin-2-yl]amino}phenyl)piperidine-1-carboxylate was prepared. The Boc-protecting group was subsequently removed using procedures known to one of skill in the art to yield the title compound. MS (EI) Calculated for C₂₇ H₃₀ N₆ O S: 486.22; Found: 487.2 (MH⁺).

### Example 10

### 8-cyclopentyl-4-methyl-2-[(4-piperazin-1-ylphenyl)amino]-6-(1,3-thiazol-5-yl)pyrido[2,3-d]pyrimidin-7(8H)-one

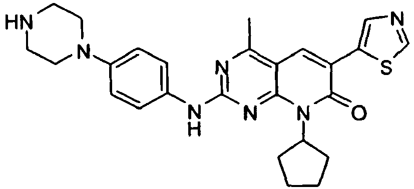

Using the same or analogous synthetic techniques as described in Scheme 3, and in particular the boronic acid coupling, 1,1-dimethylethyl 4-(4-{[8-cyclopentyl-4-methyl-7-oxo-6-(1,3-thiazol-5-yl)-7,8-dihydropyrido[2,3-*d*]pyrimidin-2-yl]amino} phenyl)piperazine-1-carboxylate was prepared. The Boc-protecting group was subsequently removed using procedures known to one of skill in the art to yield the title compound. MS (EI) Calculated for C₂₆H₂₉N₇OS: 487.22; Found: 488.1 (MH⁺).

### Example 11

### 4-methyl-2-[(4-piperazin-1-ylphenyl)amino]-8-(tetrahydrofuran-3-yl)pyrido[2,3-d]pyrimidin-7(8H)-one

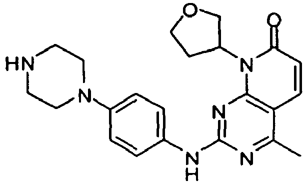

### Step 1

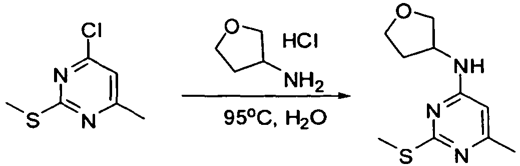

To a round-bottomed flask were added 4-chloro-6-methyl-2-(methylthio)pyrimidine (11.62 g, 66.5 mmol), tetrahydro-furan-3-ylamine hydrochloride (Small Molecules, 8.22 g, 66.51 mmol), triethylamine (20.15 g, 199.5 mmol) and water (100 mL). The reaction mixture was heated to 95 °C for 5 days. After completion, the reaction mixture was partitioned with ethyl acetate and saturated sodium chloride solution. The organic layer was separated and aqueous layer was extracted with ethyl acetate (50 mL x 2). After evaporation of solvent, the residue was submitted to column chromatography to give 6-methyl-2-(methylthio)-*N*-(tetrahydrofuran-3-yl)pyrimidin-4-amine (14.7g, 98% yield) with 20~40% ethyl acetate/hexane as eluent. ¹H NMR (400 MHz, CDCl₃): δ 5.87 (s, 1H), 4.93 (bs, 1H), 4.47(bs, 1H), 4.00~3.92 (m, 2H), 3.90~3.81 (m, 1H), 3.73~3.68 (m, 1H), 2.49(s, 3H), 2.36~2.30 (m, 1H), 2.28 (s, 3H), 1.91~1.82 (m, 1H).

### Step 2

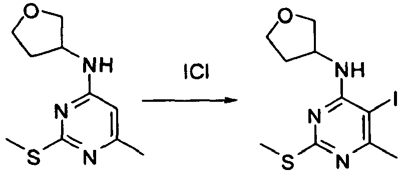

To the solution of 6-methyl-2-(methylthio)-*N*-(tetrahydrofuran-3-yl)pyrimidin-4-amine (14.7g, 65 mmol) in methanol was added iodine monochloride (11.12 g, 68.5 mmol) in a small portion at 0 °C. The reaction mixture was stirred overnight. After evaporation of solvent, the residue was triturated with acetone. 5-Iodo-6-methyl-2-(methylthio)-*N-*(tetrahydrofuran-3-yl)pyrimidin-4-amine (11g, 48% yield) was collected by filtration. ¹H NMR (400 MHz, CDCl₃): δ 5.4 (bs, 1H), 4.67 (bs, 1H), 4.05~3.96 (m, 2H), 3.88~3.82 (m, 1H), 3.77~3.73 (m, 1H), 2.51 (s, 3H), 2.49 (s, 3H), 2.40~2.31 (m, 1H), 1.95~1.86 (m, 1H).

### Step 3

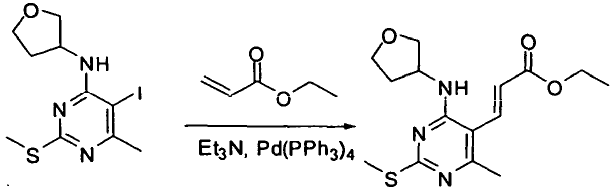

To the solution of 5-iodo-6-methyl-2-(methylthio)-N-(tetrahydrofuran-3-yl)pyrimidin-4-amine (8 g, 23.86 mmol) in DMF (50 mL) were added ethyl acrylate (3.58 g, 35.8 mmol), Pd(PPh₃)₄(0.30 g, 0.24 mmol) and triethylamine (7.23 g, 71.6 mmol). The reaction mixture was heated to 90 °C for 3 hours. After evaporation of solvent, the residue was diluted with water and the aqueous layer was extracted with ethyl acetate. (*E*)-Ethyl-3-(4-methyl-2-(methylthio)-6-(tetrahydrofuran-3-ylamino)pyrimidin-5-yl)acrylate (6.56 g, 85% yield) was isolated by silica gel column chromatography with 20% ethyl acetate in hexane as eluent. ¹H NMR (400 MHz, CDCl₃): δ 7.62 (d, J = 12.4Hz, 1H), 6.20 (d, J = 16.4Hz, 1H), 5.24 (bs, 1H), 4.74 (m, 1H), 4.30(q, J = 5.1Hz, 2H), 4.01~3.95 (m, 2H), 3.86~3.80 (m, 1H), 3.74~3.71 (m, 1H), 2.52 (s, 3H), 2.38 (s, 3H), 2.37~2.33 (m, 1H), 1.91~1.82 (m, 1H), 1.68 (t, J = 5.1Hz, 3H).

### Step 4

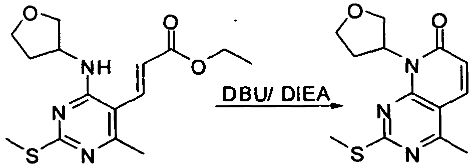

To the solution of (*E*)-ethyl-3-(4-methyl-2-(methylthio)-6-(tetrahydrofuran-3-ylamino)pyrimidin-5-yl)acrylate (6.56g, 20.28 mmol) in DIEA (26 ml) was added DBU (6.16 g, 40.56 mmol) at room temperature. The reaction mixture was heated to reflux and reacted for 15 hrs. After evaporation of solvent, the residue was purified by column chromatography with 30~50% ethyl acetate/hexane as eluent to give 4-methyl-2-(methylthio)-8-(tetrahydrofuran-3-yl)pyrido[2,3-*d*]pyrimidin-7(8*H*)-one (3.75 g, 67% yield). ¹H NMR (400 MHz, CDCI₃): δ 7.78 (d, J = 7.5Hz, 1H), 6.60 (d, J = 7.5Hz, 1H), 6.36~6.28 (m, 1H), 4.39~4.33 (m, 1H), 4.17~4.11 (m, 1H), 4.02~4.0 (m, 2H), 2.67 (s, 3H), 2.63 (s, 3H), 2.60~2.55 (m, 1H), 2.25~2.15 (m, 1H).

### Step 5

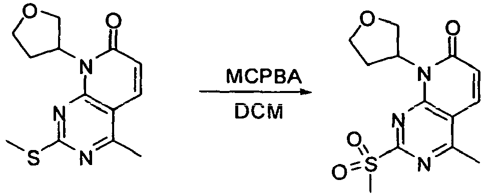

To the solution of 4-methyl-2-(methylthio)-8-(tetrahydrofuran-3-yl)pyrido[2,3-*d*]pyrimidin-7(8*H*)-one (1.9 g, 6.85 mmol) in DCM (50 mL) was added MCPBA (3.84 g, 77%, 17.1 mmol) in a small portion at room temperature. The reaction mixture was stirred for 4 hrs. After completion of the reaction, 100 mL of DCM was added and the reaction mixture was washed with saturated NaHCO₃ solution. 4-Methyl-2-(methylsulfonyl)-8-(tetrahydrofuran-3-yl)pyrido[2,3-*d*]pyrimidin-7(8*H*)-one (1.9 g, 90%) was obtained by silica gel column chromatography. ¹H NMR (400 MHz, CD₃OD): δ 7.91(d, J = 7.20 Hz, 1H), 6.88(d, J = 7.2Hz, 1H), 6.30~6.22 (m, 1H), 4.48~4.42 (m, 1H), 4.13~3.98 (m, 3H), 3.43(s, 3H), 2.87(s, 3H), 2.54~2.45 (m, 1H), 2.33~2.24 (m, 1H).

### Step 6

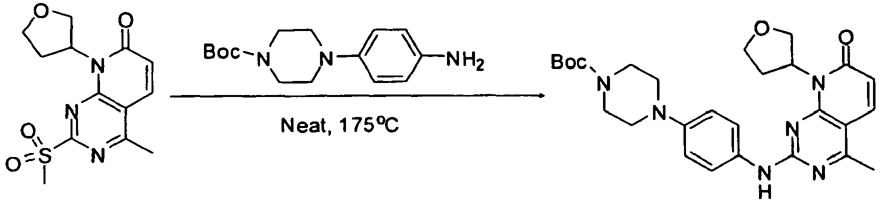

The mixed reaction mixture of 4-methyl-2-(methylsulfonyl)-8-(tetrahydrofuran-3-yl)pyrido[2,3-*d*]pyrimidin-7(8*H*)-one (0.475 g, 1.53 mmol) and *tert*-butyl 4-(4-aminophenyl)piperazine-1-carboxylate (0.426 g, 1.53 mmol) was heated at 160 °C for 8 minutes. After cooling to room temperature, the reaction mixture was dissolved in DCM and separated by column chromatography to give *tert*-butyl 4-(4-(4-methyl-7-oxo-8-(tetrahydrofuran-3-yl)-7,8-dihydropyrido[2,3-*d*]pyrimindin-2-ylamino)phenyl)piperazine-1-carboxylate (0.4 g, 51.4%) with 30%∼50% ethyl acetate/hexane as eluent. ¹H NMR (400 MHz, CDCl₃): δ 7.70(d, J = 7.20 Hz, 1H), 7.49(d, J = 6.6Hz, 2H), 7.15 (bs, 1H), 6.97 (d, J = 6.6 Hz, 2H), 6.40 (d, J = 7.20 Hz, 2H), 6.25∼6.21 (m, 1H), 4.48∼4.42 (m, 1H), 4.28∼4.20 (m, 1H), 4.15∼3.98 (m, 3H), 3.60(m, 4H), 3.13(m, 4H), 2.60(s, 3H), 2.58∼2.53 (m, 1H), 2.18∼2.12 (m, 1H), 1.49 (s, 9H).

### Step 7

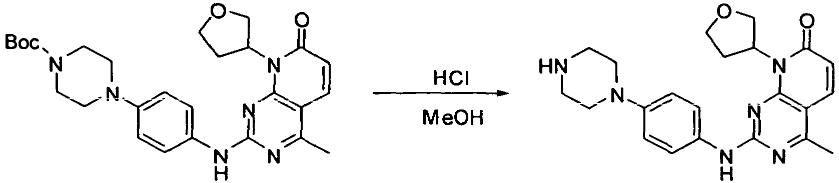

To the solution of *tert*-Butyl 4-(4-(4-methyl-7-oxo-8-(tetrahydrofuran-3-yl)-7,8-dihydropyrido[2,3-*d*]pyrimindin-2-ylamino)phenyl)piperazine-1-carboxylate (1.6 g, 3.16 mmol) in methanol (12 mL) was added a solution of 4 M HCl in dioxane at room temperature. The reaction mixture was heated to 50 °C for 4 hours. After cooling, 4-methyl-2-[(4-piperazin-1-ylphenyl)amino]-8-(tetrahydrofuran-3-yl)pyrido[2,3-*d*]pyrimidin-7(8*H*)-one (1.411 g) was collected on a filter paper. ¹H NMR (400 MHz, CD₃OD): δ 8.01(d, J = 7.50 Hz, 1H), 7.46(d, J = 9.0Hz, 2H), 7.20 (d, J = 9.0 Hz, 2H), 6.56 (d, J 7.5 Hz, 1H), 6.04∼5.96 (m, 1H), 4.00∼3.70 (m, 4H), 3.53∼3.50 (m, 4H), 3.49∼3.41 (m, 4H), 2.80(s, 3H), 2.43∼2.35 (m, 1H), 2.13∼2.03 (m, 1H). MS (EI) for C₂₂H₂₆N₆O₂: 407.3 (M+H).

The compounds in Examples 12, 13, and 14 were prepared using procedures analogous to those in Example 11.

**Example 12:** 8-ethyl-4-methyl-2-[(4-piperazin-1-ylphenyl)amino]pyrido[2,3-*d*]pyrimidin-7(8*H*)-one (95 mg, 20% yield). ¹H NMR (400 MHz, d3-ACN): 9.21 (br s, 1H), 8.88 (br s, 2H), 7.87 (d, 1H), 7.73 (d, 2H)6.99 (d, 2H), 6.33 (d, 1 H), 4.36 (q, 2H), 3.33 (m, 4H), 3.28 (br m, 4H), 2.57 (s, 3H), 1.27 (t, 3H); MS (EI) 365.26 (MH⁺).

**Example 13:** 8-ethyl-4-methyl-2-[(6-piperazin-1-ylpyridin-3-yl)amino]pyrido[2,3-*d*]pyrimidin-7(8*H*)-one (35 mg, 20% yield). ¹H NMR (400 MHz, d₃-ACN): 8.44 (s, 1H), 7.99 (dd, 1H), 7.85 (d, 1), 7.6 (s, 1H), 6.83 (d, 1H), 6.35 (d, 1H), 4.35 (q, 2H), 3.60 (m, 4H), 3.13 (m, 4H), 2.57 (s, 3H), 1.26 (t, 3H); MS (EI) 366.04 (MH+).

**Example 14:** 8-ethyl-4-methyl-2-{[6-(4-methylpiperazin-1-yl)pyridin-3-yl]amino}pyrido[2,3-*d*]pyrimidin-7(8*H*)-one (50 mg, 27% yield). ¹H NMR (400 MHz, CD3CN) δ 8.44 (d, 1H), 8.00 (dd, 1H), 7.90 (s, 1H), 7.86 (d, 1H), 6.86 (d, 1H), 6.36 (d, 1H), 4.3 (q, 2H), 3.6 (t, 4H), 2.9 (t, 4H), 2.6 (s, 3H), 2.55 (s, 3H), 1.3 (t, 3H): MS (EI) for C₂₀H₂₅N₇O: 380.1 (MH⁺).

### Example 19

### 8-ethyl-2-{[4-(1H-imidazol-1-yl)phenyl]amino}-4-methylpyrido[2,3-d]pyrimidin-7(8H)-one

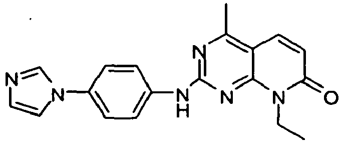

The first six steps of Scheme 1 were used to prepare 8-ethyl-2-methylthio-4-methylpyrido[2,3-*d*]pyrimidin-7(8*H*)-one which was then treated as described in Scheme 7 to yield the title Compound. (65 mg, 30% yield). ¹H NMR (400 MHz, DMSO) δ 10.4 (s, 1H), 9.5 (s, 1H), 8.2 (s, 1H), 8.0 (q, 3H), 7.9 (s, 1H), 7.8 (d, 2H), 6.4 (d, 1H), 4.4 (q, 2H), 2.6 (s, 3H), 1.3 (t, 3H): MS C₁₉H₁₈N₆O: 347.3 (MH⁺).

### Example 20

### 8-cyclopentyl-4-methyl-2-{[4-(1H-pyrazol-1-yl)phenyl]amino}pyrido[2,3-d]pyrimidin-7(8H)-one

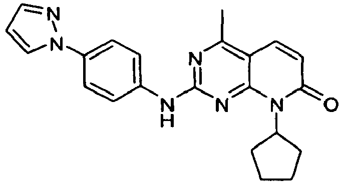

The first six steps of Scheme 1 were used to prepare 8-ethyl-2-methylthio-4-methylpyrido[2,3-*d*]pyrimidin-7(8*H*)-one which was then treated as described in Scheme 7 to yield the title Compound. (65 mg, 30% yield). (64 mg, 59% yield). ¹H NMR (400 MHz, DMSO); δ 10.1 (s, 1H), 8.45 (d, 2H), 7.96 (d, 1H), 7.78 (m, 4H), 7.71 (d, 1H), 6.51 (m, 1H), 6.32 (d, 1H), 5.92 (m, 1H), 2.62 (s, 3H), 2.26 (m, 2H), 1.94 (m, 2H), 1.79 (m, 2H), 1.63 (m, 2H); MS (EI) for C₂₂H₂₂N₆OS: 389.2 (MH⁺).

### Example 21

### 8-cyclopentyl-4-methyl-2-{[4-(1,2,3-thiadiazol-4-yl)phenyl]amino}pyrido[2,3-d]pyrimidin-7(8H)-one

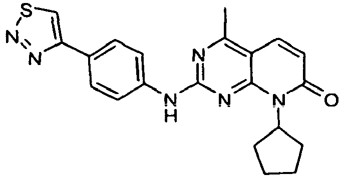

The first six steps of Scheme 1 were used to prepare 8-ethyl-2-methylthio-4-methylpyrido[2,3-*d*]pyrimidin-7(8*H*)-one which was then treated as described in Scheme 7 to yield the title Compound. (65 mg, 30% yield). (86 mg, 34% yield); ¹H NMR (400 MHz, DMSO); δ 10.2 (s, 1H), 9.54 (s, 1H), 8.11 (d, 2H), 7.8 (d, 1H), 7.93 (d, 2H), 6.34 (d, 1H), 5.95 (m, 1H), 2.64 (s, 3H), 2.25 (m, 2H), 1.98 (m, 2H), 1.82 (m, 2H), 1.64 (m, 2H); MS (EI) for C₂₁H₂₀N₆OS: 405.2 (MH⁺).

### Example 22

### 4-[(8-cyclopentyl-4-methyl-7-oxo-7,8-dihydropyrido[2,3-d]pyrimidin-2-yl)amino]benzoic acid

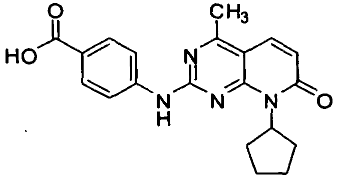

The first six steps of Scheme 1 were used to prepare 8-ethyl-2-methylthio-4-methylpyrido[2,3-*d*]pyrimidin-7(8*H*)-one which was then treated as described in Scheme 7 to yield methyl 4-(8-cyclopentyl-4-methyl-7-oxo-7,8-dihydropyrido[2,3-*d*]pyrimidin-2-ylamino)benzoate which was then treated with LiOH in a mixture of THF and water to yield the title Compound. (65 mg, 30% yield). (36.5 mg, 30% yield). ¹H NMR (400 MHz, DMSO); δ 10.3 (s, 1H), 7.99 (d, 1H), 7.88 (m, 4H), 6.36 (d, 1H), 5.92 (m, 1H), 2.64 (s, 3H), 2.27 (m, 2H), 1.97 (m, 2H), 1.80 (m, 2H), 1.64 (m, 2H); MS (EI) for C₂₀H₂₀N₄O₃: 365.1 (MH⁺).

### Example 23

### 8-cyclopentyl-4-methyl-2-{[4-(piperidin-1-ylcarbonyl)phenyl]amino}pyrido[2,3-d]pyrimidin-7(8H)-one

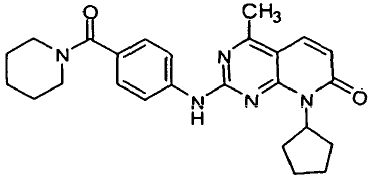

The titled Compound of Example 22 was reacted with piperidine using the conditions described in Scheme 8 to yield the title compound. ¹H NMR (400 MHz, DMSO); δ 10.3 (s, 1H), 7.97 (d, 1H), 7.78 (d, 2H), 7.35 (d, 2H), 6.33 (d, 1H), 5.89 (m, 1H), 3.45 (bs, 4H), 2.50 (s, 3H), 2.26 (m, 2H), 1.92 (m, 2H), 1.77 (m, 2H), 1.61 (m, 4H), 1.50 (m, 4H); MS (EI) for C₂₅H₂₉N₅O₂: 432.2 (MH⁺).

### Example 24

### Methyl 4-[({4-[(8-cyclopentyl-4-methyl-7-oxo-7,8-dihydropyrido[2,3-d]pyrimidin-2-yl)amino]phenyl}carbonyl)amino]-1-methyl-1H-pyrrole-2-carboxylate

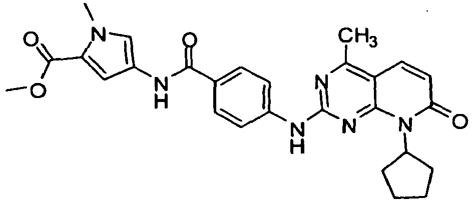

The titled Compound of Example 22 was reacted with methyl 4-amino-1-methyl-1*H-*pyrrole-2-carboxylate using the conditions described in Scheme 8 to yield the title compound. (20.3 mg, 31% yield). ¹H NMR (400 MHz, DMSO); δ 10.3 (s, 1H), 10.2 (s, 1H), 7.99(d, 1H), 7.90(m, 4H), 7.52 (d, 1H), 6.95 (d, 1H), 6.35 (d, 1H), 5.94 (m, 1H), 3.86 (s, 3H), 3.75 (s, 3H), 2.64 (s, 3H), 2.27 (m, 2H), 1.98 (m, 2H), 1.80 (m, 2H), 1.65 (m, 2H); MS (EI) for C₂₇H₂₈N₆O₄: 501.1 (MH⁺).

### Example 25

### 4-[(8-cyclopentyl-4-methyl-7-oxo-7,8-dihydropyrido[2,3-d]pyrimidin-2-yl)amino]-N-(phenylmethyl)benzamide

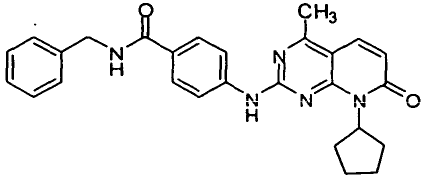

The titled Compound of Example 22 was reacted with benzylamine using the conditions described in Scheme 8 to yield the title compound. (11.4 mg, 12% yield). ¹H NMR (400 MHz, DMSO); δ 10.2 (s, 1H), 8.94 (s, 1H), 7.97 (d, 1H), 7.87 (m, 4H), 7.32 (m, 4H), 7.25 (m, 1H), 6.35 (d, 1H), 5.93 (m, 1H), 4.49 (d, 2H), 2.63 (s, 3H), 2.27 (m, 2H), 1.97 (m, 2H), 1.80 (m, 2H), 1.65 (m, 2H); MS (EI) for C₂₇H₂₇N₂O₂: 454.1 (MH⁺).

### Example 26

### 4-[(8-cyclopentyl-4-methyl-7-oxo-7,8-dihydropyrido[2,3-d]pyrimidin-2-yl)amino]-N-ethylbenzamide

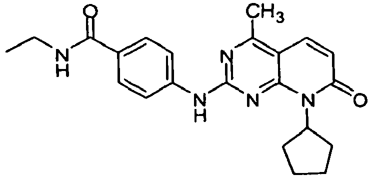

The titled Compound of Example 22 was reacted with ethylamine using the conditions described in Scheme 8 to yield the title compound. (9.5 mg, 13% yield). ¹H NMR (400 MHz, DMSO); δ 10.2 (s, 1H), 8.35 (t, 1H), 7.98 (d, 1H), 7.02 (s, 4H), 6.34 (d, 1H), 5.93 (m, 1H), 3.29 (qr, 2H), 2.60 (s, 3H), 2.25 (m, 2H), 1.98 (m, 2H), 1.79 (m, 2H), 1.65 (m, 2H), 1.12 (t, 3H); MS (EI) for C₂₂H₂₅N₅O₂: 392.1 (MH⁺).

### Example 27

### 1,1-dimethylethyl 4-({4-[(8-cyclopentyl-4-methyl-7-oxo-7,8-dihydropyrido[2,3-d]pyrimidin-2-yl)amino]phenyl}carbonyl)piperazine-1-carboxylate

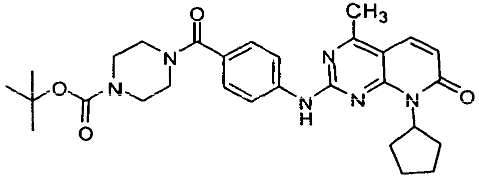

The titled Compound of Example 22 was reacted with *tert*-butyl piperazine-1-carboxylate using the conditions described in Scheme 8 to yield the title compound. (20.3 mg, 29% yield). ¹H NMR (400 MHz DMSO): δ 10.2 (s, 1H), 7.98 (d, 1H), 7.82 (d, 2H), 7.40 (d, 2H), 6.34 (d, 1H), 5.90(m, 1H), 3.48 (bs, 4H), 3.38 (bs, 4H), 2.62 (s, 3H), 2.26 (m, 2H), 1.94 (m, 2H), 1.78 (m, 2H), 1.62 (m, 2H), 1.41 (s, 9H); MS (EI) for C₂₉H₃₆N₆O₄: 533.3 (MH⁺).

### Example 28

### 8-cyclopentyl-4-methyl-2-{[4-(piperazin-1-ylcarbonyl)phenyl]amino}pyrido[2,3-d]pyrimidin-7(8H)-one

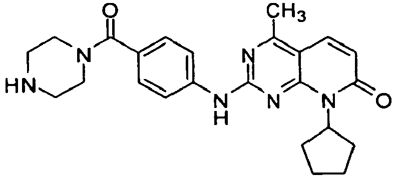

The titled Compound of Example 27 was deprotected using conditions known to one of skill in the art to yield the title compound. ¹H NMR (400 MHz, DMSO-d6); δ 10.2 (s, 1H), 9.42 (bs, 2H), 7.98 (d, 1H), 7.84 (d, 2H), 3.46 (d, 2H), 6.34 (dd, 2H), 5.91 (m, 1H), 3.70 (m, 2H), 3.48 (m, 2H), 3.15 (bs, 4H), 2.62 (s, 3H), 2.67 (m, 2H), 1.95 (m, 2H), 1.78 (m, 2H), 1.62 (m, 2H). MS (EI) for C₂₄H₂₈N₆O₂: 433.1 [MH]⁺. Analytical HPLC purity > 98%.

### Example 29

2-[(4-aminophenyl)amino]-8-ethyl-4-methylpyrido[2,3-d]pyrimidin-7(8*H*-one (70 mg, 40% yield). 1H NMR (400 MHz, CD3CN) δ 7.96 (d, 1H), 7.76 (d, 2H), 7.52 (d, 2H), 6.72 (d, 1H), 4.3 (q, 2H), 2.8 (s, 3H), 1.2 (t, 3H): MS C₁₆H₁₇N₅O: 296.3 (MH⁺).

### Example 30

2-{[4-(dimethylamino)phenyl]amino}-8-ethyl-4-methylpyrido[2,3-*d*]pyrimidin-7(8*H*)-one (44 mg, 28% yield); 1H NMR (400 MHz, DMSO) δ 9.8 (s, 1H), 7.94 (d, 1H), 7.62 (d, 2H), 6.74 (d, 2H), 6.30 (s, 1H), 4.3 (q, 2H), 2.9 (s, 6H), 2.55 (s, 3H), 1.2 (t, 3H): MS C₁₈H₂₁N₅O: 324.1 (MH⁺).

### Biological Examples

### Biological Example 1

### PI3Kalpha Luciferase-Coupled Chemiluminescence Assay Protocol

PI3Kα activity was measured as the percent of ATP consumed following the kinase reaction using luciferase-luciferin-coupled chemiluminescence. Reactions were conducted in 384-well white, medium binding microtiter plates (Greiner). Kinase reactions were initiated by combining test compounds, ATP, substrate (PIP2), and kinase in a 20 µL volume in a buffer solution. The standard PI3Kalpha assay buffer was composed 50 mM Tris, pH 7.5, 1 mM EGTA, 10 mM MgCl₂, 1 mM DTT and 0.03% CHAPS. The standard assay concentrations for enzyme, ATP, and substrate were 0.5-1.1 nM, 1µM, and 7.5 µM, respectively. The reaction mixture was incubated at ambient temperature for approximately 2 h. Following the kinase reaction, a 10 µL aliquot of luciferase-luciferin mix (Promega Kinase-Glo) was added and the chemiluminescence signal measured using a Victor2 plate reader (Perkin Elmer). Total ATP consumption was limited to 40-60% and IC50 values of control compounds correlate well with literature references.

Compounds in Table 1, 2, and 3 were tested in this assay and demonstrated the ability to bind to PI3K. For example, in one embodiment, the PI3K inhibitor is selected from the compounds in Table 1, 2, and 3 having a PI3K-binding affinity of about 0.5 µM or less. In another embodiment, the PI3K inhibitor is selected from the compounds in Table 1, 2, and 3 having a PI3K-binding affinity of about 0.3 µM or less. In another embodiment, the PI3K inhibitor is selected from the compounds in Table 1, 2, and 3 having a PI3K-binding affinity of about 0.2 µM or less. In another embodiment, the PI3K inhibitor is selected from the compounds in Table 1, 2, and 3 having a PI3K-binding affinity of about 0.1 µM or less. In another embodiment, the PI3K inhibitor is selected from the compounds in Table 2 having a PI3K-binding affinity of about 0.04 µM or less.

### Biological Example 2

Phospho AKT assayPC-3 cells were seeded in 96-well plates at 24,000 cells/well. Cells were cultured for 2 days, then treated with compounds in serum-free medium for 3 hr. EGF (100 ng/mL) was added for the last 10 min. Cells were lysed in RIPA buffer. Phospho T308 Akt (Cell Signaling Technology, 7144) and total Aktl (Cell Signaling Technology, 7142) were quantified by ELISA performed according to the manufacturer's protocol. The readings of phospho Akt were normalized to total Akt readings.(RIPA: 50mM Tris, pH 7.5, 150 mM NaCl, 1% Triton X-100, 0.5% sodium deoxycholate, 0.1% SDS, 1mM EDTA, 1mM Na3VO4, 1mM NaF, 1 mM β glycerol phosphate, Complete protease inhibitors (Roche, 11 873 580 001), and phosphatase inhibitor cocktail 1 (Sigma, P2850).

### Biological Example 3

### Phospho S6 assay

PC3 cells are seeded on 96-well plates at 8,000 cells/well. For each experiment, cells are seeded and treated in duplicated plates: one plate for phospho S6 CellELISA, and one plate for total S6 CellELISA. Cells are cultured on the plates for 3 days, then treated with compounds in serum-free medium for 3 hr in triplicate. Cells are fixed with 4% formaldehyde, quenched with 0.6% H₂O₂, blocked with 5% BSA, incubated with either phospho S6 antibody or total S6 antibody overnight, incubated with goat-anti-rabbit-IgG-HRP for 1 hr, and developed in chemiluminescent substrate.

### Biological Example 4

### PIP₃ assay

MCF-7 cells grown in 10-cm dishes are starved for 3 hours in DMEM, and then treated with compounds for 20 minutes. In the last 2 minutes of the incubation with the compounds, EGF (100 ng/mL) is added to stimulate the production of PIP3. The medium was aspirated and the cells are scraped with 10% trichloroacetic acid. The lipids are extracted from the pellet after the cell lysates are centrifuged. PIP3 in the cellular lipid extraction is quantified with the AlphaScreen assay in which Grp1-PH is used as the PIP3 specific probe. The amount of cellular PIP3 is calculated from the standard curve of diC₈ PI (3,4,5) P3.

### Biological Example 5-10 In vivo models

Female and male athymic nude mice (NCr) 5-8 weeks of age and weighing approximately 20 g are used in the following model. Prior to initiation of a study, the animals are allowed to acclimate for a minimum of 48 h. During these studies, animals are provided food and water ad libitum and housed in a room conditioned at 70-75°F and 60% relative humidity. A 12 h light and 12 h dark cycle is maintained with automatic timers. All animals are examined daily for compound-induced or tumor-related deaths.

PC-3 human prostate adenocarcinoma cells are cultured in vitro in DMEM (Mediatech) supplemented with 20% Fetal Bovine Serum (Hyclone), Penicillin-Streptomycin and non-essential amino acids at 37°C in a humidified 5% CO₂ atmosphere. On day 0, cells are harvested by trypsinization and 3x10⁶ cells (passage 13, 99% viability) in 0.1 mL of ice-cold Hank's balanced salt solution are implanted subcutaneously into the hindflank of 5-8 week old male nude mice. A transponder is implanted in each mouse for identification, and animals are monitored daily for clinical symptoms and survival. Body weights are recorded daily.

U-87 MG human glioblastoma cells are cultured in vitro in DMEM (Mediatech) supplemented with 10% Fetal Bovine Serum (Hyclone), Penicillin-Streptomycin and non-essential amino acids at 37°C in a humidified 5% CO₂ atmosphere. On day 0, cells are harvested by trypsinization and 2x10⁶ cells (passage 5, 96% viability) in 0.1 mL of ice-cold Hank's balanced salt solution were implanted intradermally into the hindflank of 5-8 week old female nude mice. A transponder is implanted in each mouse for identification, and animals are monitored daily for clinical symptoms and survival. Body weights are recorded daily.

A549 human lung carcinoma cells are cultured in vitro in DMEM (Mediatech) supplemented with 10% Fetal Bovine Serum (Hyclone), Penicillin-Streptomycin and non-essential amino acids at 37°C in a humidified 5% CO₂ atmosphere. On day 0, cells are harvested by trypsinization and 10x10⁶ cells (passage 12, 99% viability) in 0.1 mL of ice-cold Hank's balanced salt solution are implanted intradermally into the hindflank of 5-8 week old female nude mice. A transponder is implanted in each mouse for identification, and animals are monitored daily for clinical symptoms and survival. Body weights are recorded daily.

MDA-MB-468 human breast adenocarcinoma cells, passage number <6, are maintained and propagated in log-phase growth in Dulbecco's Modification of Eagles's Medium (DMEM; Mediatech) containing L-Glutamine supplemented with 10% Fetal Bovine Serum (Hyclone), Penicillin-Streptomycin and non-essential amino acids at 37 °C in a humidified, 5% CO₂ atmosphere. On day 0, cells are harvested by trypsinization, and 10 x 10⁶ cells (passage 10, 98% viability) in 50% cold Hanks balanced salt solution/50% Matrigel (100 µL total volume per mouse) are implanted subcutaneously into the mammary fat pads of female nude mice.

Calu-6 human lung anaplastic carcinoma cells are cultured in vitro in DMEM (Mediatech) supplemented with 10% Fetal Bovine Serum (Hyclone), Penicillin-Streptomycin and non-essential amino acids at 37 °C in a humidified, 5% CO₂ atmosphere. On day 0, cells are harvested by trypsinization, and 5x10⁶ cells (passage #8, 96% viability) in 0.1 mL ice-cold Hank's balanced salt solution are implanted intradermally in the hind-flank of 5-8 week old female athymic nude mice. A transponder is implanted in each mouse for identification, and animals are monitored daily for clinical symptoms and survival. Body weights are recorded daily.

For subcutaneous or intradermal tumors, the mean tumor weight of each animal in the respective control and treatment groups is determined twice weekly during the study. Tumor weight (TW) is determined by measuring perpendicular diameters with a caliper, using the following formula: $tumor weight \left(mg\right) = \left[tumor volume=length \left(mm\right)⁢x {width}^{2}\left({mm}^{2}\right)\right] / 2$

These data were recorded and plotted on a tumor weight vs. days post-implantation line graph and presented graphically as an indication of tumor growth rates. Percent inhibition of tumor growth (TGI) is determined with the following formula: $\left[1-\left(\frac{\left({X}_{f}-{X}_{0}\right)}{\left({Y}_{f}-{X}_{0}\right)}\right)\right] * 100$
where X₀ = average TW of all tumors on group day
X_{f} = TW of treated group on Day f
Y_{f} = TW of vehicle control group on Day f
If tumors regress below their starting sizes, then the percent tumor regression is determined with the following formula: $\left(\frac{{X}_{0} - {X}_{f}}{{X}_{0}}\right) * 100$
Tumor size is calculated individually for each tumor to obtain a mean ± SEM value for each experimental group. Statistical significance is determined using the 2-tailed Student's t-test (significance defined as P<0.05).

The foregoing invention has been described in some detail by way of illustration and example, for purposes of clarity and understanding. The invention has been described with reference to various embodiments and techniques. However, it should be understood that many variations and modifications may be made while remaining within the scope of the invention. It will be obvious to one of skill in the art that changes and modifications may be practiced within the scope of the appended claims. Therefore, it is to be understood that the above description is intended to be illustrative and not restrictive. The scope of the invention should, therefore, be determined not with reference to the above description, but should instead be determined with reference to the following appended claims, along with the full scope of equivalents to which such claims are entitled.

## Claims

1. A Compound of Formula (III): 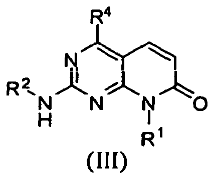 or a pharmaceutically acceptable salt thereof and additionally optionally as a solvate and additionally optionally as a hydrate thereof, wherein
R¹ is alkyl, cycloalkyl, or heterocycloalkyl;
R⁴ is alkyl;
R² is aryl which is substituted with R¹⁸ and additionally optionally substituted with one or two R⁸;
each R⁸, when present, is independently hydroxy, halo, alkyl, haloalkyl, alkylcarbonyl, alkoxy, alkoxycarbonyl, haloalkoxy, alkoxyalkyl, amino, alkylamino, dialkylamino, aminocarbonyl, alkylaminocarbonyl, dialkylaminocarbonyl, aminoalkyl, alkylaminoalkyl, dialkylaminoalkyl, aminoalkyloxy, alkylaminoalkyloxy, dialkylaminoalkyloxy, or alkoxyalkylaminoalkyl;
R¹⁸ is -L-R⁹, -C(O)NR²³R^{23a}, or -N(R¹²)C(O)R^{13a};
L is heterocycloalkyl; and
R⁹ is -C(O)NHR¹⁰, -C(O)R¹⁵, -S(O)₂R¹⁶ or -S(O)₂NR¹⁷R¹⁹;
R¹⁰ is alkyl, cycloalkyl, cycloalkylalkyl, aryl, arylalkyl, heterocycloalkyl, heterocycloalkylalkyl, heteroaryl, or heteroarylalkyl;
R¹¹ is hydrogen or alkyl;
each R¹² is independently hydrogen or alkyl;
each R¹³, R¹⁵, R¹⁶, and R¹⁹ is independently alkyl;
R¹⁷ is hydrogen or alkyl;
R²³ is hydrogen or alkyl; and
R^{23a} is substituted cycloalkyl, substituted heterocycloalkyl, substituted heteroaryl, or substituted aryl.

2. The Compound of Claim 1, of formula: 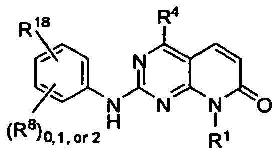 or a pharmaceutically acceptable salt thereof and additionally optionally as a solvate and additionally optionally as a hydrate thereof.

3. The Compound of Claim 1, of formula: 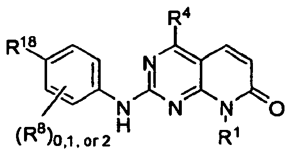 or a pharmaceutically acceptable salt thereof and additionally optionally as a solvate and additionally optionally as a hydrate thereof.

4. The Compound of Claim 3, of the formula: 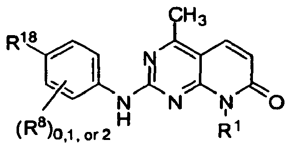 or a pharmaceutically acceptable salt thereof and additionally optionally as a solvate and additionally optionally as a hydrate thereof.

5. The Compound of Claim 1, 2, 3, or 4, wherein R¹ is alkyl or cycloalkyl; or a pharmaceutically acceptable salt thereof and additionally optionally as a solvate and additionally optionally as a hydrate thereof.

6. The Compound of Claim 1, wherein R¹⁸ is -L-R⁹ or -NHC(O)R¹³ and L is heterocycloalkyl, R⁹ is -C(O)R¹⁵ where R¹⁵ is alkyl, and R¹³ is alkyl; or a pharmaceutically acceptable salt thereof and additionally optionally as a solvate and additionally optionally as a hydrate thereof.

7. The Compound of Claim 5, wherein R¹⁸ is -C(O)NHR^{23a} where R^{23a} is substituted aryl, substituted heteroaryl or substituted heterocyloalkyl; or a pharmaceutically acceptable salt thereof and additionally optionally as a solvate and additionally optionally as a hydrate thereof.

8. The Compound of Claim 1, 2, 3, or 4, wherein R¹ is alkyl or cycloalkyl; and R¹⁸ is -L-R⁹ or -NHC(O)R¹³ and L is heterocycloalkyl, R⁹ is -C(O)R¹⁵ where R¹⁵ is alkyl, and wherein R¹³ is alkyl; or a a pharmaceutically acceptable salt thereof and additionally optionally as a solvate and additionally optionally as a hydrate thereof.

9. The Compound of Claim 1, 2, 3, or 4, wherein R¹ is alkyl or cycloalkyl; and R¹⁸ is -C(O)NHR^{23a} where R^{23a} is substituted aryl, substituted heteroaryl or substituted heterocyloalkyl; or a pharmaceutically acceptable salt thereof and additionally optionally as a solvate and additionally optionally as a hydrate thereof.

10. The Compound of Claim 1 selected from:
2-{[4-(4-acetylpiperazin-1-yl)phenyl]amino}-8-ethyl-4-methylpyrido[2,3-d]pyrimidin-7(8*H*)-one;
*N*-{4-[(8-ethyl-4-methyl-7-oxo-7,8-dihydropyrido[2,3-d]pyrimidin-2-yl)amino]phenyl}acetamide;
*N*-{4-[(8-ethyl-4-methyl-7-oxo-7,8-dihydropyrido[2,3-d]pyrimidin-2-yl)amino]phenyl}-*N*²,*N*²-dimethylglycinamide;
*N*-{4-[(8-ethyl-4-methyl-7-oxo-7,8-dihydropyrido[2,3-d]pyrimidin-2-yl)amino]phenyl}-2,2,2-trifluoroacetamide;
methyl 4-[({4-[(8-cyclopentyl-4-methyl-7-oxo-7,8-dihydropyrido[2,3-d]pyrimidin-2-yl)amino]phenyl}carbonyl)amino]-1-methyl-1*H*-pyrrole-2-carboxylate; and
*N*-{4-[(8-ethyl-4-methyl-7-oxo-7,8-dihydropyrido[2,3-d]pyrimidin-2-yl)amino]phenyl}-2-methylalaninamide; and
optionally as a pharmaceutically acceptable salt and additionally optionally as a solvate and additionally optionally as a hydrate thereof.

11. A pharmaceutical composition which comprises a compound of Claim 1 or 10 optionally as a pharmaceutically acceptable salt and additionally optionally as a solvate and additionally optionally as a hydrate thereof, and a pharmaceutically acceptable carrier, excipient, or diluent.

12. A Compound of Claim 1 or 10, optionally as a pharmaceutically acceptable salt and additionally optionally as a solvate and additionally optionally as a hydrate thereof, for use in medicine.

13. A Compound of Claim 1 or 10, optionally as a pharmaceutically acceptable salt and additionally optionally as a solvate and additionally optionally as a hydrate thereof, for use in the treatment of cancer.

14. The Compound of Claim 13 where the cancer is breast cancer, colon cancer, rectal cancer, endometrial cancer, gastric carcinoma, glioblastoma, hepatocellular carcinoma, small cell lung cancer, non-small cell lung cancer, melanoma, ovarian cancer, cervical cancer, pancreatic cancer, prostate carcinoma, acute myelogenous leukemia (AML), chronic myelogenous leukemia (CML), non-Hodgkin's lymphoma, or thyroid carcinoma.

15. The Compound of claim 13, wherein said treatment is in combination with one or more treatments selected from surgery, one or more chemotherapeutic agents, one or more hormone therapies, one or more antibodies, one or more immunotherapies, radioactive iodine therapy, and radiation.

## Patentansprüche

1. Eine Verbindung der Formel (III): 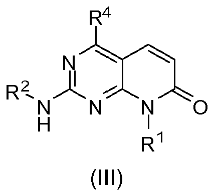 oder ein pharmazeutisch akzeptables Salz davon und zusätzlich optional als ein Solvat und zusätzlich optional als ein Hydrat davon, wobei
R¹ Alkyl, Cycloalkyl oder Heterocycloalkyl ist;
R⁴ Alkyl ist;
R² Aryl ist, das mit R¹⁸ substituiert ist und zusätzlich optional mit einem oder zwei R⁸ substituiert ist;
jeder R⁸, wenn vorhanden, unabhängig Hydroxy, Halogen, Alkyl, Halogenalkyl, Alkylcarbonyl, Alkoxy, Alkoxycarbonyl, Halogenalkoxy, Alkoxyalkyl, Amino, Alkylamino, Dialkylamino, Aminocarbonyl, Alkylaminocarbonyl, Dialkylaminocarbonyl, Aminoalkyl, Alkylaminoalkyl, Dialkylaminoalkyl, Aminoalkyloxy, Alkylaminoalkyloxy, Dialkylaminoalkyloxy oder Alkoxyalkylaminoalkyl ist;
R¹⁸-L-R⁹, -C(O)NR²³R^{23a} oder -N(R¹²)C(O)R^{13a} ist;
L Heterocycloalkyl ist; und
R⁹ -C(O)NHR¹⁰, -C(O)R¹⁵, -S(O)₂R¹⁶ oder -S(O)₂NR¹⁷R¹⁹ ist;
R¹⁰ Alkyl, Cycloalkyl, Cycloalkylalkyl, Aryl, Arylalkyl, Heterocycloalkyl, Heterocycloalkylalkyl, Heteroaryl oder Heteroarylalkyl ist;
R¹¹ Wasserstoff oder Alkyl ist;
jeder R¹² unabhängig Wasserstoff oder Alkyl ist;
jeder R¹³, R¹⁵, R¹⁶ und R¹⁹ unabhängig Alkyl ist;
R¹⁷ Wasserstoff oder Alkyl ist;
R²³ Wasserstoff oder Alkyl ist; und
R^{23a} substituiertes Cycloalkyl, substituiertes Heterocycloalkyl, substituiertes Heteroaryl oder substituiertes Aryl ist.

2. Verbindung gemäß Anspruch 1 der Formel: 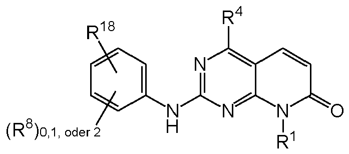 oder ein pharmazeutisch akzeptables Salz davon und zusätzlich optional als ein Solvat und zusätzlich optional als ein Hydrat davon.

3. Verbindung gemäß Anspruch 1 der Formel: 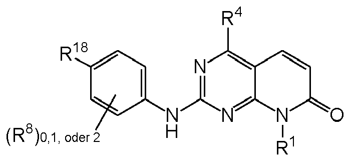 oder ein pharmazeutisch akzeptables Salz davon und zusätzlich optional als ein Solvat und zusätzlich optional als ein Hydrat davon.

4. Verbindung gemäß Anspruch 3 der Formel: 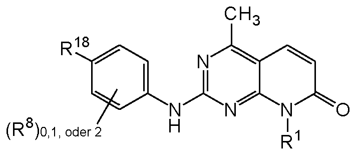 oder ein pharmazeutisch akzeptables Salz davon und zusätzlich optional als ein Solvat und zusätzlich optional als ein Hydrat davon.

5. Verbindung gemäß Anspruch 1, 2, 3 oder 4, wobei R¹ Alkyl oder Cycloalkyl ist; oder ein pharmazeutisch akzeptables Salz davon und zusätzlich optional als ein Solvat und zusätzlich optional als ein Hydrat davon.

6. Verbindung gemäß Anspruch 1, wobei R¹⁸ -L-R⁹ oder -NHC(O)R¹³ ist und L Heterocycloalkyl ist, R⁹ -C(O)R¹⁵ ist, wobei R¹⁵ Alkyl ist, und R¹³ Alkyl ist; oder ein pharmazeutisch akzeptables Salz davon und zusätzlich optional als ein Solvat und zusätzlich optional als ein Hydrat davon.

7. Verbindung gemäß Anspruch 5, wobei R¹⁸ -C(O)NHR^{23a} ist, wobei R^{23a} substituiertes Aryl, substituiertes Heteroaryl oder substituiertes Heterocycloalkyl ist; oder ein pharmazeutisch akzeptables Salz davon und zusätzlich optional als ein Solvat und zusätzlich optional als ein Hydrat davon.

8. Verbindung gemäß Anspruch 1, 2, 3 oder 4, wobei R¹ Alkyl oder Cycloalkyl ist; und R¹⁸ -L-R⁹ oder -NHC(O)R¹³ ist und L Heterocycloalkyl ist, R⁹ -C(O)R¹⁵ ist, wobei R¹⁵ Alkyl ist, und wobei R¹³ Alkyl ist; oder ein pharmazeutisch akzeptables Salz davon und zusätzlich optional als ein Solvat und zusätzlich optional als ein Hydrat davon.

9. Verbindung gemäß Anspruch 1, 2, 3 oder 4, wobei R¹ Alkyl oder Cycloalkyl ist; und R¹⁸ -C(O)NHR^{23a} ist, wobei R^{23a} substituiertes Aryl, substituiertes Heteroaryl oder substituiertes Heterocycloalkyl ist; oder ein pharmazeutisch akzeptables Salz davon und zusätzlich optional als ein Solvat und zusätzlich optional als ein Hydrat davon.

10. Verbindung gemäß Anspruch 1, ausgewählt aus:
2-{[4-(4-Acetylpiperazin-1-yl)phenyl]amino}-8-ethyl-4-methylpyrido[2,3-d]pyrimidin-7(8*H*)-on;
*N*-{4-[(8-Ethyl-4-methyl-7-oxo-7,8-dihydropyrido[2,3-d]pyrimidin-2-yl)amino]phenyl}acetamid;
*N*-{4-[(8-Ethyl-4-methyl-7-oxo-7,8-dihydropyrido[2,3-d]pyrimidin-2-yl)amino]phenyl}-*N*²,*N*²-dimethylglycinamid;
N-{4-[(8-Ethyl-4-methyl-7-oxo-7,8-dihydropyrido[2,3-d]pyrimidin-2-yl)aminol]phenyl}-2,2,2-trifluoracetamid;
Methyl-4-[({4-[(8-cyclopentyl-4-methyl-7-oxo-7,8-dihydropyrido[2,3-d]pyrimidin-2-yl)amino]phenyl}carbonyl)amino]-1-methyl-1 H-pyrrol-2-carboxylat; und
*N*-{4-[(8-Ethyl-4-methyl-7-oxo-7,8-dihydropyrido[2,3-d]pyrimidin-2-yl)amino]phenyl}-2-methylalaninamid; und
optional als ein pharmazeutisch akzeptables Salz und zusätzlich optional als ein Solvat und zusätzlich optional als ein Hydrat davon.

11. Eine pharmazeutische Zusammensetzung, die eine Verbindung gemäß Anspruch 1 oder 10, optional als ein pharmazeutisch akzeptables Salz und zusätzlich optional als ein Solvat und zusätzlich optional als ein Hydrat davon, und einen pharmazeutisch akzeptablen Träger, Hilfsstoff oder Verdünner beinhaltet.

12. Verbindung gemäß Anspruch 1 oder 10, optional als ein pharmazeutisch akzeptables Salz und zusätzlich optional als ein Solvat und zusätzlich optional als ein Hydrat davon, zur Verwendung in der Medizin.

13. Verbindung gemäß Anspruch 1 oder 10, optional als ein pharmazeutisch akzeptables Salz und zusätzlich optional als ein Solvat und zusätzlich optional als ein Hydrat davon, zur Verwendung bei der Behandlung von Krebs.

14. Verbindung gemäß Anspruch 13, wobei der Krebs Brustkrebs, Kolonkrebs, Rektalkrebs, Endometriumkrebs, Magenkarzinom, Glioblastom, Leberzellkarzinom, kleinzelliger Lungenkrebs, nicht-kleinzelliger Lungenkrebs, Melanom, Eierstockkrebs, Gebärmutterhalskrebs, Bauchspeicheldrüsenkrebs, Prostatakarzinom, akute myeloische Leukämie (AML), chronische myeloische Leukämie (CML), Non-Hodgkin-Lymphom oder Schilddrüsenkarzinom ist.

15. Verbindung gemäß Anspruch 13, wobei die Behandlung in Kombination mit einer oder mehreren Behandlungen, ausgewählt aus Chirurgie, einem oder mehreren chemotherapeutischen Mitteln, einer oder mehreren Hormontherapien, einem oder mehreren Antikörpern, einer oder mehreren Immuntherapien, Radiojodtherapie und Bestrahlung, erfolgt.

## Revendications

1. Un composé de Formule (III) : 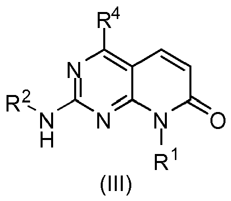 ou un sel pharmaceutiquement acceptable de celui-ci et en outre facultativement sous la forme d'un solvate et facultativement encore sous la forme d'un hydrate de celui-ci, dans lequel
R¹ est alkyle, cycloalkyle, ou hétérocycloalkyle ;
R⁴ est alkyle ;
R² est un aryle qui est substitué par R¹⁸ et en outre facultativement substitué par un ou deux R⁸ ;
chaque R⁸, lorsqu'il est présent, est indépendamment hydroxy, halo, alkyle, haloalkyle, alkylcarbonyle, alcoxy, alcoxycarbonyle, haloalcoxy, alcoxyalkyle, amino, alkylamino, dialkylamino, aminocarbonyle, alkylaminocarbonyle, dialkylaminocarbonyle, aminoalkyle, alkylaminoalkyle, dialkylaminoalkyle, aminoalkyloxy, alkylaminoalkyloxy, dialkylaminoalkyloxy, ou alcoxyalkylaminoalkyle ; R¹⁸ est -L-R⁹, -C(O)NR²³R^{23a}, ou -N(R¹²)C(O)R^{13a} ;
L est hétérocycloalkyle ; et
R⁹ est -C(O)NHR¹⁰-C(O)R¹⁵, -S(O)₂R¹⁶ ou -S(O)₂NR¹⁷R¹⁹ ;
R¹⁰ est alkyle, cycloalkyle, cycloalkylalkyle, aryle, arylalkyle, hétérocycloalkyle, hétérocycloalkylalkyle, hétéroaryle, ou hétéroarylalkyle ;
R¹¹ est hydrogène ou alkyle ;
chaque R¹² est indépendamment hydrogène ou alkyle ;
chaque R¹³, R¹⁵, R¹⁶, et R¹⁹ est indépendamment alkyle ;
R¹⁷ est hydrogène ou alkyle ;
R²³ est hydrogène ou alkyle ; et
R^{23a} est un cycloalkyle substitué, un hétérocycloalkyle substitué, un hétéroaryle substitué, ou un aryle substitué.

2. Le composé de la revendication 1, de formule : 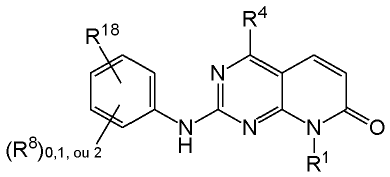 ou un sel pharmaceutiquement acceptable de celui-ci et en outre facultativement sous la forme d'un solvate et facultativement encore sous la forme d'un hydrate de celui-ci.

3. Le composé de la revendication 1, de formule : 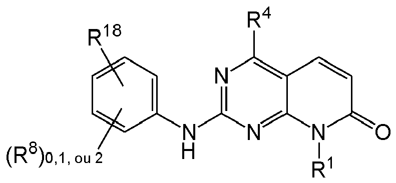 ou un sel pharmaceutiquement acceptable de celui-ci et en outre facultativement sous la forme d'un solvate et facultativement encore sous la forme d'un hydrate de celui-ci.

4. Le composé de la revendication 3, répondant à la formule : 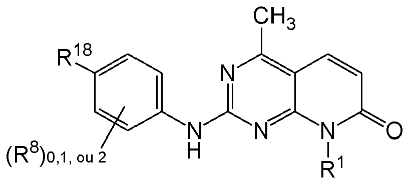 ou un sel pharmaceutiquement acceptable de celui-ci et en outre facultativement sous la forme d'un solvate et facultativement encore sous la forme d'un hydrate de celui-ci.

5. Le composé de la revendication 1, 2, 3, ou 4, dans lequel R¹ est alkyle ou cycloalkyle ; ou un sel pharmaceutiquement acceptable de celui-ci et en outre facultativement sous la forme d'un solvate et facultativement encore sous la forme d'un hydrate de celui-ci.

6. Le composé de la revendication 1, dans lequel R¹⁸ est -L-R⁹ ou -NHC(O)R¹³ et L est hétérocycloalkyle, R⁹ est -C(O)R¹⁵ où R¹⁵ est alkyle, et R¹³ est alkyle ; ou un sel pharmaceutiquement acceptable de celui-ci et en outre facultativement sous la forme d'un solvate et facultativement encore sous la forme d'un hydrate de celui-ci.

7. Le composé de la revendication 5, dans lequel R¹⁸ est -C(O)NHR^{23a} où R^{23a} est un aryle substitué, un hétéroaryle substitué ou un hétérocycloalkyle substitué ; ou un sel pharmaceutiquement acceptable de celui-ci et en outre facultativement sous la forme d'un solvate et facultativement encore sous la forme d'un hydrate de celui-ci.

8. Le composé de la revendication 1, 2, 3, ou 4, dans lequel R¹ est alkyle ou cycloalkyle ; et R¹⁸ est -L-R⁹ ou -NHC(O)R¹³ et L est hétérocycloalkyle, R⁹ est -C(O)R¹⁵ où R¹⁵ est alkyle, et dans lequel R¹³ est alkyle ; ou un sel pharmaceutiquement acceptable de celui-ci et en outre facultativement sous la forme d'un solvate et facultativement encore sous la forme d'un hydrate de celui-ci.

9. Le composé de la revendication 1, 2, 3, ou 4, dans lequel R¹ est alkyle ou cycloalkyle ; et R¹⁸ est -C(O)NHR^{23a} où R^{23a} est un aryle substitué, un hétéroaryle substitué ou un hétérocycloalkyle substitué ; ou un sel pharmaceutiquement acceptable de celui-ci et en outre facultativement sous la forme d'un solvate et facultativement encore sous la forme d'un hydrate de celui-ci.

10. Le composé de la revendication 1 sélectionné parmi :
2-{[4-(4-acétylpipérazin-1-yl)phényl]amino}-8-éthyl-4-methylpyrido[2,3-d]pyrimidin-7(8*H*)-one ;
*N*-{4-[(8-éthyl-4-méthyl-7-oxo-7,8-dihydropyrido[2,3-d]pyrimidin-2-yl)amino]phényl}acétamide ;
N-{4-[(8-éthyl-4-méthyl-7-oxo-7,8-dihydropyrido[2,3-d]pyrimidin-2-yl)amino]phényl}-*N*²,*N*²-diméthylglycinamide ;
*N*-{4-[(8-éthyl-4-méthyl-7-oxo-7,8-dihydropyrido[2,3-d]pyrimidin-2-yl)aminol]phényl}-2,2,2-trifluoroacétamide ;
4-[({4-[(8-cyclopentyl-4-méthyl-7-oxo-7,8-dihydropyrido[2,3-d]pyrimidin-2-yl)amino]phényl}carbonyl)amino]-1-méthyl-1*H*-pyrrole-2-carboxylate de méthyle; et
*N*-{4-[(8-éthyl-4-méthyl-7-oxo-7,8-dihydropyrido[2,3-d]pyrimidin-2-yl)amino]phényl}-2-méthylalaninamide ; et
facultativement sous la forme d'un sel pharmaceutiquement acceptable et en outre facultativement sous la forme d'un solvate et facultativement encore sous la forme d'un hydrate de celui-ci.

11. Une composition pharmaceutique qui comprend un composé de la revendication 1 ou de la revendication 10 facultativement sous la forme d'un sel pharmaceutiquement acceptable et en outre facultativement sous la forme d'un solvate et facultativement encore sous la forme d'un hydrate de celui-ci, et un véhicule, excipient, ou diluant pharmaceutiquement acceptable.

12. Un composé de la revendication 1 ou de la revendication 10, facultativement sous la forme d'un sel pharmaceutiquement acceptable et en outre facultativement sous la forme d'un solvate et facultativement encore sous la forme d'un hydrate de celui-ci, destiné à être utilisé en médecine.

13. Un composé de la revendication 1 ou de la revendication 10, facultativement sous la forme d'un sel pharmaceutiquement acceptable et en outre facultativement sous la forme d'un solvate et facultativement encore sous la forme d'un hydrate de celui-ci, destiné à être utilisé dans le traitement du cancer.

14. Le composé de la revendication 13 où le cancer est un cancer du sein, un cancer du côlon, un cancer du rectum, un cancer de l'endomètre, un carcinome de l'estomac, un glioblastome, un carcinome hépatocellulaire, un cancer du poumon à petites cellules, un cancer du poumon non à petites cellules, un mélanome, un cancer de l'ovaire, un cancer du col de l'utérus, un cancer du pancréas, un carcinome de la prostate, une leucémie myéloïde aiguë (LMA), une leucémie myéloïde chronique (LMC), un lymphome non hodgkinien, ou un carcinome de la thyroïde.

15. Le composé de la revendication 13, dans lequel ledit traitement se fait en combinaison avec un ou plusieurs traitements sélectionnés parmi la chirurgie, un ou plusieurs agents chimiothérapeutiques, une ou plusieurs hormonothérapies, un ou plusieurs anticorps, une ou plusieurs immunothérapies, le traitement par l'iode radioactif, et le rayonnement.
